# EUROPEAN PATENT APPLICATION

(11) **EP 4 775 573 A1**
(43) Date of publication of application: **15.07.2026**
(21) Application number: 24862891.9
(22) Date of filing: 06.09.2024
(51) Int. Cl.: C07D 403/04, C07D 403/14, C07D 405/14, C07D 451/00

(54) **SELECTIVE PRODUCTION OF SINGLE AXIALLY CHIRAL COMPOUND**

(30) Priority: 08.09.2023 WO PCT/JP2023/032816
(71) Applicant: Astellas Pharma Inc., Tokyo 103-8411 (JP)
(72) Inventor: YOSHINARI, Tomohiro, Tokyo 103-8411 (JP); IMADA, Sunao, Tokyo 103-8411 (JP); ISHIOKA, Hiroki, Tokyo 103-8411 (JP); KAWAGUCHI, Kenichi, Tokyo 103-8411 (JP); KAWAMINAMI, Eiji, Tokyo 103-8411 (JP); OHTAKA, Junpei, Tokyo 103-8411 (JP); HIROSE, Yuki, Tokyo 103-8411 (JP)
(74) Representative: Potter Clarkson
(86) International application number: PCT/JP2024/031959
(87) International publication number: WO 2025/053242

(57) **Abstract**

The present invention, in one embodiment, relates to a method for producing a compound of formula (I) or a salt thereof, as defined in the specification. The production method includes reacting a compound of formula (IIA) or a salt thereof with a compound of formula (IIIA) or a salt thereof, or alternatively reacting a compound of formula (IIB) or a salt thereof with a compound of formula (IIIB) or a salt thereof, to obtain the compound of formula (I) or a salt thereof. In the compound of formula (I), it is preferred that R⁴ is formula (VI) or (VII) and R⁵ is formula (VIII) or (IX) (each of the formulas, together with R⁴ and R⁵, is as defined in the specification).

## Description

### TECHNICAL FIELD

The present invention relates to methods for selectively producing one axially chiral compound among compounds having axial chirality. Specifically, the present invention relates to, for example, a method for selectively producing one axially chiral compound or a salt thereof among compounds of formula (I) having axial chirality or salts thereof.

The present invention also relates to a method for efficiently producing a compound for use in a method for selectively producing an axially chiral compound, as well as to an axially chiral compound obtained by such a method for selectively producing an axially chiral compound.

### BACKGROUND ART

PTL 1 (International Publication No. WO 2022/173032) discloses, for example, a compound represented by formula (A) or a salt thereof, as a quinazoline compound for inducing degradation of a G12D mutant KRAS protein, and a method for producing such a compound. 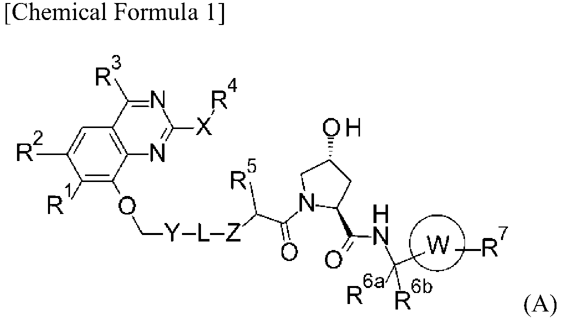 (For the definition of each group in the formula, see PTL 1.)

### CITATION LIST

### PATENT LITERATURE

PTL 1: International Publication No. WO 2022/173032

### SUMMARY OF INVENTION

As described above, PTL 1 describes a method for producing a compound represented by formula (A). During the production process, however, a compound having an axis of chirality is obtained, whereby a production intermediate or a target compound may be synthesized in the form of a stereoisomeric mixture. For example, in Production Example 11 of PTL 1, tert-butyl (1S,4S)-5-{8-(benzyloxy)-7-bromo-6-cyclopropyl-2-[(oxan-4-yl)oxy]quinazolin-4-yl}-2,5-diazabicyclo[2.2.1]heptane-2-carboxylate (3.15 g) and 6-fluoro-5-methyl-1-(tetrahydro-2H-pyran-2-yl)-4-(4,4,5,5-tetramethyl-1,3,2-dioxaborolan-2-yl)-1H-indazole (1.92 g) were reacted to give, as the M-isomer, tert-butyl (1S,4S)-5-{8-(benzyloxy)-6-cyclopropyl-7-[6-fluoro-5-methyl-1-(oxan-2-yl)-1H-indazol-4-yl1]-2-[(oxan-4-yl)oxy]quinazolin-4-yl}-2,5-diazabicyclo[2.2.1]heptane-2-carboxylate (1.42 g), and as the P-isomer, tert-butyl (1S,4S)-5-{8-(benzyloxy)-6-cyclopropyl-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (1.37 g). The M:P ratio in Production Example 11 was about 1:1. Even if a mixture of stereoisomers is obtained upon synthesis, each stereoisomer can be isolated by fractionation using a common fractionation operation, for example, ODS column chromatography or silica gel column chromatography. From the viewpoint of improving the yield of the target compound and reducing the number of fractionation steps, however, it is more desirable to establish a method for selectively obtaining one axially chiral compound.

Moreover, if a single axially chiral compound represented by formula (IIA) is obtained, the compound of formula (A) can be efficiently produced.

Furthermore, from the viewpoint of cost reduction, it is desirable to establish a novel synthetic method for the compound represented by formula (IIA), for use in the method for selectively producing an axially chiral compound.

An object of the present invention is to develop a production method for selectively obtaining one axially chiral compound or a salt thereof in the synthesis of a compound represented by formula (A).

The present inventors, through intensive investigation, have found a method for selectively obtaining one axially chiral compound or a salt thereof by controlling axial chirality using a chiral auxiliary. In the course of developing the above method, the present inventors have also found a method for efficiently producing a compound for use in a method for selectively producing an axially chiral compound, as well as an axially chiral compound obtained by such a method for selectively producing an axially chiral compound.

The present inventors have also found a method for producing a compound of formula (#1) described later by reacting a compound of formula (#2) described later with a diazo-transfer reagent.

Without being limited thereto, the present invention encompasses the following aspects.
[1] A method for producing a compound of formula (I) or a salt thereof: 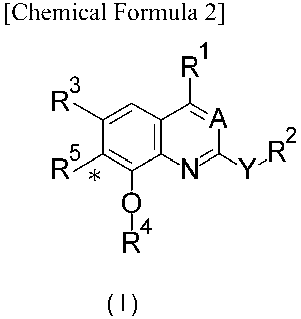 in which
   A is N or CH,
   Y is a bond, -CH₂-, -O-, -S-, or -NR^{Y}-,
   R^{Y} is H or optionally substituted C₁₋₃ alkyl,
   R¹ is formula (IV) or (V): 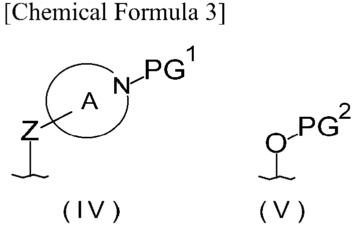
   ring A is a 7- to 9-membered optionally substituted bridged heterocycloalkane containing 1 or 2 nitrogen atoms or a 4- to 6-membered optionally substituted heterocycloalkane containing 1 or 2 nitrogen atoms,
   Z is a bond, -CH₂-, -O-, -S-, or -N(R^{Z1})-,
   R^{Z1} is H or optionally substituted C₁₋₃ alkyl,
   PG¹ is a protective group of NH contained in ring A,
   PG² is a protective group of OH,
   R² is optionally substituted C₁₋₁₅ alkyl or optionally substituted heterocycloalkyl,
   R³ is halogen, C₃₋₆ cycloalkyl, vinyl, or optionally substituted C₁₋₃ alkyl,
   R⁴ is formula (VI) or (VII), 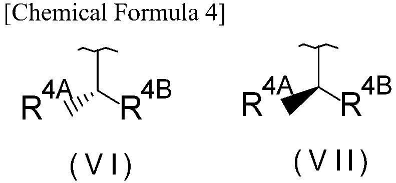
   R^{4A} is C₁₋₃ alkyl,
   R^{4B} is naphthyl, phenanthrenyl, or optionally substituted phenyl,
   R⁵ is formula (VIII) or (IX), 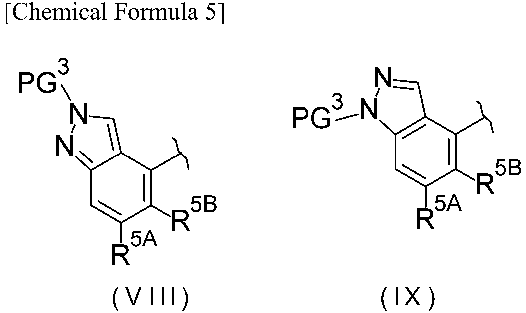
   PG³ is a protective group of NH,
   R^{5A} is H, methyl, F or Cl,
   R^{5B} is Cl, methyl, ethyl, or vinyl, and
   * denotes an axis of chirality,
   the method including reacting a compound of formula (IIA) or a salt thereof with a compound of formula (IIIA) or a salt thereof, or alternatively reacting a compound of formula (IIB) or a salt thereof with a compound of formula (IIIB) or a salt thereof, to obtain the compound of formula (I) or a salt thereof: 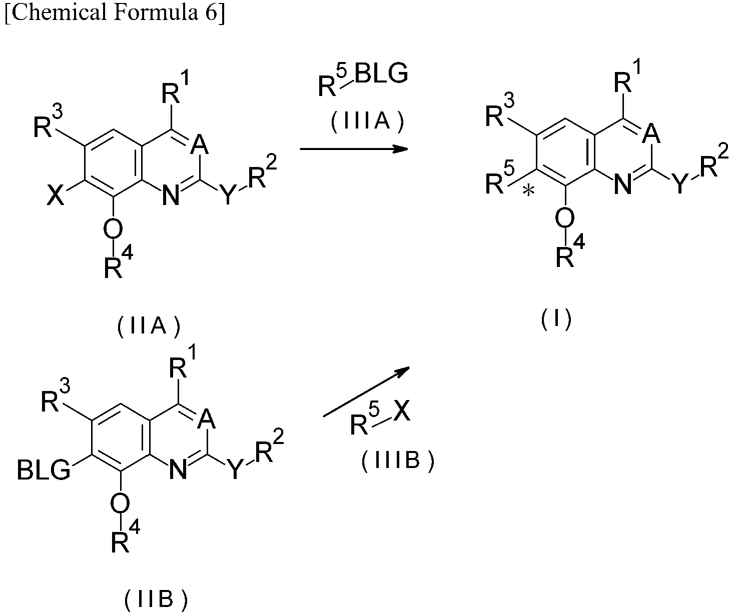 in which
      A, Y, R^{Y}, R¹, ring A, Z, R^{Z1}, PG¹, PG², R², R³, R⁴, R^{4A}, R^{4B}, R⁵, PG³, R^{5A}, R^{5B}, and * are as defined above,
      X is Cl, Br, I, a methanesulfonyloxy group, or a p-toluenesulfonyloxy group, and
      BLG is a boronic acid group, a boronic ester group, a trifluoroborate group, or a triolborate group,
      in which one axially chiral compound or a salt thereof among compounds of formula (I) having axial chirality or salts thereof is selectively produced.
[2] The method according to [1], including reacting the compound of formula (IIA) or a salt thereof with the compound of formula (IIIA) or a salt thereof,
   in which
   X is Cl or Br,
   Y is -O- or -S-,
   R¹ is a group selected from the group consisting of formulas (IV-1), (IV-2), (IV-3), and (V): 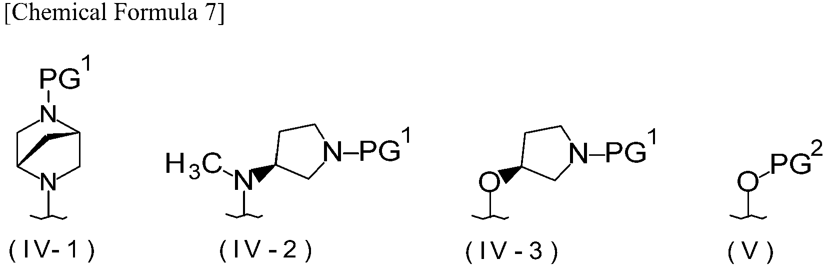
   R² is tetrahydropyranyl or C₁₋₃ alkyl optionally substituted with -OCH₃,
   R³ is cyclopropyl,
   R⁴ is a group selected from the group consisting of formulas (VI-1), (VI-2), and (VI-3): 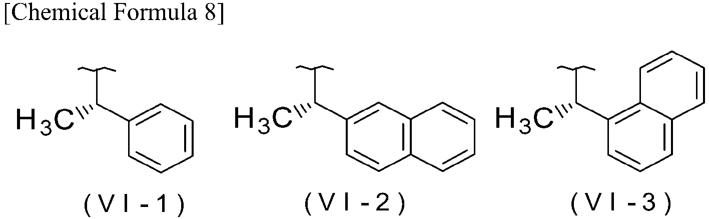 , and
   R⁵ is a group selected from the group consisting of formulas (VIII-1) and (IX-1). 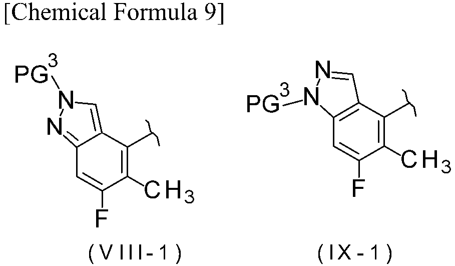
[3] The method according to [2], including reacting the compound of formula (IIA) or a salt thereof with the compound of formula (IIIA) or a salt thereof,
   in which
   A is N,
   R¹ is a group selected from the group consisting of formulas (IV-1), (IV-2), and (V): 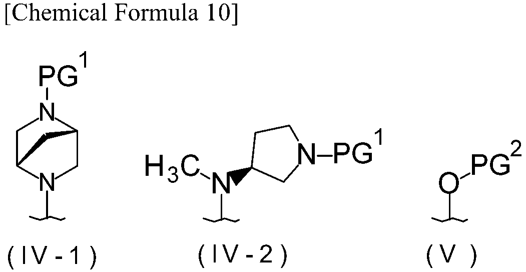 , and
   R⁵ is a group represented by formula (VIII-1). 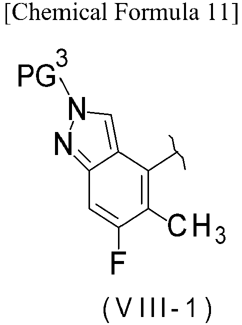
[4] The method according to [3], in which
   the compound of formula (IIA) or a salt thereof is a compound represented by formula (IIA-1): 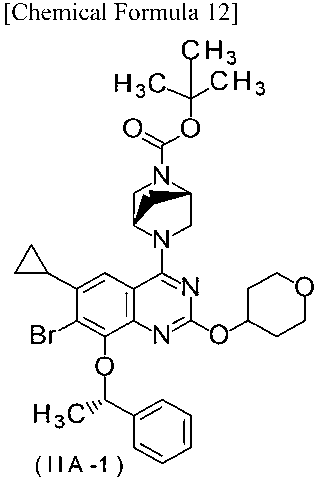 , and
   the compound of formula (IIIA) or a salt thereof is a compound represented by formula (IIIA-1): 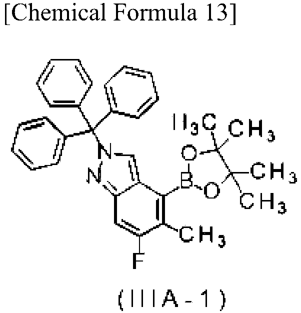
   the method including obtaining a compound represented by formula (I-1) by the following step. 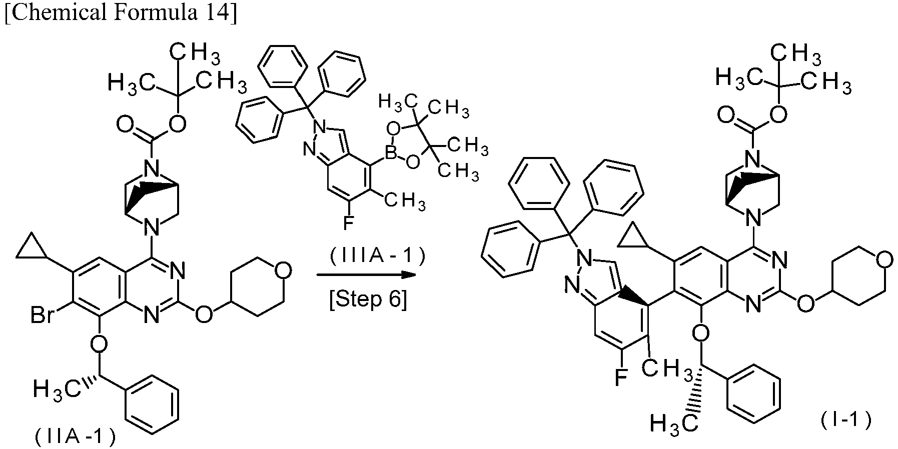
[5] The method according to [4], including obtaining compound (1) or a salt thereof by the following steps. 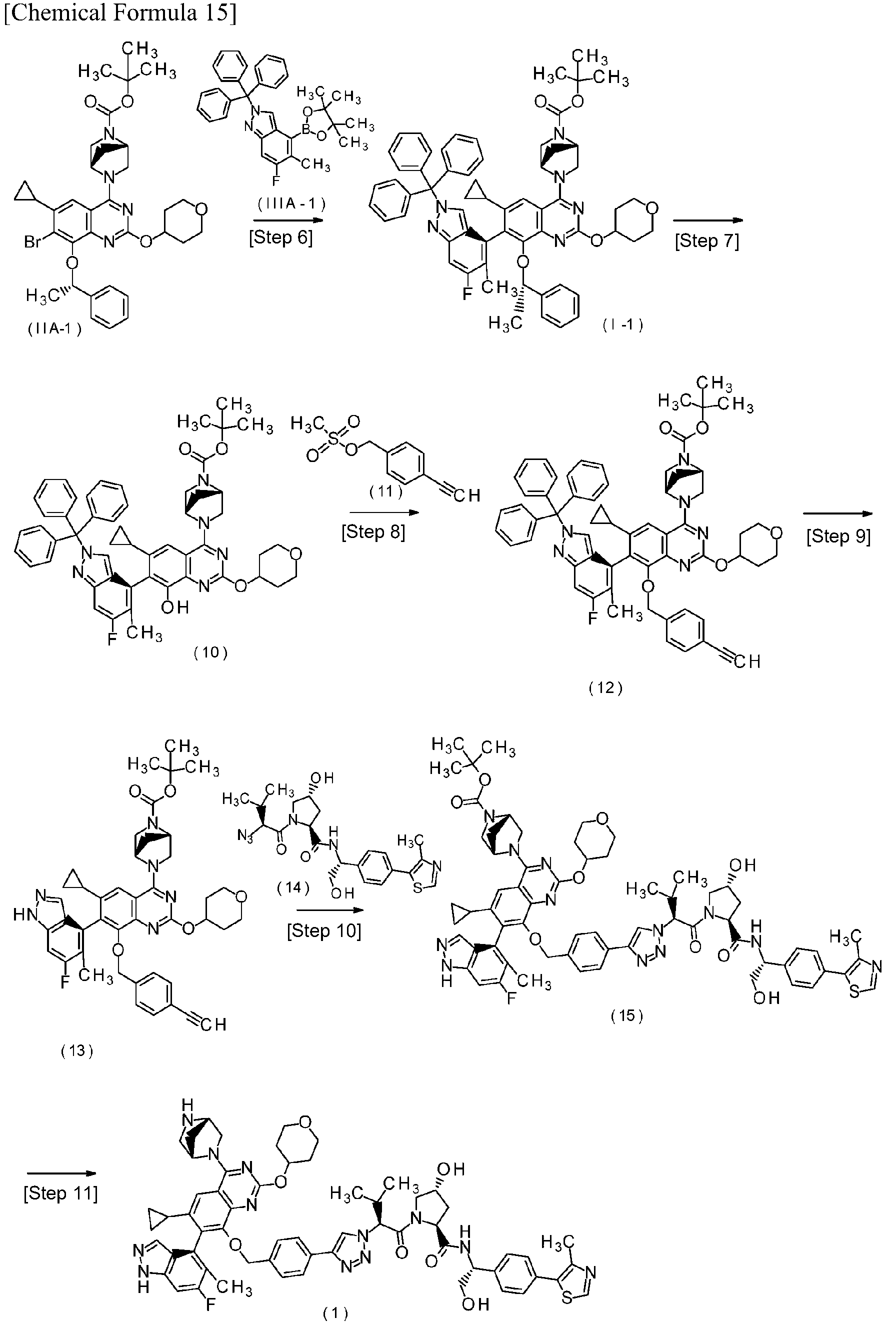
[6] The method according to [5], including obtaining compound (14) from compound (16) and compound (17) by the following steps. 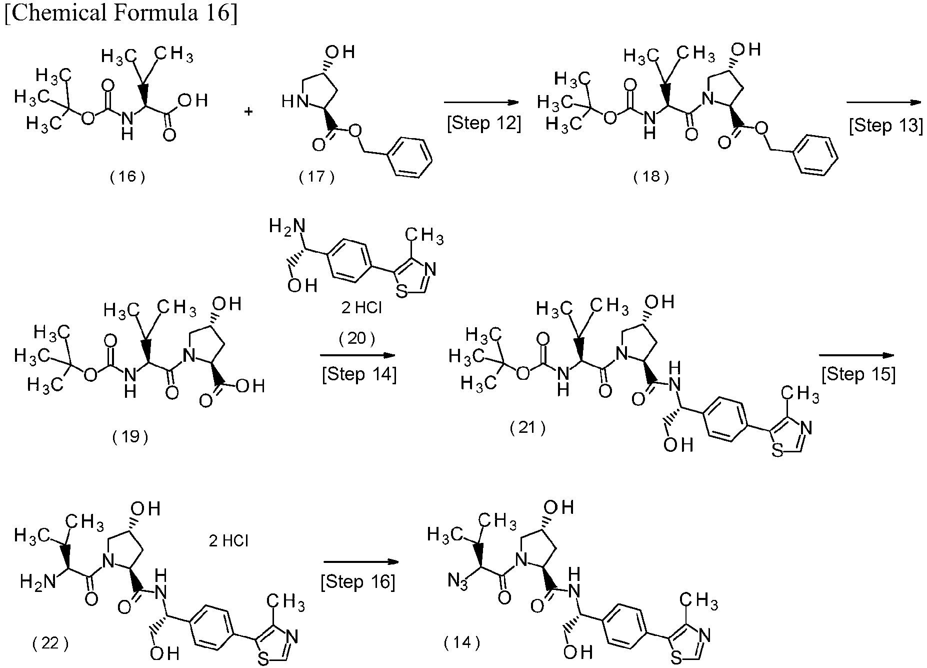
[7] The method according to [5], including obtaining a compound of formula (IIA-1) from compound (2) by the following steps. 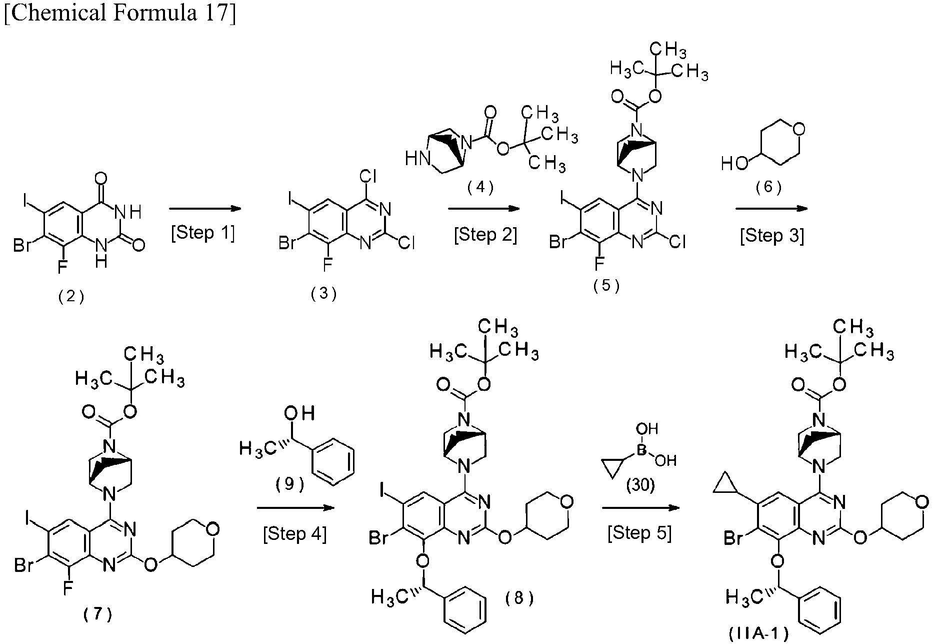
[8] The method according to [6], including obtaining compound (20) from compound (24) and compound (25) by the following steps. 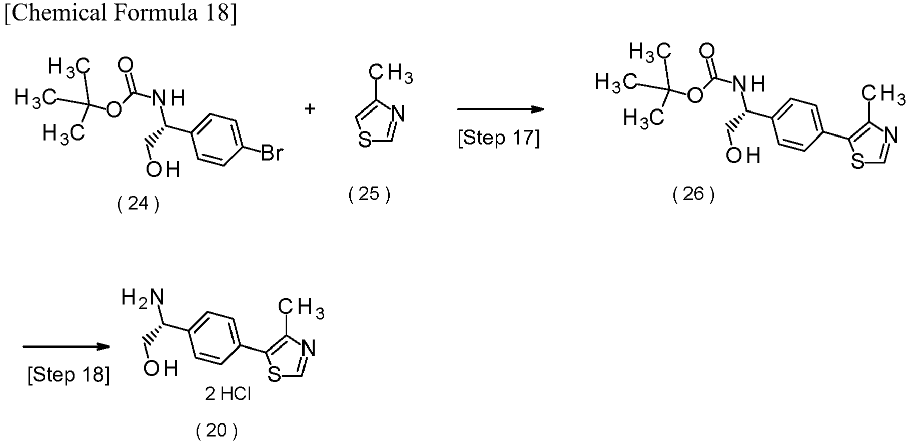
[9] The method according to any one of [4] to [8], including obtaining a compound of formula (IIIA-1) from compound (27) by the following steps. 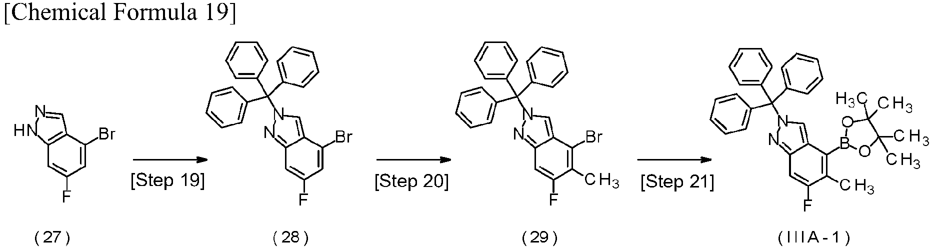
[10] The method according to [3], in which
   the compound of formula (IIA) or a salt thereof is a compound represented by formula (IIA-2): 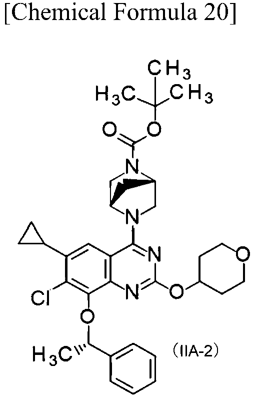 , and
   the compound of formula (IIIA) or a salt thereof is a compound represented by formula (IIIA-1): 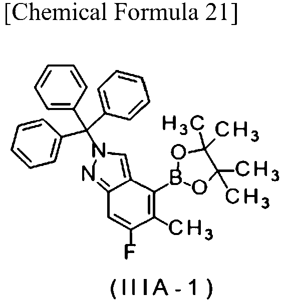
   the method including obtaining a compound represented by formula (I-1) by the following step. 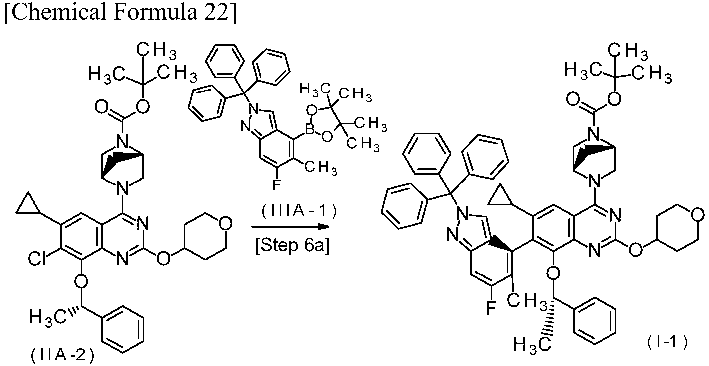
[11] The method according to [10], including obtaining compound (1) or a salt thereof by the following steps. 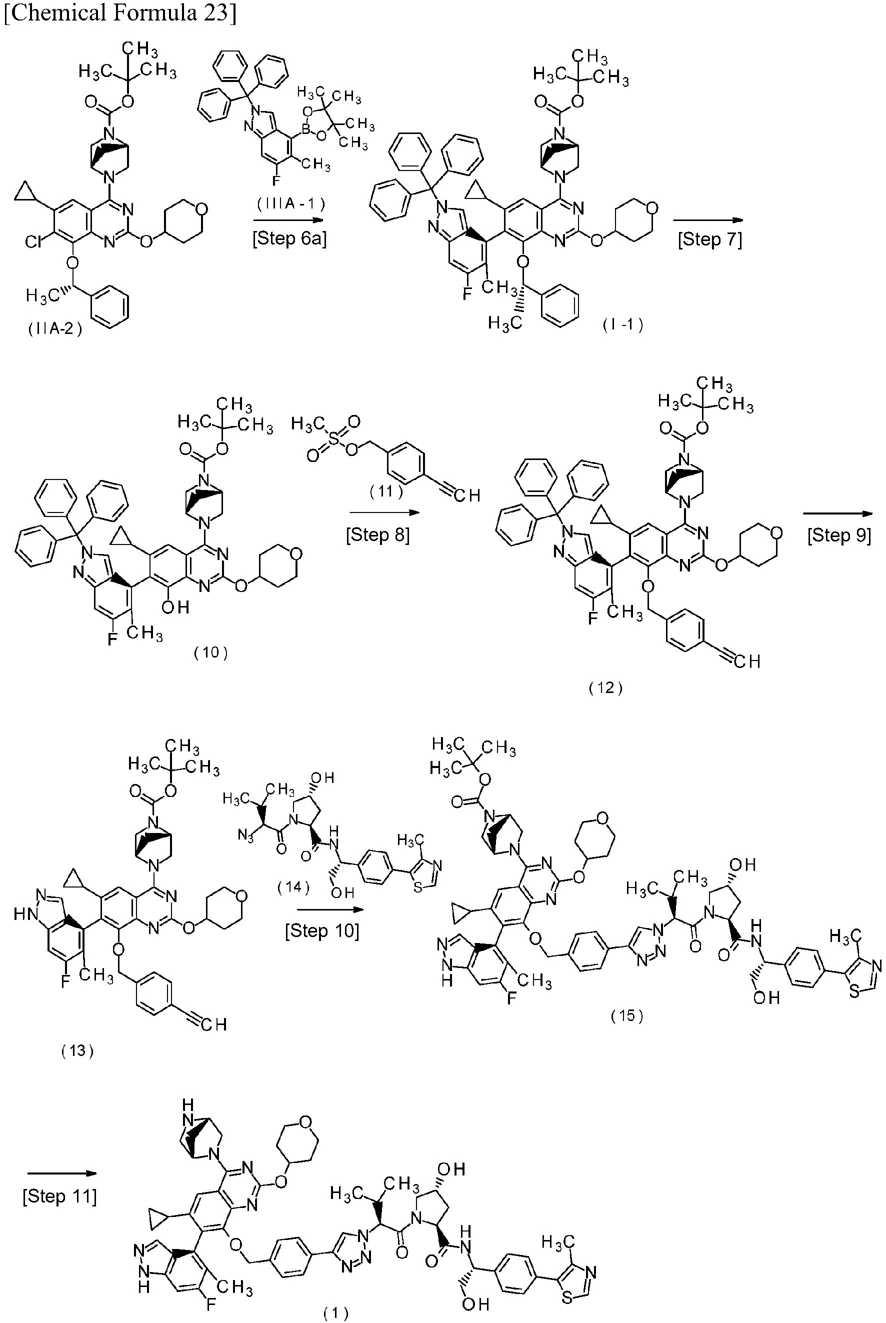
[12] The method according to [10] or [11], including obtaining the compound of formula (IIA-2) or a salt thereof by the following steps. 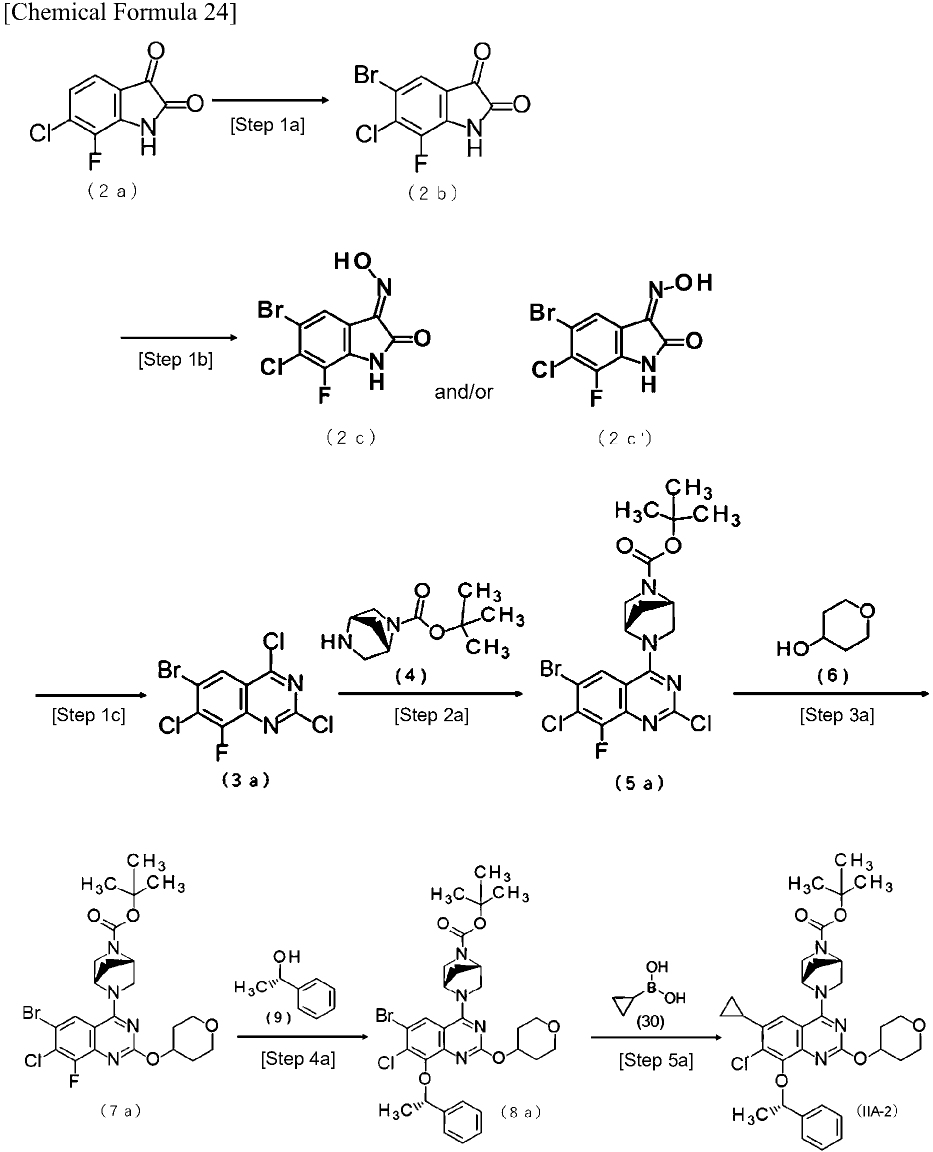
[13] A compound represented by any one of the following formulas or a salt thereof. 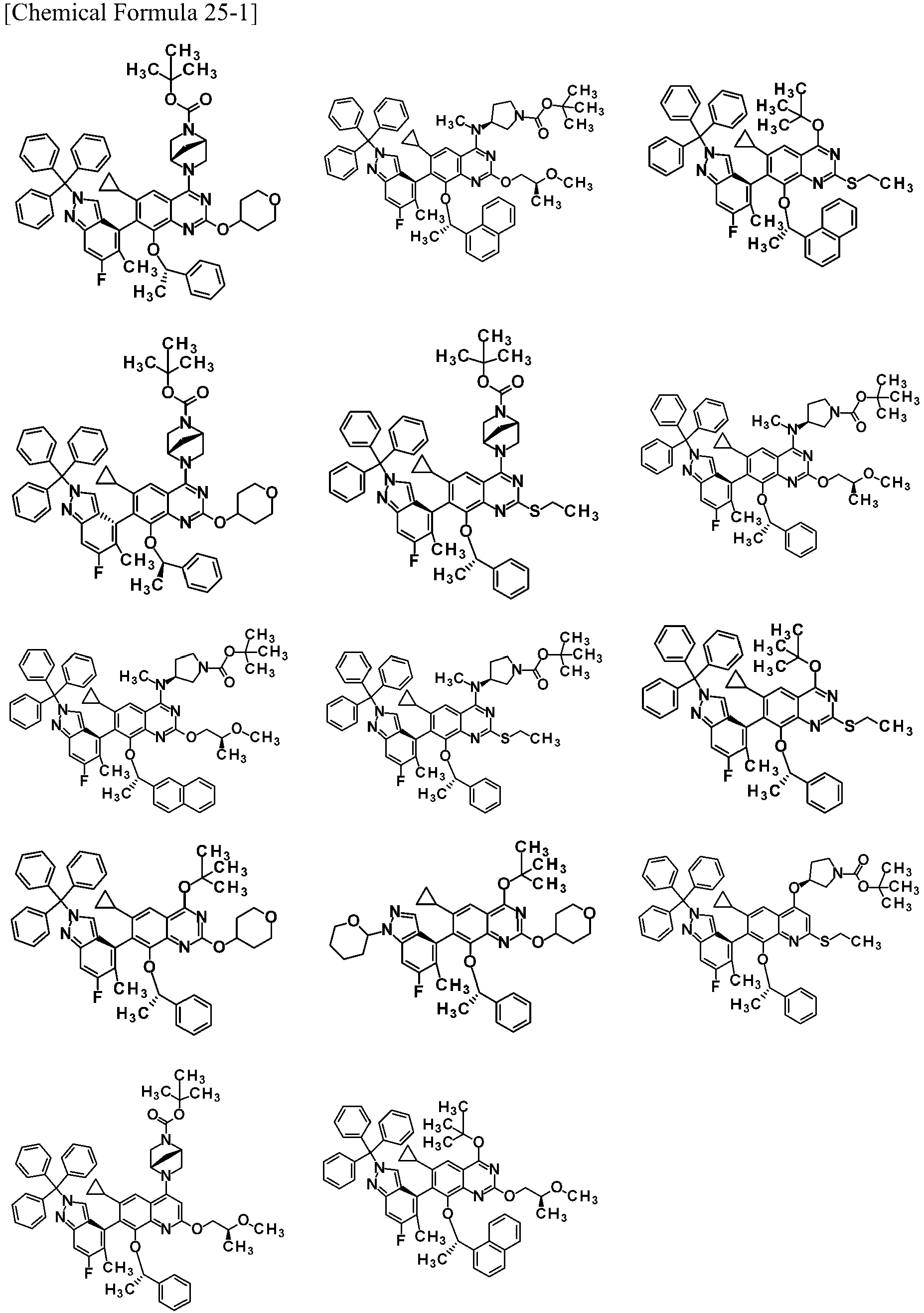 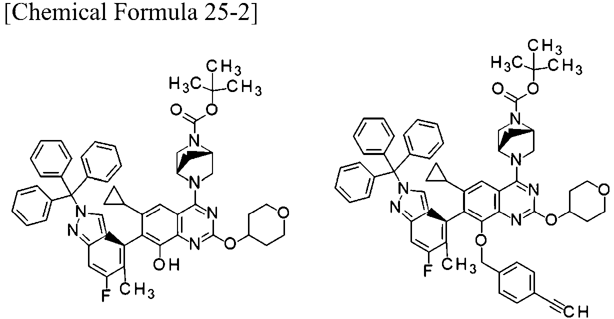 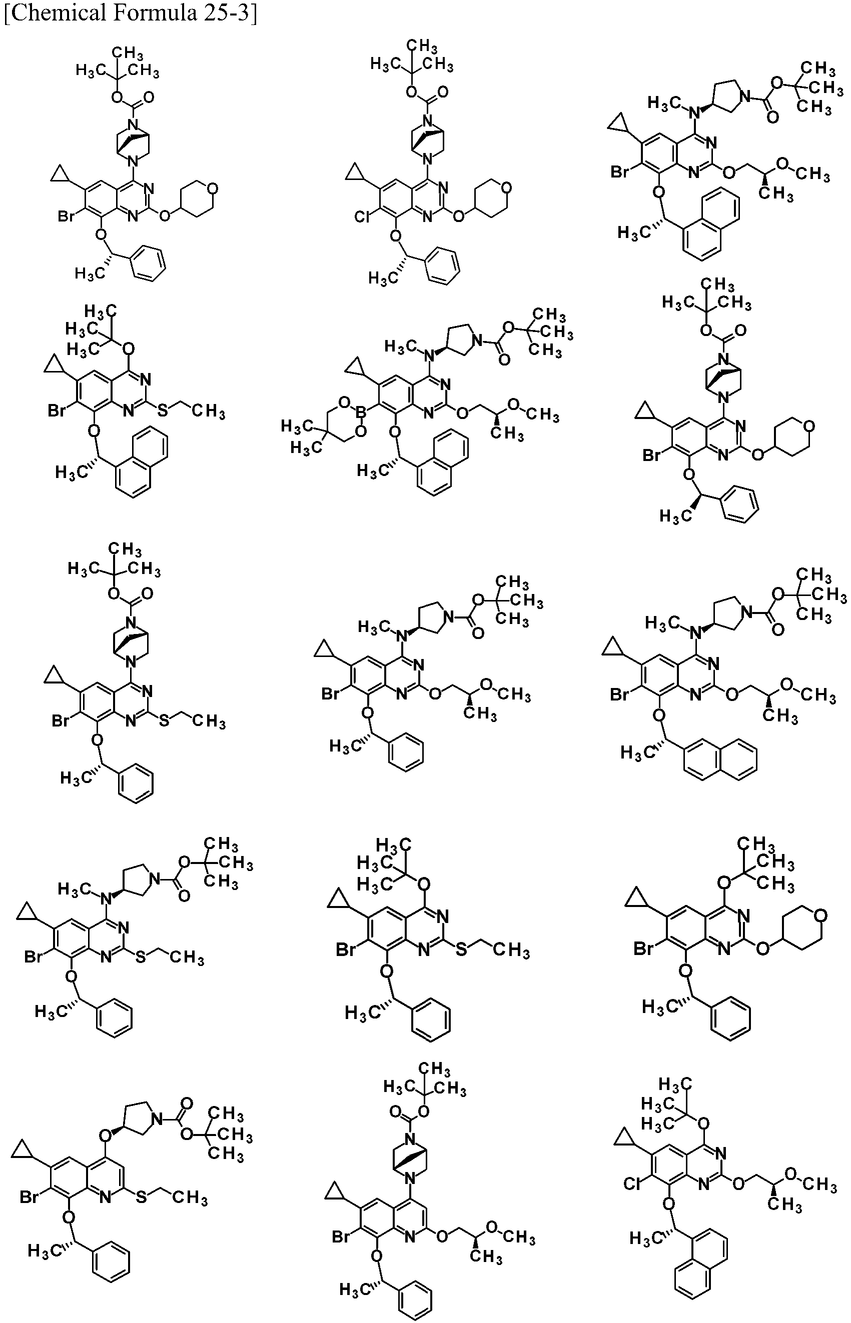
[14] The method according to [1], including
   reacting the compound of formula (IIB) or a salt thereof with the compound of formula (IIIB) or a salt thereof,
   in which
   X is Cl or Br,
   Y is -O- or -S-,
   R¹ is a group selected from the group consisting of formulas (IV-1), (IV-2), (IV-3), and (V): 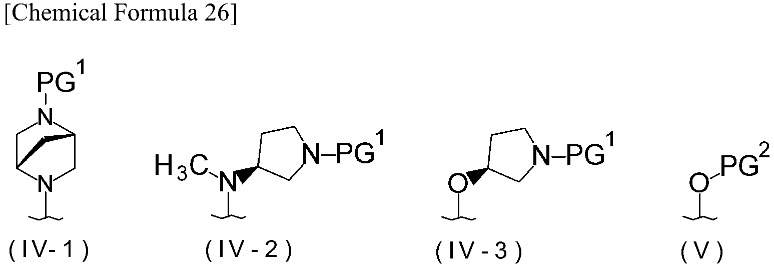
   R² is tetrahydropyranyl or C₁₋₃ alkyl optionally substituted with -OCH₃,
   R³ is cyclopropyl,
   R⁴ is a group selected from the group consisting of formulas (VI-1), (VI-2), and (VI-3): 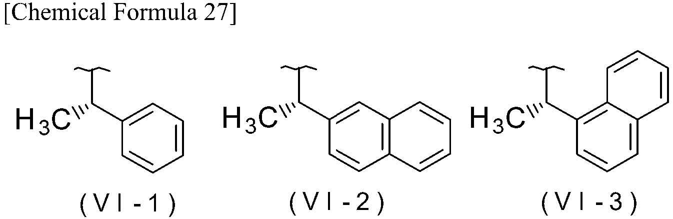 , and
   R⁵ is a group selected from the group consisting of formulas (VIII-1) and (IX-1). 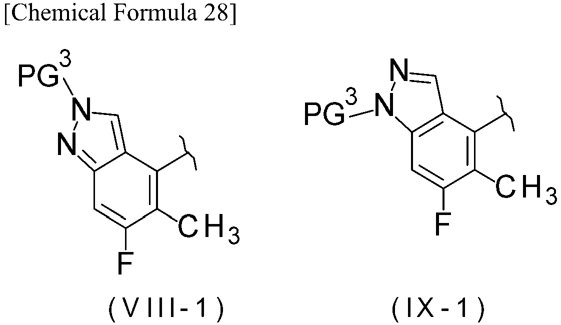
[15] The method according to [14], including
   reacting the compound of formula (IIB) or a salt thereof with the compound of formula (IIIB) or a salt thereof,
   in which
   A is N,
   R¹ is a group selected from the group consisting of formulas (IV-1), (IV-2), and (V): 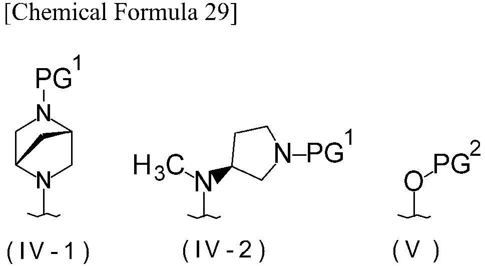 , and
   R⁵ is a group represented by formula (VIII-1). 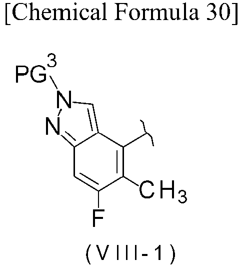
[16] The method according to [15], in which
   the compound of formula (IIB) or a salt thereof is a compound represented by formula (IIB-1): 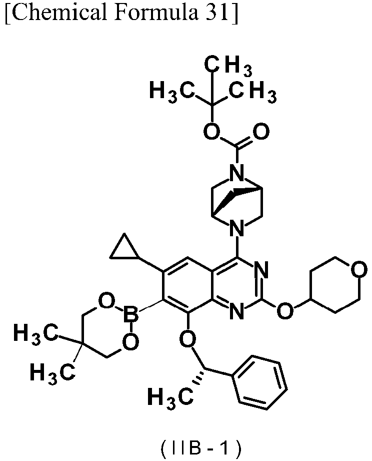 , and
   the compound of formula (IIIB) or a salt thereof is a compound represented by formula (IIIB-1): 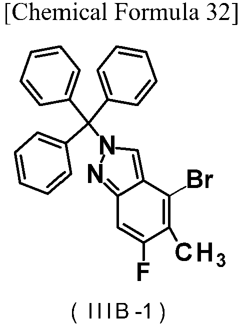
   the method including obtaining a compound represented by formula (I-1) by the following step. 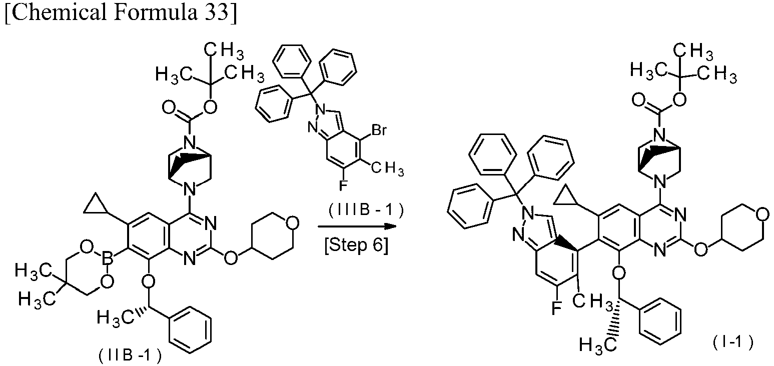
[17] The method according to any one of [1] to [4] and [14] to [16], in which the compound is produced in the presence of a palladium catalyst or a palladium catalyst precursor, and a ligand.
[18] The method according to [17], in which the compound is produced in the presence of a base.
[19] The method according to [18], in which the compound is produced in a solvent inactive for the reaction at 20°C to 140°C.
[20] The method according to [1], including
   reacting the compound of formula (IIA) or a salt thereof with the compound of formula (IIIA) or a salt thereof,
   in which R⁵ is a group selected from the group consisting of formulas (VIII-1) and (IX-1). 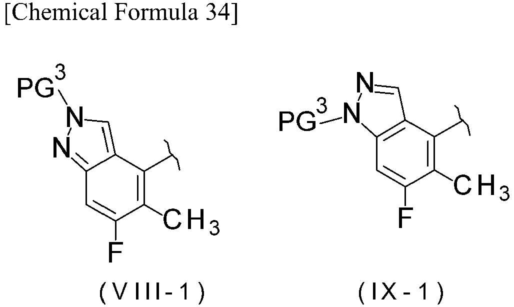
[21] The method according to [20], in which
   R⁴ is a group selected from the group consisting of formulas (VI-1), (VI-2), and (VI-3). 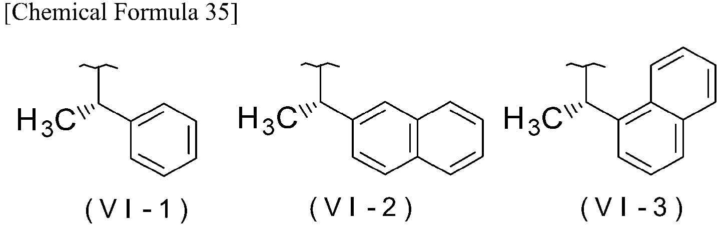
[22] The method according to [20] or [21], in which
   R³ is C₃₋₆ cycloalkyl, vinyl, or optionally substituted C₁₋₃ alkyl, and
   R³ is preferably C₃₋₆ cycloalkyl.
[23] The method according to any one of [20] to [22], in which
   X is Cl, Br, or I, and
   X is preferably Cl or Br.
[24] The method according to any one of [20] to [23], in which A is N.
[25] The method according to any one of [20] to [24], in which
   R¹ is formula (V). 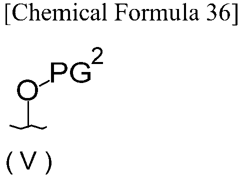
[26] The method according to any one of [20] to [25], in which
   Y is a bond, and
   R² is optionally substituted heterocycloalkyl, the heterocycloalkyl being a 4- to 7-membered saturated heterocyclic group containing 1 to 4 heteroatoms selected from the group consisting of oxygen, sulfur, and nitrogen.
[27] The method according to [26], in which
   R² is a group selected from the group consisting of formulas (X-1), (X-2), (X-3), (X-4), (X-5), (X-6), and (X-7): 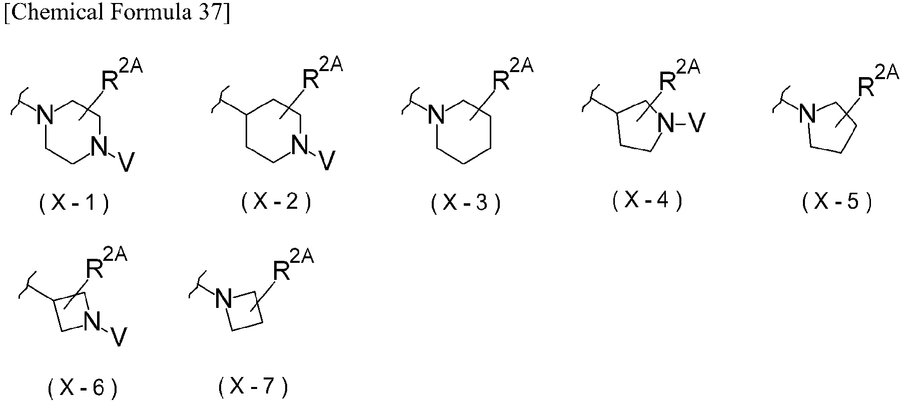
   R^{2A} is H or optionally substituted C₁₋₃ alkyl,
   V is PG⁴ or optionally substituted C₁₋₃ alkyl, and
   PG⁴ is a protective group of NH.
[28] The method according to any one of [20] to [27], in which
   R² is formula (X-1): 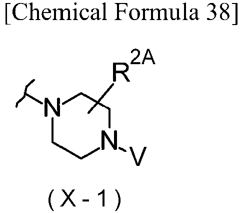
   R^{2A} is H or C₁₋₃ alkyl,
   V is PG⁴ or C₁₋₃ alkyl,
   PG⁴ is a protective group of NH,
   R³ is cyclopropyl,
   R⁴ is formula (VI-1): 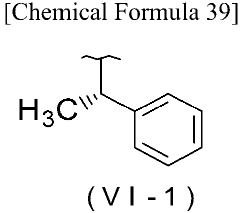 , and
   R⁵ is formula (VIII-1). 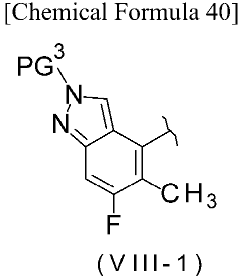
[29] A method for producing a compound of formula (#1) or a salt thereof: 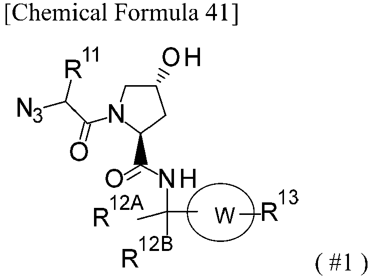 in which
   R¹¹ is optionally substituted C₁₋₆ alkyl, optionally substituted C₃₋₆ cycloalkyl, or an optionally substituted 4- to 6-membered saturated heterocyclic group containing one heteroatom selected from the group consisting of oxygen, sulfur, and nitrogen,
   R^{12A} and R^{12B}, which are the same as or different from each other, are H or optionally substituted C₁₋₆ alkyl, or R^{12A} and R^{12B}, together with the carbon to which they are attached, form optionally substituted C₃₋₆ cycloalkyl, or an optionally substituted 4- to 6-membered saturated heterocyclic ring containing one heteroatom selected from the group consisting of oxygen, sulfur, and nitrogen,
   R¹³ is H; halogen; C₁₋₃ alkyl; -SO₂CH₃; C₃₋₆ cycloalkyl; an optionally substituted 4-to 6-membered saturated heterocyclic group containing 1 or 2 heteroatoms selected from the group consisting of oxygen, sulfur and nitrogen; optionally substituted 5-membered heteroaryl containing 1 to 4 heteroatoms selected from the group consisting of oxygen, sulfur and nitrogen; or 6-membered heteroaryl containing 1 to 3 nitrogen atoms, and
   W is optionally substituted phenyl or optionally substituted 6-membered heteroaryl containing 1 to 3 nitrogen atoms,
   the method including reacting a compound of formula (#2) with a diazo-transfer reagent to obtain the compound of formula (#1) or a salt thereof: 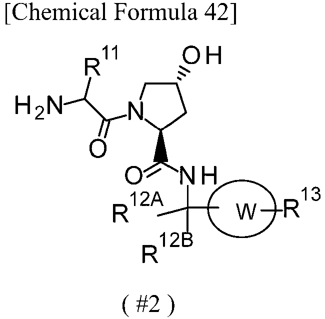 in which
      R¹¹, R^{12A}, R^{12B}, R¹³, and W are as defined above.
[30] The method according to [29], excluding use of 2-azido-1,3-dimethylimidazolium hexafluorophosphate as the diazo-transfer reagent.
[31] The method according to [29] or [30], in which
   R¹¹ is ethyl, isopropyl, tert-butyl, or C₃₋₆ cycloalkyl,
   R^{12A} and R^{12B}, which are the same as or different from each other, are H or C₁₋₃ alkyl optionally substituted with a group selected from the group consisting of F, OH, and N(CH₃)₂, or R^{12A} and R^{12B}, together with the carbon to which they are attached, form cyclopropyl,
   R¹³ is H, halogen, or a group selected from the group consisting of: 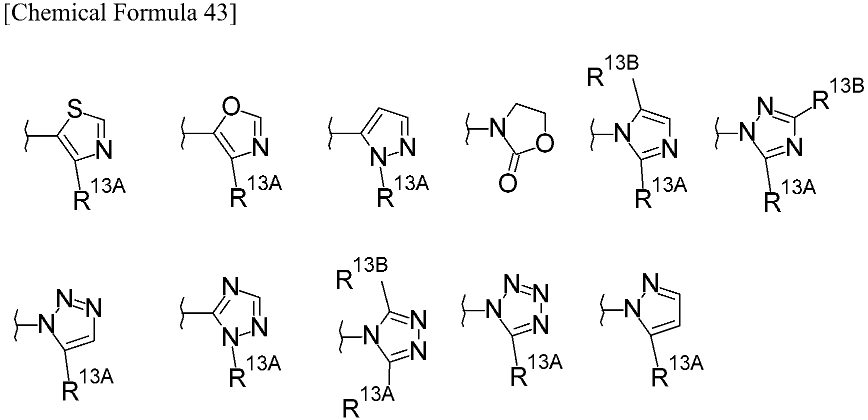
   R^{13A} and R^{13B}, which are the same as or different from each other, are H or C₁₋₃ alkyl optionally substituted with OH, and
   W is phenyl.
[32] The method according to any one of [29] to [31], in which
   R¹¹ is isopropyl,
   R^{12A} is H,
   R^{12B} is C₁₋₃ alkyl optionally substituted with OH,
   R¹³ is a group below, 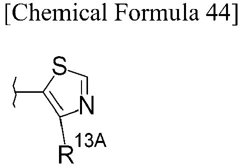
   R^{13A} is C₁₋₃ alkyl, and
   W is phenyl.
[33] The method according to any one of [29] to [32], in which the diazo-transfer reagent is perfluoroalkylsulfonyl azide.
[34] The method according to any one of [29] to [32], in which the diazo-transfer reagent is nonafluorobutanesulfonyl azide.
[35] The method according to any one of [29] to [34], in which a basic reagent is used.
[36] The method according to any one of [29] to [35], in which an additive is used.
[37] The method according to any one of [29] to [36], in which a polar solvent is used.
[38] The method according to any one of [29] to [37], in which a reaction is carried out at a temperature of 10°C to 25°C.
[39] The method according to any one of [1] to [28], including the method according to any one of [29] to [38] as a step.
[40] A compound below or a salt thereof. 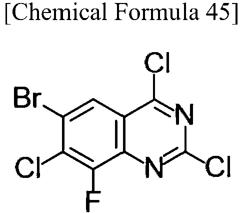
[41] A compound below or a salt thereof.

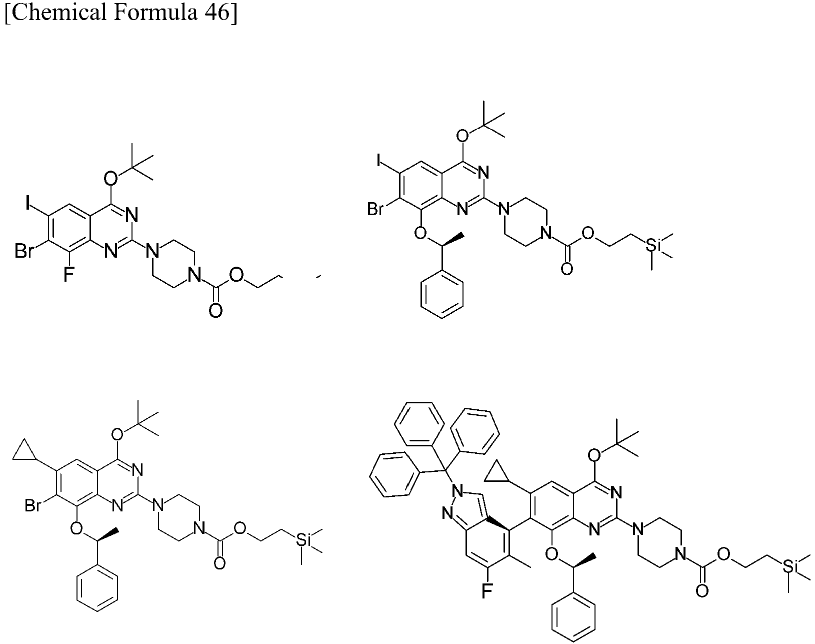

### ADVANTAGEOUS EFFECTS OF INVENTION

In one embodiment of the present invention, a chiral auxiliary is introduced during the production process, and the axis of chirality can be controlled using a chiral auxiliary, thereby enabling the selective preparation of one axially chiral compound or a salt thereof. According to the present invention, it is possible to efficiently produce a compound for use in the method for selectively producing an axially chiral compound, and also to obtain the axially chiral compound by such a method for selectively producing an axially chiral compound.

According to one embodiment of the present invention, it is possible to efficiently obtain the compound of formula (#1) or a salt thereof by reacting the compound of formula (#2) with a diazo-transfer reagent.

### DETAILED DESCRIPTION OF THE INVENTION

Hereinafter, the present invention is described in detail.

### 1. Definition

As described above, the present invention, in one aspect, relates to a method for selectively obtaining one axially chiral compound or a salt thereof. As used herein, the expression "selectively obtaining one axially chiral compound or a salt thereof" means that a ratio of an M-isomer to a P-isomer (M:P ratio) is not 1:1, but that one axially chiral compound or a salt thereof is obtained in a higher proportion than the other axially chiral compound or a salt thereof. In one aspect, in the method of the present invention, the M-isomer is obtained in a higher proportion than the P-isomer. The ratio (M:P ratio) is not particularly limited, but may be, for example, 1.3:1 or more, 1.4:1 or more, 1.5:1 or more, 1.6:1 or more, 2:1 or more, 3:1 or more, 4:1 or more, 5:1 or more, 6:1 or more, 7:1 or more, 8:1 or more, 10:1 or more, 20:1 or more, 40:1 or more, 60:1 or more, 80:1 or more, or 90:1 or more. When an M-isomer is obtained in a higher proportion than a P-isomer, the expression "1.3:1 or more" means that, relative to one unit (in gram amount or molar amount) of the P-isomer, 1.3 units or more of the M-isomer are obtained. For example, relative to 1 g of a P-isomer, 1.3 g or more of an M-isomer is obtained. In the present invention, since the M-isomer and the P-isomer have the same molecular weight, the M:P ratio expressed as a gram ratio coincides with the M:P ratio expressed as a molar ratio.

In one aspect, in the method of the present invention, the P-isomer is obtained in a higher proportion than the M-isomer. The ratio (M:P ratio) is not particularly limited, but may be, for example, 1:1.3 or more, 1:1.4 or more, 1:1.5 or more, 1:1.6 or more, 1:2 or more, or 1:5 or more. When a P-isomer is obtained in a higher proportion than an M-isomer, as described above, the expression "1:1.3 or more" means that, relative to one unit of the M-isomer, 1.3 units or more of the P-isomer are obtained.

In a method for producing an axially chiral compound without the use of a chiral auxiliary, the target compound is obtained as a mixture of stereoisomers with an M:P ratio of about 1:1. Compared with such a method, the method of the present invention is advantageous in terms of enhancing stereoisomeric selectivity when the desired axially chiral compound is obtained.

In one aspect, the present invention relates to a method for producing an axially chiral compound using a chiral auxiliary, whereby an axially chiral compound having a chiral auxiliary is obtained. The present inventors have also found that an axially chiral compound having a chiral auxiliary is easier to separate and fractionate compared with compounds without a chiral auxiliary. Specifically, a mixture of axially chiral compounds (M-isomer and P-isomer) having a chiral auxiliary obtained by the production method of the present invention can be purified to one axially chiral compound in high purity by a simple operation such as recrystallization. Furthermore, one axially chiral compound can also be purified in high purity by a common fractionation operation such as column chromatography. Thus, the production method of the present invention achieves both improved yield of the target compound having a predetermined stereoisomerism and reduction of separation and fractionation steps, thereby contributing to lower production costs.

The obtained axially chiral compound or a salt thereof can be analyzed by a known method such as HPLC or NMR. Note that there is no substantial difference between the M:P ratio calculated by HPLC analysis and the M:P ratio calculated by NMR analysis.

In the present specification, the HPLC analysis conditions described below are employed. However, the analysis conditions are not limited thereto, as long as the target compound or a salt thereof can be separated and analyzed. Note that the HPLC analysis conditions described in Tables 2 and 3 correspond to any of the following.

### <HPLC Analysis Condition 1>

· Column: InfinityLab Poroshell 120 EC-C8, 2.1 × 100 mm, 2.7 µm (Agilent)
· Mobile phase: (A) pH 2.5 aqueous HClO₄ solution; (B) iPrOH
· Elution method: B) 45% (0 min) → 45% (2 min) → 90% (22 min) → 90% (25 min)
· Flow rate: 0.1 mL/min
· Column temperature: 40°C
· Detection wavelength: 254 nm

### <HPLC Analysis Condition 2>

· Column: CORTECS C18+, 4.6 × 150 mm, 2.7 µm (Waters)
· Mobile phase: (A) 0.03% TFA in Water/MeCN (95/5); (B) 0.03% TFA in Water/MeCN (5/95)
· Elution method: B) 45% (0 min) → 70% (8 min) → 70% (20 min) → 95% (21 min) → 95% (23 min)
· Flow rate: 1.0 mL/min
· Column temperature: 40°C
· Detection wavelength: 210 nm

### <HPLC Analysis Condition 3>

·Column: InfinityLab Poroshell 120 EC-C8, 2.1 × 100 mm, 2.7 µm (Agilent)
· Mobile phase: (A) pH 2.5 aqueous HClO₄-NaClO₄ buffer solution; (B) MeOH
· Elution method: B) 70% (0 min) → 99% (30 min) → 99% (33 min)
· Flow rate: 0.15 mL/min
· Column temperature: 40°C
· Detection wavelength: 254 nm

### <HPLC Analysis Condition 4>

· Column: Ascentis Express C18, 2.1 × 100 mm, 2.7 µm (Supelco, Sigma-Aldrich)
· Mobile phase: (A) pH 2.5 aqueous HClO₄ solution; (B) MeOH
· Elution method: B) 95% (0 min) → 99% (15 min) → 99% (20 min)
· Flow rate: 0.2 mL/min
· Column temperature: 40°C
· Detection wavelength: 254 nm

### <HPLC Analysis Condition 5>

· Column: YMC-Pack C4, 4.6 × 150 mm, 3 µm (YMC)
· Mobile phase: (A) pH 7, 10 mM phosphate buffer/MeCN (95/5); (B) MeCN
· Elution method: B) 0% (0 min) → 79% (10 min) → 79% (25 min)
· Flow rate: 1.5 mL/min
· Column temperature: 40°C
· Detection wavelength: 220 nm

### <HPLC Analysis Condition 6>

· Column: ZORBAX RRHD Eclipse Plus C18, 2.1 x 50 mm, 1.8 µm (Agilent)
· Mobile phase: (A) 0.1% formic acid aqueous solution; (B) 0.1% formic acid MeCN solution
· Elution method: B) 2% (0 min) → 100% (10 min)
· Flow rate: 1.0 mL/min
· Column temperature: 40°C
· Detection wavelength: 254 nm
(Note) 1 to 2 mg of the compound was dissolved in MeOH, an appropriate amount of 4M hydrochloric acid/ethyl acetate was added thereto, and the mixture was shaken several times to remove the protective group, followed by HPLC measurement.

### <HPLC Analysis Condition 7>

·Column: InfinityLab Poroshell 120 EC-C8, 2.1 × 100 mm, 2.7 µm (Agilent)
· Mobile phase: (A) pH 2.5 aqueous HClO₄ solution; (B) iPrOH
· Elution method: B) 50% (0 min) → 65% (15 min) → 90% (23 min) → 90% (25 min)
· Flow rate: 0.1 mL/min
· Column temperature: 40°C
· Detection wavelength: 254 nm

### <HPLC Analysis Condition 8>

· Column: YMC-Pack C4, 4.6 mm × 150 mm, 3 µm (YMC)
· Mobile phase: (A) pH 7, 10 mM aqueous K₂HPO₄ buffer solution/MeCN (95/5); (B) MeCN
· Elution method: B) 0% (0 min) → 79% (10 min) → 79% (45 min)
· Flow rate: 1.5 mL/min
· Column temperature: 40°C
· Detection wavelength: 220 nm

### <HPLC Analysis Condition 9>

· Column: YMC-Pack Pro C4, 2.0 × 100 mm, 3 µm (YMC)
· Mobile phase: (A) pH 7, 10 mM aqueous K₂HPO₄ buffer solution/MeCN (95/5); (B) MeCN
· Elution method: B) 74% (0 min) → 74% (60 min)
· Flow rate: 0.4 mL/min
· Column temperature: 40°C
· Detection wavelength: 220 nm

"C₁₋₁₅ Alkyl" is linear or branched alkyl having 1 to 15 carbon atoms, and examples thereof include methyl, ethyl, n-propyl, isopropyl, n-butyl, isobutyl, sec-butyl, tert-butyl, n-pentyl, n-hexyl, dodecyl, tridecyl, tetradecyl, and pentadecyl. The C₁₋₁₅ alkyl is n-propyl in one aspect and ethyl in one aspect (hereinafter, the number of carbon atoms is expressed in the same manner).

"C₁₋₆ Alkyl" is linear or branched alkyl having 1 to 6 carbon atoms, and examples thereof include methyl, ethyl, n-propyl, isopropyl, n-butyl, isobutyl, sec-butyl, tert-butyl, n-pentyl, and n-hexyl. The C₁₋₆ alkyl is isopropyl in one aspect.

Similarly, "C₁₋₃ alkyl" refers to linear or branched alkyl having 1 to 3 carbon atoms, and examples thereof include methyl, ethyl, n-propyl, and isopropyl. The C₁₋₃ alkyl is n-propyl, ethyl, or methyl in one aspect, n-propyl in one aspect, ethyl in one aspect, and methyl in one aspect.

"C₃₋₆ Cycloalkyl" refers to cycloalkyl having 3 to 6 carbon atoms, and examples thereof include cyclopropyl, cyclobutyl, cyclopentyl and cyclohexyl. In one aspect, the C₃₋₆ cycloalkyl is cyclopropyl.

"Heterocycloalkane" is a 4- to 7-membered saturated heterocyclic ring containing 1 to 4 heteroatoms selected from the group consisting of oxygen, sulfur, and nitrogen as ring-forming atoms and may partially contain an unsaturated bond. The sulfur atom as the ring-forming atom of the saturated heterocyclic ring may be oxidized. In one aspect, the "heterocycloalkane" may contain 1 or 2 heteroatoms selected from the group consisting of oxygen, sulfur, and nitrogen as ring-forming atoms, and may contain 1 or 2 nitrogen atoms. The "heterocycloalkane" may be a 4- to 6-membered saturated heterocyclic ring in one aspect, and a 5- or 6-membered saturated heterocyclic ring in one aspect. The "heterocycloalkane" in one aspect is a "4- to 6-membered heterocycloalkane containing 1 or 2 nitrogen atoms", in one aspect is oxetane, tetrahydrofuran, tetrahydropyran, azetidine, pyrrolidine, piperidine, oxazolidine, imidazolidine, piperazine, morpholine, thiomorpholine, or dioxothiomorpholine, and in one aspect is pyrrolidine.

"Heterocycloalkyl" is a 4- to 7-membered saturated heterocyclic group containing 1 to 4 heteroatoms selected from the group consisting of oxygen, sulfur, and nitrogen as ring-forming atoms and may partially contain an unsaturated bond. In one aspect, the "heterocycloalkyl" may contain 1 or 2 heteroatoms selected from the group consisting of oxygen, sulfur, and nitrogen as ring-forming atoms, and may contain 1 or 2 nitrogen atoms. The "heterocycloalkyl" may be a 4- to 6-membered saturated heterocyclic group in one aspect, and a 5- or 6-membered saturated heterocyclic group in one aspect. The sulfur atom as a ring-forming atom of the saturated heterocyclic group is optionally oxidized. The "heterocycloalkyl" in one aspect is oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl, or dioxothiomorpholinyl, and in one aspect is tetrahydropyranyl or piperazinyl, and in one aspect is tetrahydropyranyl, and in one aspect is piperazinyl.

"Bridged heterocycloalkane" is a 7- to 9-membered bridged heterocyclic ring containing 1 or 2 nitrogen atoms as ring-forming atoms. The "bridged heterocycloalkane" in one aspect is "a 7- to 9-membered bridged heterocycloalkane containing 1 or 2 nitrogen atoms", and is diazabicyclo[2.2.2]octane, diazabicyclo[3.2.1]octane, diazabicyclo[3.1.1]heptane, diazabicyclo[2.2.1]heptane, or diazabicyclo[3.3.1]nonane, and in one aspect is diazabicyclo[2.2.1]heptane, and in one aspect is 2,5-diazabicyclo[2.2.1]heptane.

"Halogen" means F, Cl, Br and I. The halogen in one aspect is F, Cl, or Br, in one aspect is F or Cl, in one aspect is F or Br, in one aspect is F, in one aspect is Cl, and in one aspect is Br.

The "protective group of OH" means a substituent that protects OH from specific chemical reactions. The protective group of OH in one aspect is a tert-butyl group, a benzyl group, a p-methoxybenzyl group, a methoxymethyl group, a trimethylsilyl group, a triethylsilyl group, a tert-butyldimethylsilyl group, an acetyl group, a benzoyl group, or a triphenylmethyl group, and in one aspect is a tert-butyl group.

The "protective group of NH" means a substituent that protects NH from specific chemical reactions. The protective group of NH in one aspect is a tert-butoxycarbonyl group, a benzyloxycarbonyl group, a 9-fluorenylmethyloxycarbonyl group, an allyloxycarbonyl group, a phthaloyl group, a 2-nitrobenzenesulfonyl group, a 2-(trimethylsilyl)ethoxycarbonyl group, a 2,2,2-trichloroethoxycarbonyl group, a triphenylmethyl group, or a tetrahydropyranyl group, and in one aspect is a tert-butoxycarbonyl group, a triphenylmethyl group, or a tetrahydropyranyl group, and in one aspect is a tert-butoxycarbonyl group, and in one aspect is 2-(trimethylsilyl)ethoxycarbonyl group, and in one aspect is a triphenylmethyl group, and in one aspect is a tetrahydropyranyl group.

In this specification, "optionally substituted" means being unsubstituted or having 1 to 5 substituents. In one aspect, the "optionally substituted" means being unsubstituted or having 1 to 3 substituents. Note that when there are a plurality of substituents, the substituents may be the same as or different from each other.

In one aspect, substituents acceptable in "optionally substituted C₁₋₁₅ alkyl", "optionally substituted C₁₋₆ alkyl", "optionally substituted C₁₋₃ alkyl", "optionally substituted C₃₋₆ cycloalkyl", "optionally substituted 7- to 9-membered bridged heterocycloalkane containing 1 or 2 nitrogen atoms", "optionally substituted 4- to 6-membered saturated heterocyclic group containing one heteroatom selected from the group consisting of oxygen, sulfur, and nitrogen", "optionally substituted 4- to 6-membered heterocycloalkane containing 1 or 2 nitrogen atoms", "optionally substituted heterocycloalkyl", "optionally substituted 5-membered heteroaryl containing 1 to 4 heteroatoms selected from the group consisting of oxygen, sulfur, and nitrogen", "optionally substituted 6-membered heteroaryl containing 1 to 3 nitrogen atoms", or "optionally substituted phenyl" are each independently F, OH, OCH₃, N(CH₃)₂, C₁₋₃ alkyl, C₂₋₃ alkenyl (ethenyl, 1-propenyl, or 2-propenyl), C₂₋₃ alkynyl (ethynyl, 1-propynyl, or 2-propynyl), hydroxymethyl, methoxymethyl, difluoroethyl, optionally substituted C₃₋₆ cycloalkyl, azabicyclo[3.3.0]octanyl, and optionally substituted 4- to 6-membered saturated heterocyclic group containing 1 or 2 heteroatoms selected from oxygen, sulfur, and nitrogen. In one aspect the substituents are OCH₃.

Depending on the type of functional group, it may be technically effective in the production to replace the functional group with an appropriate protective group (group that can be readily converted back to the functional group) during the reaction process. Examples of the protective group include protective groups described in P. G. M. Wuts and T. W. Greene, "Greene's Protective Groups in Organic Synthesis", 5th edition, John Wiley & Sons Inc., 2014, and a group appropriately selected from the protective groups is used depending on the reaction conditions. A reaction is carried out with the protective group introduced, and then the protective group is removed, as required, whereby a desired compound can be obtained. In the present specification, the term "protective group" is not particularly limited as long as it achieves the foregoing object.

In one aspect, examples of the protective group include a benzyl group, a p-methoxybenzyl group, a benzyloxycarbonyl group, a tert-butyl(dimethyl)silyl group, a (trimethylsilyl)ethoxymethyl group, an acetyl group, a trifluoroacetyl group, a benzoyl group, a tert-butyl group, a tert-butoxycarbonyl group, a triphenylmethyl group, and a tetrahydropyranyl group.

### 2. Method for Selectively Producing One Axially Chiral Compound or Salt Thereof by Controlling Axial Chirality Using Chiral Auxiliary

The present invention, in one aspect, is a method for selectively producing one axially chiral compound or a salt thereof among compounds of formula (I) having axial chirality or salts thereof, the method including reacting a compound of formula (IIA) or a salt thereof with a compound of formula (IIIA) or a salt thereof, or alternatively reacting a compound of formula (IIB) or a salt thereof with a compound of formula (IIIB) or a salt thereof. In addition, the present invention, in one aspect, is a method for selectively producing one axially chiral compound or a salt thereof among compounds of formula (I) having axial chirality or salts thereof, the method including reacting a compound of formula (IIA) or a salt thereof with a compound of formula (IIIA) or a salt thereof. Note that * denotes an axis of chirality.

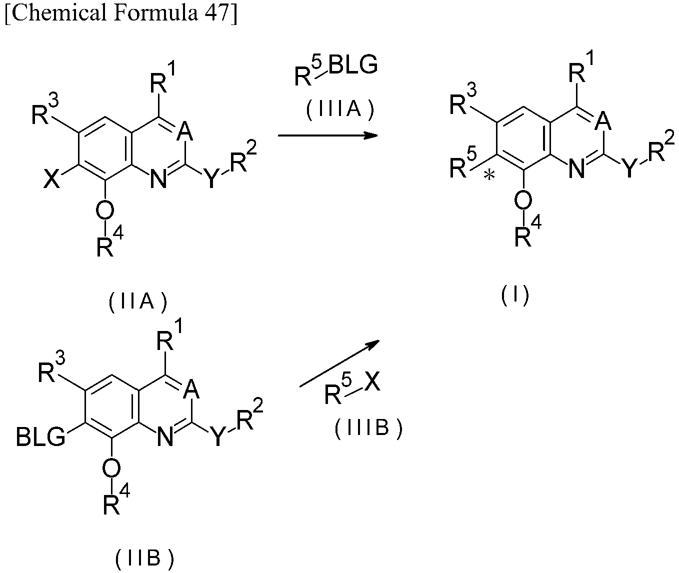

In the method, A is N or CH. In one aspect, A is N, and in one aspect, A is CH.

In the method, X is Cl, Br, I, a methanesulfonyloxy group, or a p-toluenesulfonyloxy group. In addition, in one aspect, X is Cl, Br or I, in one aspect, X is Cl or Br, in one aspect, X is Cl, and in one aspect, X is Br.

In the method, Y is a bond, -CH₂-, -O-, -S-, -NR^{Y}-, -S(=O) or -SO₂-, where R^{Y} is H or optionally substituted C₁₋₃ alkyl. In one aspect, Y is a bond, -CH₂-, -O-, -S-, or -NR^{Y}-, where R^{Y} is H or optionally substituted C₁₋₃ alkyl. In one aspect, Y is -O- or -S-, in one aspect, Y is -O-, in one aspect, Y is -S-, and in one aspect, Y is a bond.

In the method, R¹ is formula (IV) or (V).

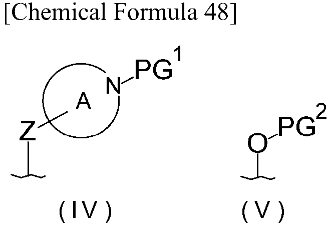

In one aspect, R¹ is a group selected from the group consisting of formulas (IV-1), (IV-2), (IV-3), and (V). In addition, in one aspect, R¹ is a group selected from the group consisting of formulas (IV-1), (IV-2), and (V), and in one aspect, R¹ is formula (IV-1). In one aspect, R¹ is formula (V).

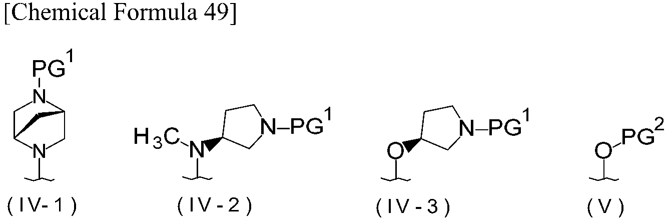

In R¹, ring A is optionally substituted 7- to 9-membered bridged heterocycloalkane containing 1 or 2 nitrogen atoms or optionally substituted 4- to 6-membered heterocycloalkane containing 1 or 2 nitrogen atoms; Z is a bond, -CH₂-, -O-, -S-, or -N(R^{Z1})-; R^{Z1} is H or optionally substituted C₁₋₃ alkyl; and PG¹ is a protective group of NH contained in ring A. Although the type thereof is not particularly limited, PG¹ in one aspect is a tert-butoxycarbonyl group, a benzyloxycarbonyl group, a 9-fluorenylmethyloxycarbonyl group, an allyloxycarbonyl group, a phthaloyl group, a 2-nitrobenzenesulfonyl group, a 2-(trimethylsilyl)ethoxycarbonyl group, or a 2,2,2-trichloroethoxycarbonyl group. PG² is a protective group of OH, and the type thereof is not particularly limited. In one aspect, PG² is a tert-butyl group, a benzyl group, a p-methoxybenzyl group, a methoxymethyl group, a trimethylsilyl group, a triethylsilyl group, a tert-butyldimethylsilyl group, an acetyl group, a benzoyl group, or a triphenylmethyl group.

In the method, R² is optionally substituted C₁₋₁₅ alkyl or optionally substituted heterocycloalkyl. In one aspect, R² is optionally substituted C₁₋₃ alkyl. In one aspect, R² is optionally substituted 4- to 7-membered heterocycloalkyl.

In one aspect, R² is tetrahydropyranyl, ethyl, or C₁₋₃ alkyl optionally substituted with -OCH₃, in one aspect, R² is tetrahydropyranyl, in one aspect, R² is ethyl, and in one aspect, R² is n-propyl substituted with -OCH₃.

In one aspect, R² is optionally substituted heterocycloalkyl, where the heterocycloalkyl is a 4- to 7-membered saturated heterocyclic group containing 1 to 4 heteroatoms selected from the group consisting of oxygen, sulfur, and nitrogen. In one aspect, the heterocycloalkyl of R² is a 4- to 6-membered saturated heterocyclic group containing 1 or 2 heteroatoms selected from the group consisting of oxygen, sulfur, and nitrogen. In one aspect, the heterocycloalkyl of R² is a 4- to 6-membered saturated heterocyclic group containing 1 or 2 nitrogen atoms. The "optionally substituted heterocycloalkyl" is to be interpreted in accordance with the foregoing description, and each is independently one or more selected from the group consisting of the substituents described above.

In one aspect, R² is a group selected from the group consisting of formulas (X-1), (X-2), (X-3), (X-4), (X-5), (X-6), and (X-7): 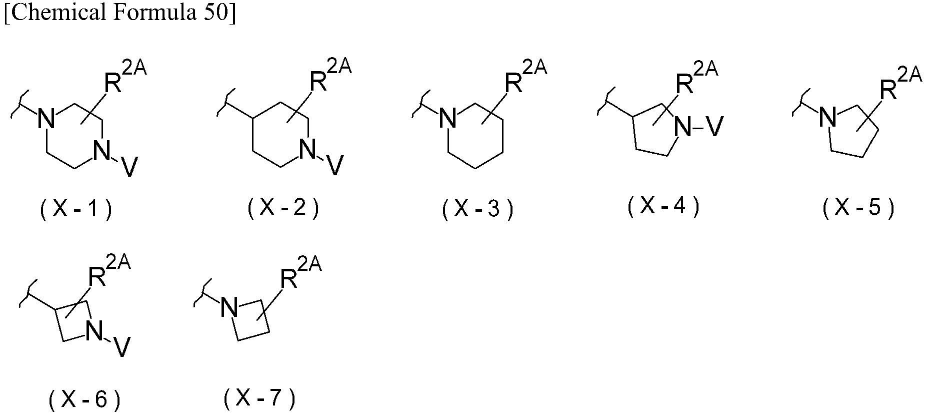 where
R^{2A} is H or optionally substituted C₁₋₃ alkyl,
V is PG⁴ or optionally substituted C₁₋₃ alkyl, and
PG⁴ is a protective group of NH.

In one aspect, R² is formula (X-1),
R^{2A} is H or C₁₋₃ alkyl,
V is PG⁴ or C₁₋₃ alkyl, and
PG⁴ is a protective group of NH.

In one aspect, PG⁴, a protective group of NH, may be one or more selected from the group consisting of the functional groups described above as "NH protective groups". In one aspect, PG⁴ is a tert-butoxycarbonyl group, a triphenylmethyl group, or a tetrahydropyranyl group, and in one aspect, PG⁴ is a 2-(trimethylsilyl)ethoxycarbonyl group.

In the method, R³ is halogen, C₃₋₆ cycloalkyl, vinyl, or optionally substituted C₁₋₃ alkyl. In one aspect, R³ is C₃₋₆ cycloalkyl, and in one aspect, R³ is cyclopropyl.

In the method, R⁴ is formula (VI) or (VII).

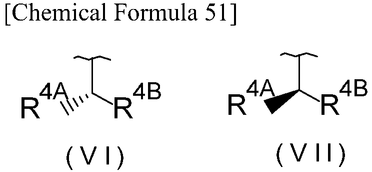

In formula (VI) or (VII), R^{4A} is C₁₋₃ alkyl, and R^{4B} is a naphthyl, phenanthrenyl, or optionally substituted phenyl.

In one aspect, R⁴ is a group selected from the group consisting of formulas (VI-1), (VI-2), and (VI-3), and in one aspect, R⁴ is formula (VI-1).

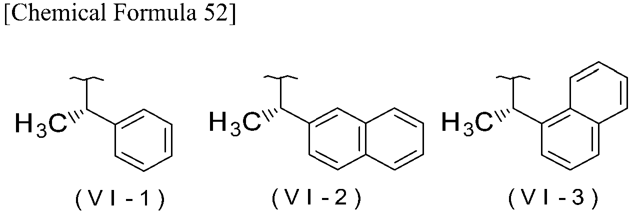

In the method, R⁵ is formula (VIII) or (IX).

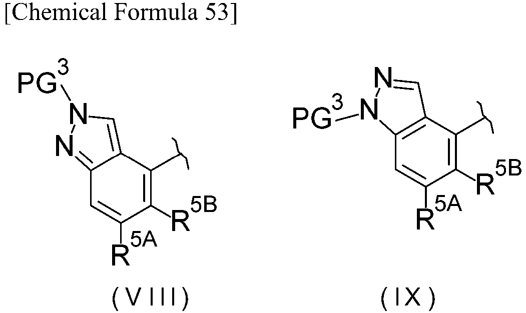

In formula (VIII) or (IX), PG³ is a protective group of NH, and the type thereof is not particularly limited. In one aspect, PG³ is a triphenylmethyl group or a tetrahydropyranyl group. R^{5A} is H, methyl, F, or Cl, and R^{5B} is Cl, methyl, ethyl, or vinyl.

In one aspect, R⁵ is formula (VIII-1) or (IX-1). In one aspect, R⁵ is formula (VIII-1).

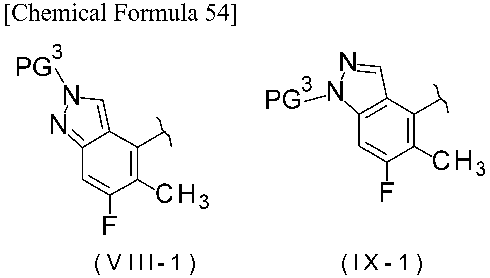

In formula (VIII-1) or (IX-1), PG³ is a protective group of NH, and the type thereof is not particularly limited. In one aspect, PG³ is a triphenylmethyl group or a tetrahydropyranyl group, and in one aspect, PG³ is a triphenylmethyl group.

In the method, BLG is a boronic acid group, a boronic ester group, a trifluoroborate group, or a triolborate group. In one aspect, BLG is a boronic acid ester group, and in one aspect, BLG is a boronic acid pinacol ester group.

In one aspect, BLG is any one of the substituents shown below.

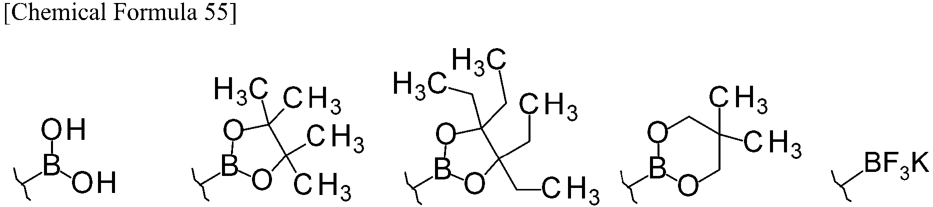

In one aspect, BLG is any one of the substituents shown below.

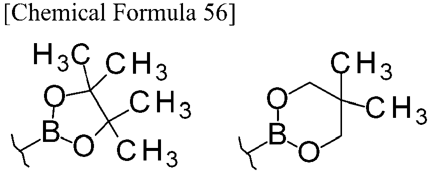

In the method for selectively producing one axially chiral compound or a salt thereof among the compounds of formula (I) having axial chirality or salts thereof, the method including reacting a compound of formula (IIA) or a salt thereof with a compound of formula (IIIA) or a salt thereof, the reaction conditions are not particularly limited. In one aspect, the production method includes using a compound of formula (IIA) or a salt thereof and a compound of formula (IIIA) or a salt thereof in an equivalent amount or with one compound thereof in an excess equivalent amount, and stirring the resulting mixture in the presence of a palladium catalyst and a ligand. In one aspect, the production method includes using a compound of formula (IIA) or a salt thereof and a compound of formula (IIIA) or a salt thereof in an equivalent amount or with one compound thereof in an excess equivalent amount, and stirring the resulting mixture in the presence of a base, a palladium catalyst, and a ligand. In one aspect, the production method includes using a compound of formula (IIA) or a salt thereof and a compound of formula (IIIA) or a salt thereof in an equivalent amount or with one compound thereof in an excess equivalent amount, and stirring the resulting mixture in a solvent inactive for the reaction, in the presence of a base, a palladium catalyst, and a ligand, from at room temperature to under reflux with heat, preferably at 20°C to 140°C. In one aspect, the production method includes using a compound of formula (IIA) or a salt thereof and a compound of formula (IIIA) or a salt thereof in an equivalent amount or with one compound thereof in an excess equivalent amount, and stirring the resulting mixture in a solvent inactive for the reaction, in the presence of a base, a palladium catalyst, and a ligand, from at room temperature to under reflux with heat, preferably at 20°C to 140°C, generally for 0.1 hours to 5 days.

Examples of the solvent used here include, but are not particularly limited to, a halogenated hydrocarbon such as dichloromethane, 1,2-dichloroethane, or chloroform, an aromatic hydrocarbon such as benzene, toluene, or xylene, an ether such as diethyl ether, THF, DOX, or 1,2-dimethoxyethane, an alcohol such as MeOH, EtOH, iPrOH, tBuOH, amyl alcohol, or 2-methyl-2-butanol, DMF, DMSO, MeCN, 1,3-dimethylimidazolidin-2-one, water, and a mixture thereof.

Examples of the base include, but are not particularly limited to, an anhydride or hydrate of an inorganic base such as barium hydroxide, tripotassium phosphate, sodium carbonate, potassium carbonate, or sodium hydroxide.

Examples of the palladium catalyst include, but are not particularly limited to, tetrakis(triphenylphosphine)palladium, bis(triphenylphosphine)palladium(II) dichloride, [1,1'-bis(diphenylphosphino)ferrocene]palladium(II) dichloride·dichloromethane adduct, (1E,4E) -1,5-diphenylpenta-1,4-dien-3-one/palladium (3:2), palladium(II) acetate, di-µ-chlorobis(2'-amino-1,1'-biphenyl-2-yl-C,N)dipalladium(II), bis(tri-tert-butylphosphine)palladium, and bis(dibenzylideneacetone)palladium.

Examples of the ligand include, but are not particularly limited to, SPhos, RuPhos, and DPPF.

In the production method, a palladium catalyst precursor may also be used instead of a palladium catalyst. Examples of the palladium catalyst precursor include, but are not particularly limited to, a palladacycle catalyst precursor, such as RuPhos Pd G3, SPhos Pd G3, RuPhos Pd G2, or SPhos Pd G2.

In one aspect, the palladium catalyst or palladium catalyst precursor is palladium(II) acetate, RuPhos Pd G3, or SPhos Pd G3. In one aspect, the ligand is SPhos or RuPhos. In one aspect, the base is barium hydroxide or tripotassium phosphate. In one aspect, the solvent is DOX, 2-methyl-2-butanol, water, or a mixture thereof. In one aspect, the reaction temperature is 50°C to 90°C. In one aspect, the reaction time is 1 hour to 2 days.

In addition, heating the mixture by microwave irradiation is sometimes advantageous for smoothly promoting the reaction.

The reaction conditions for reacting a compound of formula (IIB) or a salt thereof with a compound of formula (IIIB) or a salt thereof are also not particularly limited, but the reaction may basically be carried out under the same conditions as the reaction conditions for reacting a compound of formula (IIA) or a salt thereof with a compound of formula (IIIA) or a salt thereof, and, in one aspect, the reaction conditions described in Example 23 may be used.

The present invention, in one aspect, is a method for producing a compound of formula (I-1) by reacting the compound of formula (IIA-1) as the compound of formula (IIA) or a salt thereof with the compound of formula (IIIA-1) as the compound of formula (IIIA) or a salt thereof. In the present specification, the reaction is referred to as "Step 6".

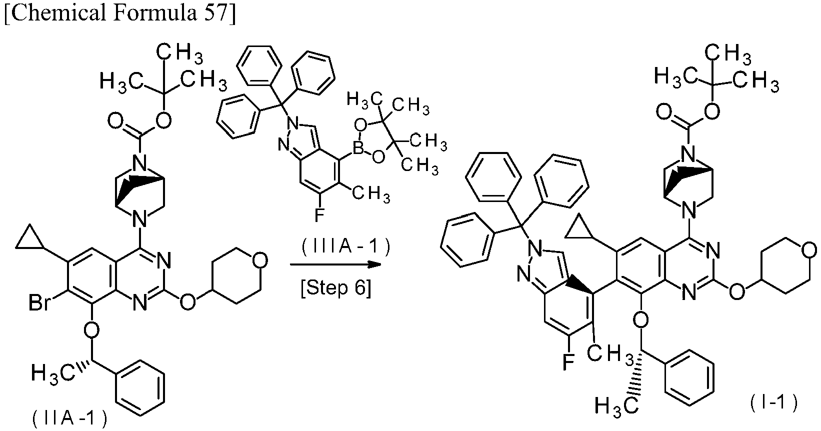

### (Step 6)

As described above, this step is a method for producing a compound of formula (I-1) by reacting the compound of formula (IIA-1) with the compound of formula (IIIA-1). As long as the reaction proceeds, the reaction conditions are not particularly limited. In one aspect, the reaction conditions are the same as the conditions for reacting the compound of formula (IIA) or a salt thereof with the compound of formula (IIIA) or a salt thereof as described above. In one aspect, the reaction conditions described in Example 3 below can be used.

The present invention, in one aspect, is a method for producing a compound of formula (I-1), the method including obtaining the compound of formula (1) from the compound of formula (IIA-1) and the compound of formula (IIIA-1) by Steps 6 to 11 described below. In one aspect, the present invention is a method for producing a compound of formula (1) from the compound of formula (IIA-1) and the compound of formula (IIIA-1), the method including Steps 6 to 11 described below.

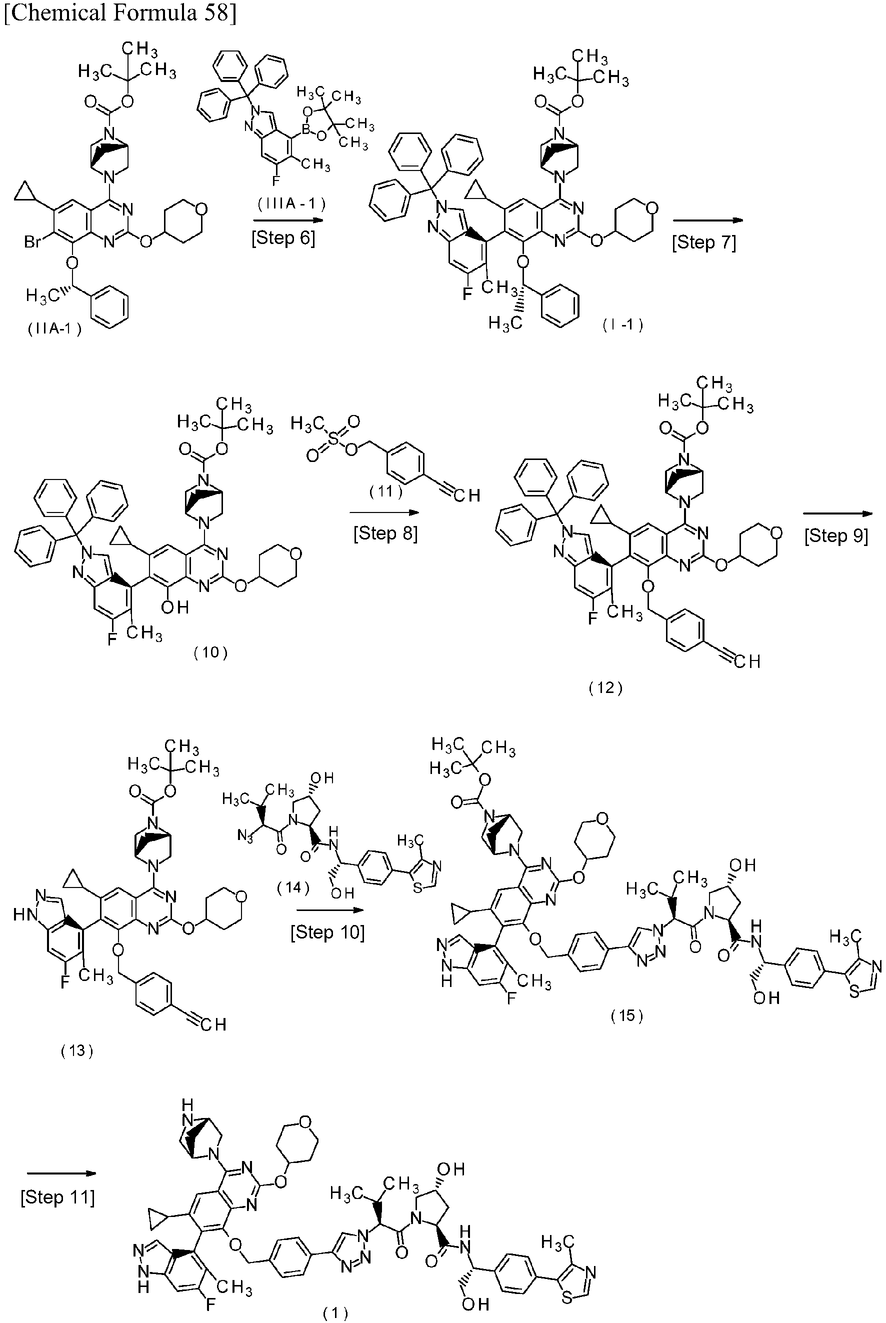

### (Step 7)

This step is a step of obtaining a compound of formula (10) by deprotecting the compound of formula (I-1) through a catalytic hydrogenation reaction. This reaction can be performed by stirring the compound of formula (I-1) under a hydrogen atmosphere, from under normal pressure to under increased pressure, in a solvent inactive for the reaction, in the presence of a metal catalyst, from under cooling to under heating, preferably at room temperature, for 1 hour to 5 days. Examples of the solvent include an alcohol such as MeOH, EtOH, or iPrOH, ethyl acetate, water, and a mixture thereof, but the solvent is not particularly limited. As a metal catalyst, although not particularly limited, a palladium catalyst or the like such as Pd/C or palladium black is used. Performing the reaction in the presence of an inorganic base, such as sodium hydrogen carbonate and potassium carbonate, is sometimes advantageous for smoothly promoting the reaction.

In one aspect, the reaction conditions described in Example 4 below can be used.

### (Step 8)

This step is a step of obtaining compound (12) by reacting compound (10) with compound (11). This reaction is performed by reacting a mixture of compound (10) and compound (11) in an equivalent amount or with one compound thereof in an excess equivalent amount in the presence of a base, in a solvent inactive for the reaction, from under cooling to under reflux with heat, preferably at 0°C to 80°C, generally for 0.1 hours to 5 days. Examples of the solvent used here include an ether such as diethyl ether, THF, DOX, or 1,2-dimethoxyethane, DMF, DMAc, and a mixture thereof, but the solvent is not particularly limited. Examples of the base include, but are not particularly limited to, an inorganic base such as potassium carbonate, cesium carbonate, or sodium hydride.

In one aspect, the reaction conditions described in Example 5 below can be used.

### (Step 9)

This step is a step of removing the protective group of NH (triphenylmethyl group) from compound (12) to obtain compound (13).

In this reaction, compound (12) and an acidic reagent are used in an equivalent amount or with one in an excess equivalent amount, and the resulting mixture is stirred in a solvent inactive for the reaction, from under cooling to under reflux with heat, preferably at room temperature, generally for 30 minutes to 1 hour. Examples of the acidic reagent used here include, but are not particularly limited to, 4-methylbenzene-1-sulfonic acid monohydrate. Examples of the solvent include an alcohol such as MeOH, EtOH, or iPrOH, but the solvent is not particularly limited.

In one aspect, the reaction conditions described in Example 6 below can be used.

### (Step 10)

This step is a step of obtaining compound (15) by a cycloaddition reaction of compound (13) and compound (14). In this reaction, compound (13) and compound (14) are used in an equivalent amount or with one compound thereof in an excess equivalent amount, and the mixture of the compounds is stirred preferably in the presence of a copper salt, more preferably in the presence of a copper salt and a reductant, in a solvent inactive for the reaction or with no solvent, from under cooling to under reflux with heat, preferably at 0°C to 100°C, generally for 0.1 hours to 5 days. Examples of the solvent used here include an ether such as diethyl ether, THF, DOX, or 1,2-dimethoxyethane, an alcohol such as MeOH, EtOH, iPrOH, or tBuOH, water, and a mixture thereof, but the solvent is not particularly limited. Examples of the copper salt include, but are not particularly limited to, copper(I) iodide, copper(II) sulfate, and copper(II) trifluoromethanesulfonate. Examples of the reductant include, but are not particularly limited to, sodium ascorbate.

In one aspect, the reaction conditions described in Example 11 below can be used.

### (Step 11)

This step is a step of removing the protective group of NH (tert-butoxycarbonyl group) from compound (15) to obtain compound (1). In this reaction, compound (15) and an acidic reagent are used in an equivalent amount or with one in an excess equivalent amount, and the resulting mixture is stirred in a solvent inactive for the reaction, from under cooling to under reflux with heat, preferably at room temperature to 60°C, generally for 30 minutes to 5 days. Examples of the acidic reagent used here include, but are not particularly limited to, methanesulfonic acid. Examples of the solvent include an alcohol such as MeOH, EtOH, iPrOH, or tBuOH, water, and a mixture thereof, but the solvent is not particularly limited.

In one aspect, the reaction conditions described in Example 12 below can be used.

In one aspect, compound (14), which is reacted in Step 10, can be obtained from compound (16) and compound (17) by Steps 12 to 16 described below.

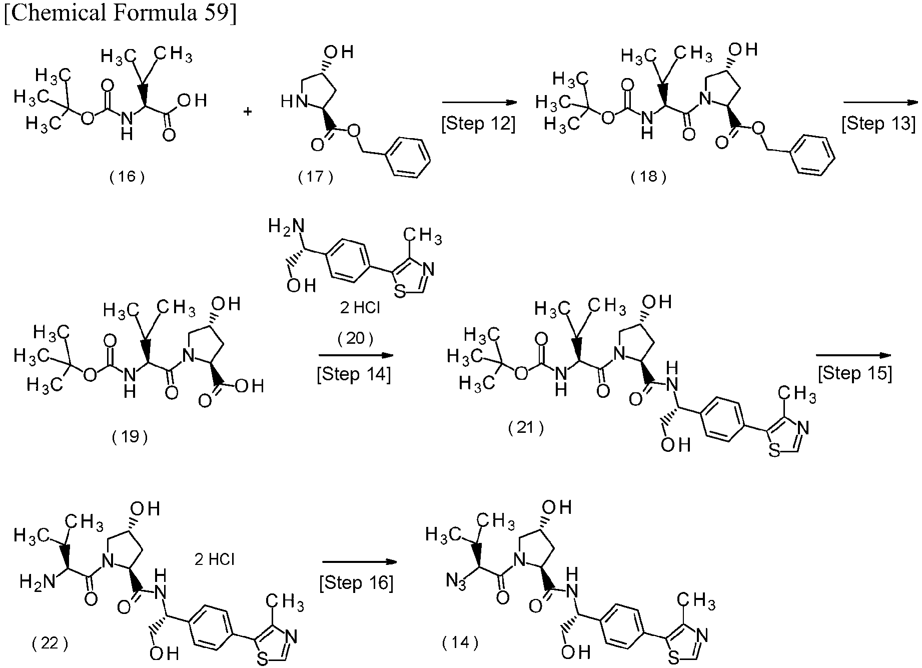

### (Step 12)

This step is a step of obtaining compound (18) by an amidation reaction of compound (16) and compound (17). In this reaction, compound (16) and compound (17) are used in an equivalent amount or with one compound thereof in an excess amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include an ether such as diethyl ether, THF, DOX, or 1,2-dimethoxyethane, DMF, and a mixture thereof, but the solvent is not particularly limited. Examples of the condensing agent include, but are not particularly limited to, HATU and EDCI. Use of an additive (for example, 1-hydroxybenzotriazole) is sometimes preferred for the reaction. Performing the reaction in the presence of an organic base, such as TEA and DIPEA, or an inorganic base, such as potassium carbonate, sodium carbonate, or potassium hydroxide, is sometimes advantageous for smoothly promoting the reaction.

In one aspect, the reaction conditions described in Example 8 below can be used.

### (Step 13)

This step is a step of obtaining compound (19) by deprotecting compound (18) through a catalytic hydrogenation reaction. The reaction conditions are the same as in Step 7.

In one aspect, the reaction conditions described in Example 9 below can be used.

### (Step 14)

This step is a step of obtaining compound (21) by an amidation reaction of compound (19) and compound (20). The reaction conditions are the same as in Step 12.

### (Step 15)

This step is a step of removing the protective group of NH (tert-butoxycarbonyl group) from compound (21) to obtain compound (22). This reaction is performed by stirring the compound from under cooling to under reflux with heat generally for 0.1 hours to 5 days. Examples of the solvent used here include, but are not particularly limited, an alcohol such as MeOH, EtOH, iPrOH, or tBuOH, ethyl acetate, and a mixture thereof. However, the solvent is not particularly limited. Examples of the deprotection reagent include, but are not particularly limited to, an acid such as hydrogen chloride (in DOX solution), hydrogen chloride (in ethyl acetate solution), trifluoroacetic acid, and methanesulfonic acid.

In one aspect, the reaction conditions of Steps 14 and 15 may employ the reaction conditions described in Example 10 below.

### (Step 16)

This step is a step of obtaining compound (14) by a reaction between compound (22) and a diazo-transfer reagent. In this reaction, compound (22) is treated with the diazo-transfer reagent in an equal amount or an excess amount in a solvent inactive for the reaction, from under cooling to under heating, preferably at 0°C to 50°C, generally for 0.1 hours to 3 days. Examples of the diazo-transfer reagent include, but are not particularly limited to, trifluoromethanesulfonyl azide, nonafluorobutanesulfonyl azide such as 1,1,2,2,3,3,4,4,4-nonafluoro-1-butanesulfonyl azide, imidazole-1-sulfonyl azide or a salt thereof, and ADMP. Performing the reaction in the presence of an organic base, such as TEA, DMAP, or 2,6-lutidine, and a catalytic amount of a copper salt, such as copper sulfate, is sometimes advantageous. Examples of the solvent include an ether such as THF, a halogenated hydrocarbon such as dichloromethane, an alcohol such as MeOH, MeCN, water, and a mixture thereof.

In one aspect, the reaction conditions of Step 16 may employ the reaction conditions described in Example 10-2 below.

In one aspect, a compound of formula (IIA-1), which is reacted in Step 6, can be obtained from compound (2) by Steps 1 to 5 described below.

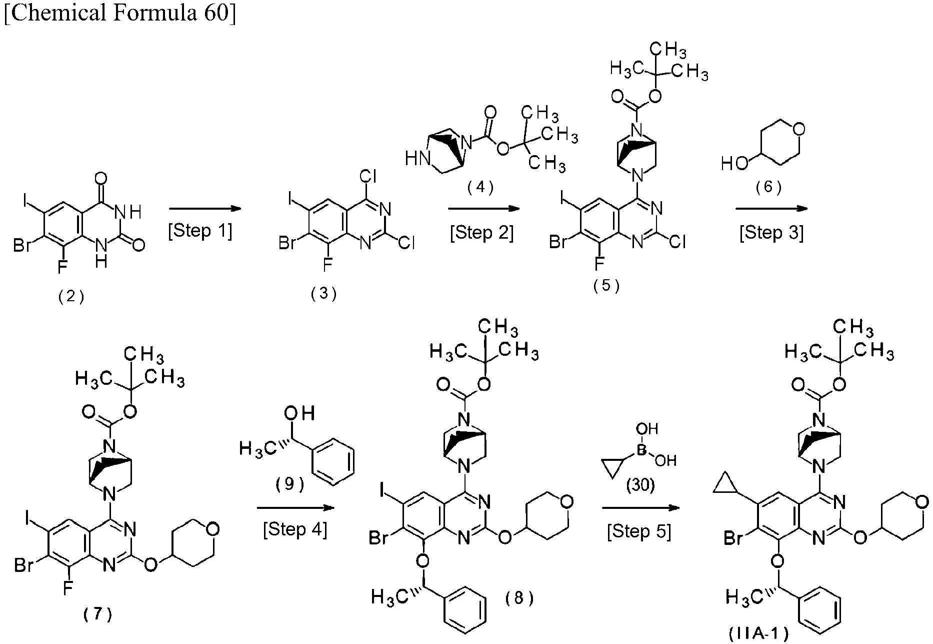

### (Step 1)

This step is a step of chlorinating compound (2) to obtain compound (3). In this reaction, compound (2) and a chlorinating reagent are used in an equivalent amount or with one in an excess equivalent amount, and the resulting mixture is stirred in the presence of a base, in a solvent inactive for the reaction or with no solvent, under reflux with heat, preferably at 90°C to 110°C, generally for 0.1 hours to 3 days. Examples of the chlorinating reagent used here include, but are not particularly limited to, phosphorus oxychloride. Examples of the base used here include, but are not particularly limited to, an organic base such as TEA or DIPEA. Examples of the solvent used here include DMF, but the solvent is not particularly limited.

### (Step 2)

This step is a step of obtaining compound (5) by an ipso substitution reaction between compound (3) and compound (4). In this reaction, compound (3) and compound (4) are used in an equivalent amount or with one compound thereof in an excess equivalent amount, and the mixture of the compounds is stirred in a solvent inactive for the reaction, or with no solvent, from under cooling to under reflux with heat, preferably at 0°C to 80°C, generally for 0.1 hours to 5 days. Examples of the solvent used here include, but are not particularly limited to, a halogenated hydrocarbon such as dichloromethane, 1,2-dichloroethane, or chloroform, an aromatic hydrocarbon such as benzene, toluene, or xylene, an ether such as diethyl ether, THF, DOX, or 1,2-dimethoxyethane, DMF, DMAc, DMSO, ethyl acetate, MeCN, and a mixture thereof. Performing the reaction in the presence of an organic base, such as TEA, DIPEA, NMM, DABCO, or tBuOK, or an inorganic base, such as sodium hydride, potassium carbonate, sodium carbonate, or cesium carbonate, is sometimes advantageous for smoothly promoting the reaction.

In one aspect, the reaction conditions described in International Publication No. WO 2022/173032 can be used.

### (Step 3)

This step is a step of obtaining compound (7) by an ipso substitution reaction between compound (5) and compound (6). The reaction conditions are the same as in Step 2.

In one aspect, the reaction conditions described in International Publication No. WO 2022/173032 can be used.

### (Step 4)

This step is a step of obtaining compound (8) by an ipso substitution reaction between compound (7) and compound (9). The reaction conditions are the same as in Step 2.

In one aspect, the reaction conditions described in Example 1 below can be used.

### (Step 5)

This step is a step of reacting compound (8) with cyclopropylboronic acid of compound (30) to obtain a compound of formula (IIA-1). In this reaction, compound (8) and the cyclopropylboronic acid of compound (30) are used in an equivalent amount or with one in an excess equivalent amount, and the resulting mixture is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, MeCN, water, and a mixture thereof. Examples of the base include inorganic bases, such as tripotassium phosphate, sodium carbonate, and potassium carbonate. Example of the palladium catalyst include, but are not particularly limited to, tetrakis(triphenylphosphine)palladium and [1,1'-bis(diphenylphosphino)ferrocene]palladium(II) dichloride·dichloromethane adduct. Performing the reaction in the presence of a ligand, such as DPPF, is sometimes advantageous for smoothly promoting the reaction. In addition, heating the mixture by microwave irradiation is sometimes advantageous for smoothly promoting the reaction.

In one aspect, the reaction conditions described in Example 2 below can be used.

In one aspect, the compound of formula (I-1) can be obtained from a compound of compound (2a) by Steps 1a to 5a to prepare a compound of formula (IIA-2), followed by Step 6a under the same reaction conditions as in Step 6.

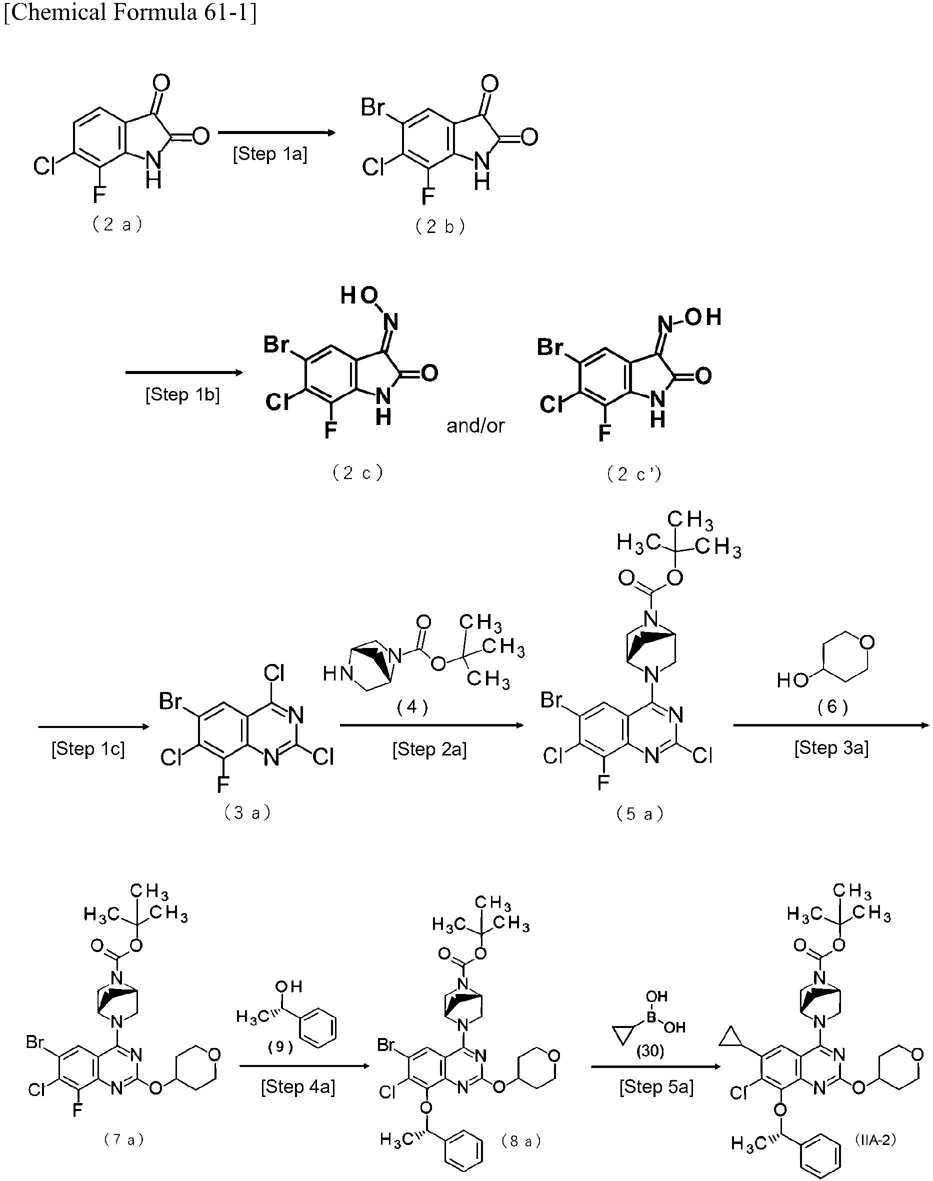

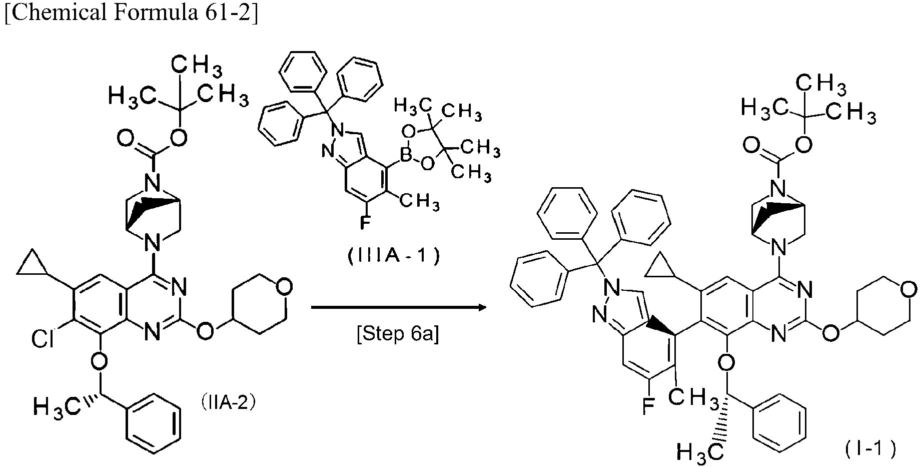

### (Step 1a)

This step is a step of brominating compound (2a) to obtain compound (2b). In this reaction, compound (2a) and brominating reagent are used in an equivalent amount or with one in an excess equivalent amount, and the resulting mixture is stirred in the presence of an acidic reagent, in a solvent inactive for the reaction, at 40°C to 70°C, generally for 1 hour to 5 days. Examples of the brominating reagent used here include, but are not particularly limited to, 1,3-dibromo-5,5-dimethylhydantoin and NBS. Examples of the acidic reagent used here include, but are not particularly limited to, methanesulfonic acid and sulfuric acid. Examples of the solvent used here include, but are not particularly limited to, an alcohol, such as MeOH, EtOH, iPrOH, or tBuOH, and MeCN.

In one aspect, the reaction conditions described in Example 14 below can be used in Step 1a.

### (Step 1b)

This step is a step of oximating compound (2b) to obtain compound (2c) and/or compound (2c'). In this reaction, compound (2b) and hydroxylamine hydrochloride are used in an equivalent amount or with one in an excess equivalent amount, and the resulting mixture is stirred in a solvent inactive for the reaction, from at room temperature to 60°C, generally for 0.5 hours to 5 days. Examples of the solvent used here include, but are not particularly limited to, an alcohol, such as MeOH, EtOH, iPrOH, or tBuOH. Performing the reaction in the presence of an additive, such as sodium acetate, is sometimes advantageous for smoothly promoting the reaction.

In one aspect, the reaction conditions described in Example 15 below can be used in Step 1b.

### (Step 1c)

This step is a step of subjecting compound (2c) and/or compound (2c') to ring-expansion reaction conditions to obtain compound (3a). In this reaction, compound (2c) and/or compound (2c') and a chlorinating reagent are used in an equivalent amount or with one in an excess equivalent amount, and the resulting mixture is stirred in the presence of triphenylphosphine oxide and a base, in a solvent inactive for the reaction, from at room temperature to 110°C, generally for 1 hour to 7 days. Examples of the chlorinating reagent used here include, but are not particularly limited to, thionyl chloride, phosphorus oxychloride, and triphosgene. Examples of the base used here include, but are not particularly limited to, DIPEA. Examples of the solvent used here include, but are not particularly limited to, an aromatic hydrocarbon, such as benzene, toluene, or xylene. Further, trialkylphosphine oxide or the like may be used instead of triphenylphosphine oxide, but are not limited thereto.

In one aspect, the reaction conditions described in Example 16 below can be used in Step 1c.

Steps 2a to 5a can each be carried out under the same reaction conditions as in Steps 2 to 5 above. Step 6a can be carried out under the same reaction conditions as in Step 6.

In one aspect, a compound of formula (IIA-2) can be obtained using the reaction conditions described in Examples 14 to 19 below, and thereafter a compound of formula (I-1) can be obtained using the reaction conditions described in Example 3.

In one aspect, compound (20), which is reacted in Step 14, can be obtained from compound (24) and compound (25) by Steps 17 and 18 described below.

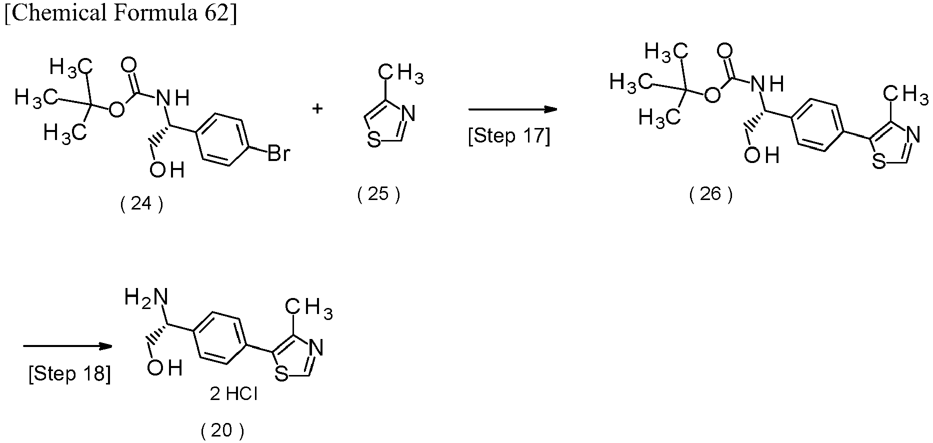

### (Step 17)

This step is a step of obtaining compound (26) by a reaction between compound (24) and compound (25). In this reaction, compound (24) and compound (25) are used in an equivalent amount or with one compound thereof in an excess equivalent amount, and the mixture of the compounds is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, an ether, such as diethyl ether, THF, DOX or 1,2-dimethoxyethane, NMP, DMF, DMAc, DMSO, MeCN, 1,3-dimethylimidazolidin-2-one, ethyl acetate, water, and a mixture thereof. Examples of the base include tripotassium phosphate, sodium carbonate, potassium carbonate, and potassium acetate. Examples of the palladium catalyst include tetrakis(triphenylphosphine)palladium, bis(triphenylphosphine)palladium(II) dichloride, [1,1'-bis(diphenylphosphino)ferrocene]palladium(II) dichloride·dichloromethane adduct, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, and palladium(II) acetate. In addition, heating the mixture by microwave irradiation is sometimes advantageous for smoothly promoting the reaction.

For example, the following can be referred as a reference about the reaction.
Synthesis 2020, 52, p. 2521-2527
PNAS 2016, 113, p. 7124-7129

### (Step 18)

This step is a step of removing the protective group of NH (tert-butoxycarbonyl group) in compound (26) to obtain a compound of formula (20). The reaction conditions are the same as in Step 15.

In one aspect, a compound of formula (IIIA-1), which is reacted in Step 6, can be obtained from compound (27) by Steps 19 to 21 described below.

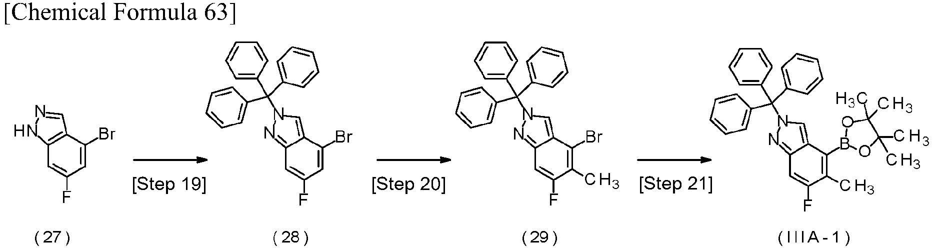

### (Step 19)

This step is a step of protecting NH in compound (27) with a triphenylmethyl group to obtain compound (28). As long as the reaction proceeds, the reaction conditions are not particularly limited. For example, the reaction may be carried out in accordance with the method described in Production Example 245 of PTL 1.

### (Step 20)

This step is a step of methylating compound (28) to obtain compound (29). As long as the reaction proceeds, the reaction conditions are not particularly limited. For example, the reaction may be carried out in accordance with the method described in Production Example 246 of PTL 1.

### (Step 21)

This step is a step of substituting Br in compound (29) with a boronic acid pinacol ester to obtain a compound of formula (IIIA-1). As long as the reaction proceeds, the reaction conditions are not particularly limited. For example, the reaction may be carried out in accordance with the method described in Production Example 247 of PTL 1.

### (Steps 3-2 to 6-2)

In one aspect, the present invention is the method for producing a compound of formula (I) or a salt thereof according to [1], the method including reacting a compound of formula (IIA) or a salt thereof with a compound of formula (IIIA) or a salt thereof,
the compound of formula (I) is a compound represented by formula (I-2), 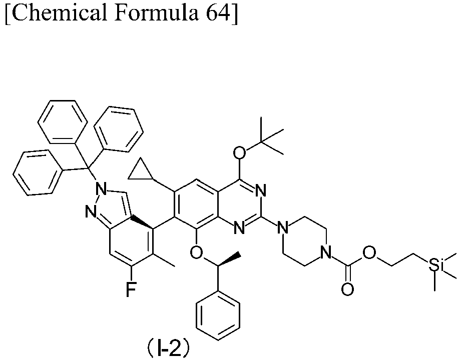
the compound of formula (IIA) is a compound represented by formula (IIA-3): 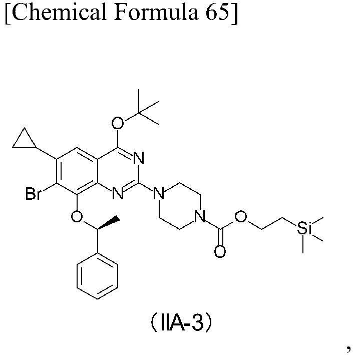
the method including obtaining a compound represented by formula (I-2) by the following step.

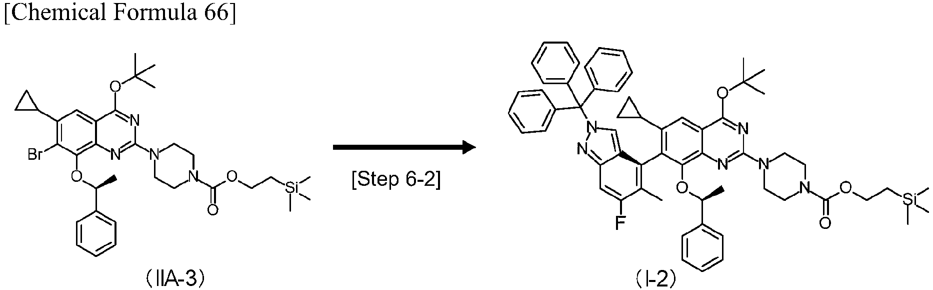

In one aspect, the compound of formula (IIIA) is the compound of formula (IIIA-1).

As described above, Step 6-2 is a method of reacting the compound of formula (IIA-3) with the compound of formula (IIIA) to produce a compound of formula (I-2). As long as the reaction proceeds, the reaction conditions are not particularly limited. In one aspect, the conditions are the same as conditions for reacting the compound of formula (IIA) or a salt thereof with the compound of formula (IIIA) or a salt thereof as described above. In one aspect, the reaction conditions described in Example 35 below can be used.

In one aspect, the compound of formula (IIA-3), which is reacted in Step 6-2, can be obtained from compound (5-2) by steps described below.

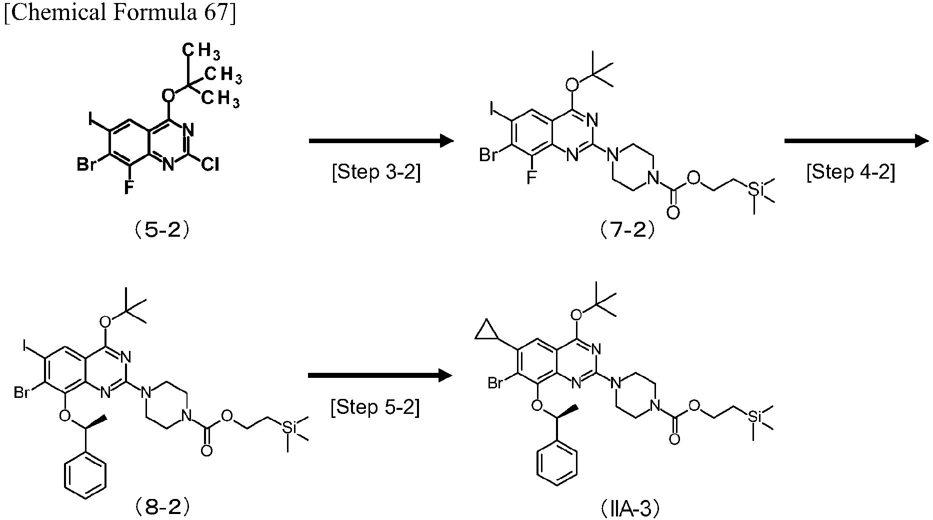

### (Step 3-2)

This step is a step of obtaining compound (7-2) from compound (5-2). The reaction conditions are the same as in Step 3. In one aspect, the reaction conditions described in Example 36 below can be used.

### (Step 4-2)

This step is a step of obtaining compound (8-2) from compound (7-2). The reaction conditions are the same as in Step 4. In one aspect, the reaction conditions described in Example 37 below can be used.

### (Step 5-2)

This step is a step of obtaining compound (IIA-3) from compound (8-2). The reaction conditions are the same as in Step 4. In one aspect, the reaction conditions described in Example 38 below can be used.

### 3. Diazo-Transfer Reaction

The present invention, in one aspect, is a method for producing a compound of formula (#1) or a salt thereof: 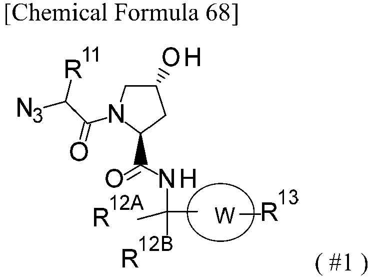 in which
R¹¹ is optionally substituted C₁₋₆ alkyl, optionally substituted C₃₋₆ cycloalkyl, or an optionally substituted 4- to 6-membered saturated heterocyclic group containing one heteroatom selected from the group consisting of oxygen, sulfur, and nitrogen,
R^{12A} and R^{12B}, which are the same as or different from each other, are H or optionally substituted C₁₋₆ alkyl, or R^{12A} and R^{12B}, together with the carbon to which they are attached, form optionally substituted C₃₋₆ cycloalkyl, or an optionally substituted 4- to 6-membered saturated heterocyclic ring containing one heteroatom selected from the group consisting of oxygen, sulfur, and nitrogen,
R¹³ is H; halogen; C₁₋₃ alkyl; -SO₂CH₃; C₃₋₆ cycloalkyl; an optionally substituted 4-to 6-membered saturated heterocyclic group containing 1 or 2 heteroatoms selected from the group consisting of oxygen, sulfur and nitrogen; optionally substituted 5-membered heteroaryl containing 1 to 4 heteroatoms selected from the group consisting of oxygen, sulfur and nitrogen; or 6-membered heteroaryl containing 1 to 3 nitrogen atoms, and
W is optionally substituted phenyl or optionally substituted 6-membered heteroaryl containing 1 to 3 nitrogen atoms,
the method including reacting a compound of formula (#2) with a diazo-transfer reagent to obtain the compound of formula (#1) or a salt thereof: 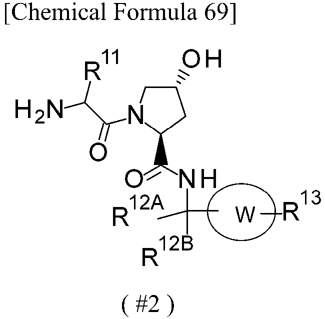 in which
   R¹¹, R^{12A}, R^{12B}, R¹³, and W are as defined above.

In one aspect, in a method for producing the compound of formula (#1) or a salt thereof,
R¹¹ is ethyl, isopropyl, tert-butyl, or C₃₋₆ cycloalkyl,
R^{12A} and R^{12B}, which are the same as or different from each other, are H or C₁₋₃ alkyl optionally substituted with a group selected from the group consisting of F, OH, and N(CH₃)₂, or R^{12A} and R^{12B}, together with the carbon to which they are attached, form cyclopropyl,
R¹³ is H, halogen, or a group selected from the group consisting of: 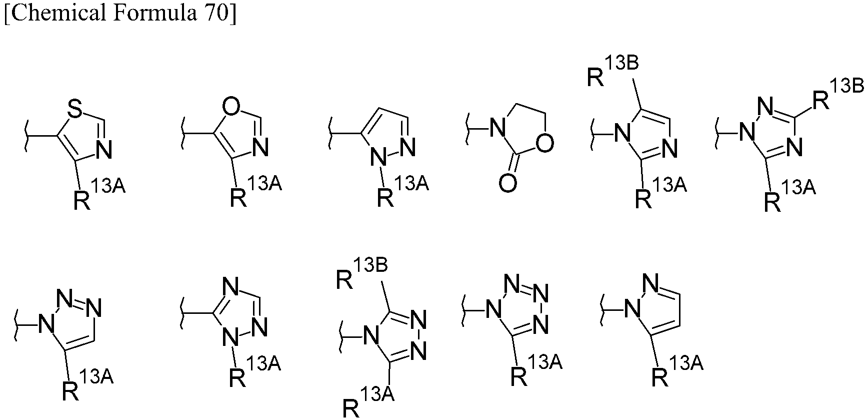
R^{13A} and R^{13B}, which are the same as or different from each other, are H or C₁₋₃ alkyl optionally substituted with OH, and
W is phenyl.

In one aspect, in the method for producing the compound of formula (#1) or a salt thereof,
R¹¹ is isopropyl,
R^{12A} is H,
R^{12B} is C₁₋₃ alkyl optionally substituted with OH,
R¹³ is a group below, 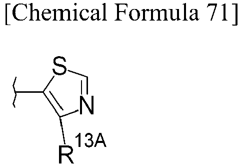
R^{13A} is C₁₋₃ alkyl, and
W is phenyl.

As long as the diazo-transfer reaction proceeds, the type of diazo-transfer reagent is not limited. For example, perfluoroalkylsulfonyl azide, imidazole-1-sulfonyl azide, or a salt thereof may be used. The perfluoroalkylsulfonyl azide is, in one aspect, nonafluorobutanesulfonyl azide, heptafluoropropanesulfonyl azide, pentafluoroethanesulfonyl azide, or trifluoromethanesulfonyl azide; in one aspect, is nonafluorobutanesulfonyl azide; and, in one aspect, is 1,1,2,2,3,3,4,4,4-nonafluoro-1-butanesulfonyl azide as one form of nonafluorobutanesulfonyl azide.

In one aspect, a basic reagent may be used in the diazo-transfer reaction. Examples of the basic reagent include an inorganic base such as potassium hydrogen carbonate or potassium carbonate.

In one aspect, an additive may be used as appropriate in the diazo-transfer reaction to promote the reaction. Examples of the additive include metal salts such as a copper salt including CuSO₄ and a zinc salt including ZnSO₄.

In one aspect, a polar solvent may be used as the solvent in the diazo-transfer reaction. Examples of the polar solvent include, water, acetonitrile, an alcohol such as methanol or ethanol, a polar organic solvent such as DMSO or MTBE, and a mixture thereof.

In one aspect, the diazo-transfer reaction can be performed at 10°C to 25°C. In one aspect, the diazo-transfer reaction can be performed at 10°C to 20°C, for example, at 15°C.

In one aspect, the diazo-transfer reaction may be performed in the presence of a metal catalyst or in the absence of a metal catalyst.

### 4. Compound

The present invention is, in one aspect, a compound represented by any one of the following formulas or a salt thereof. The compound or a salt thereof may be obtained by the method for producing the compound of formula (I) or a salt thereof described above, or in each of Steps 7 to 10 described above.

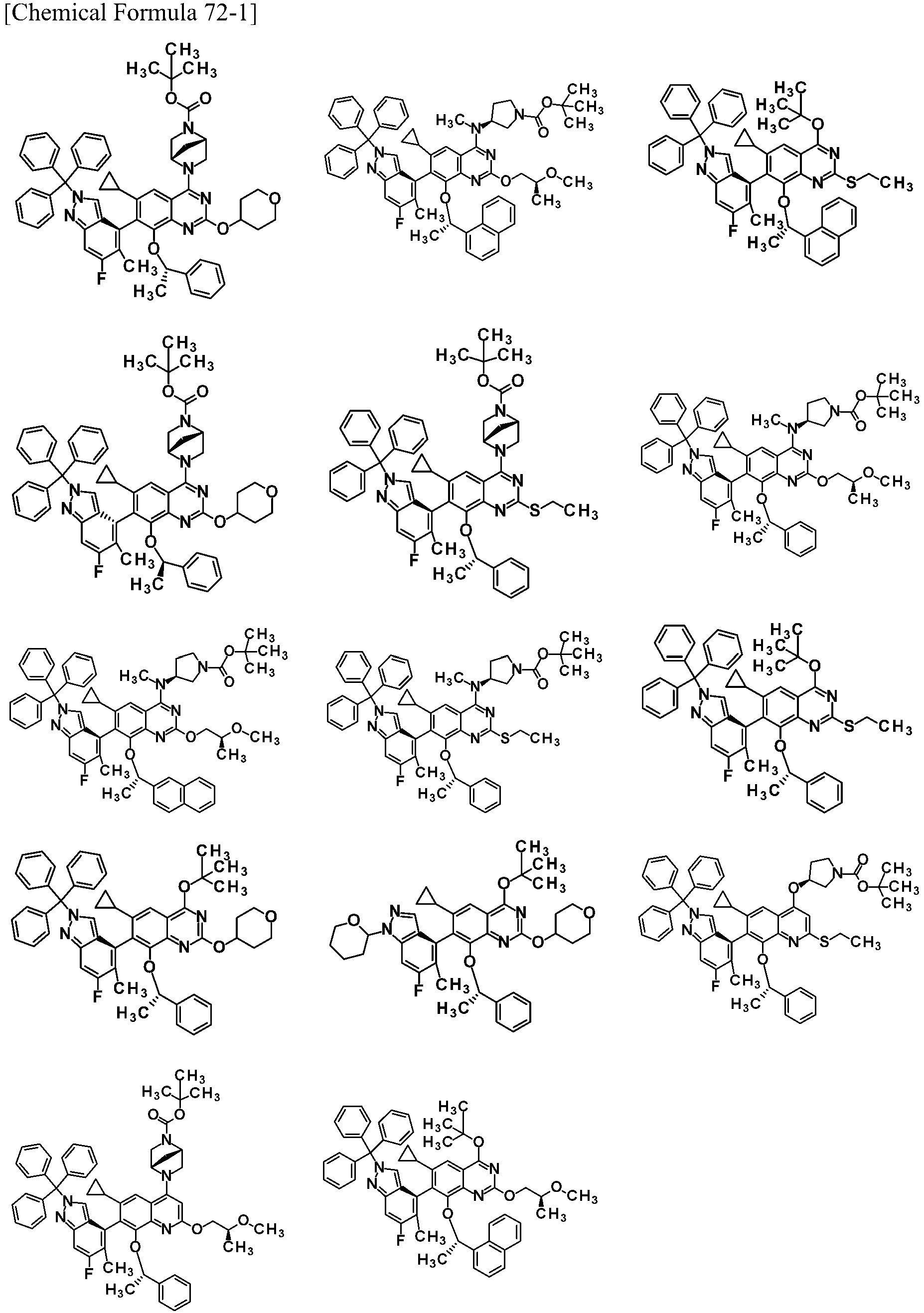

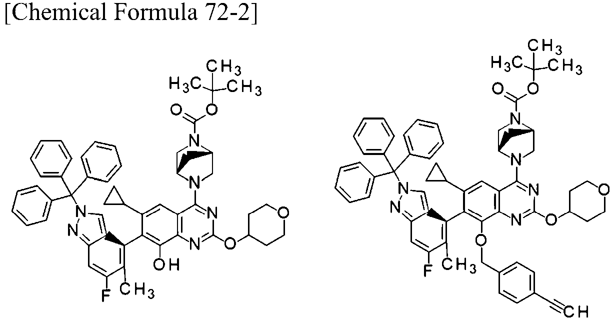

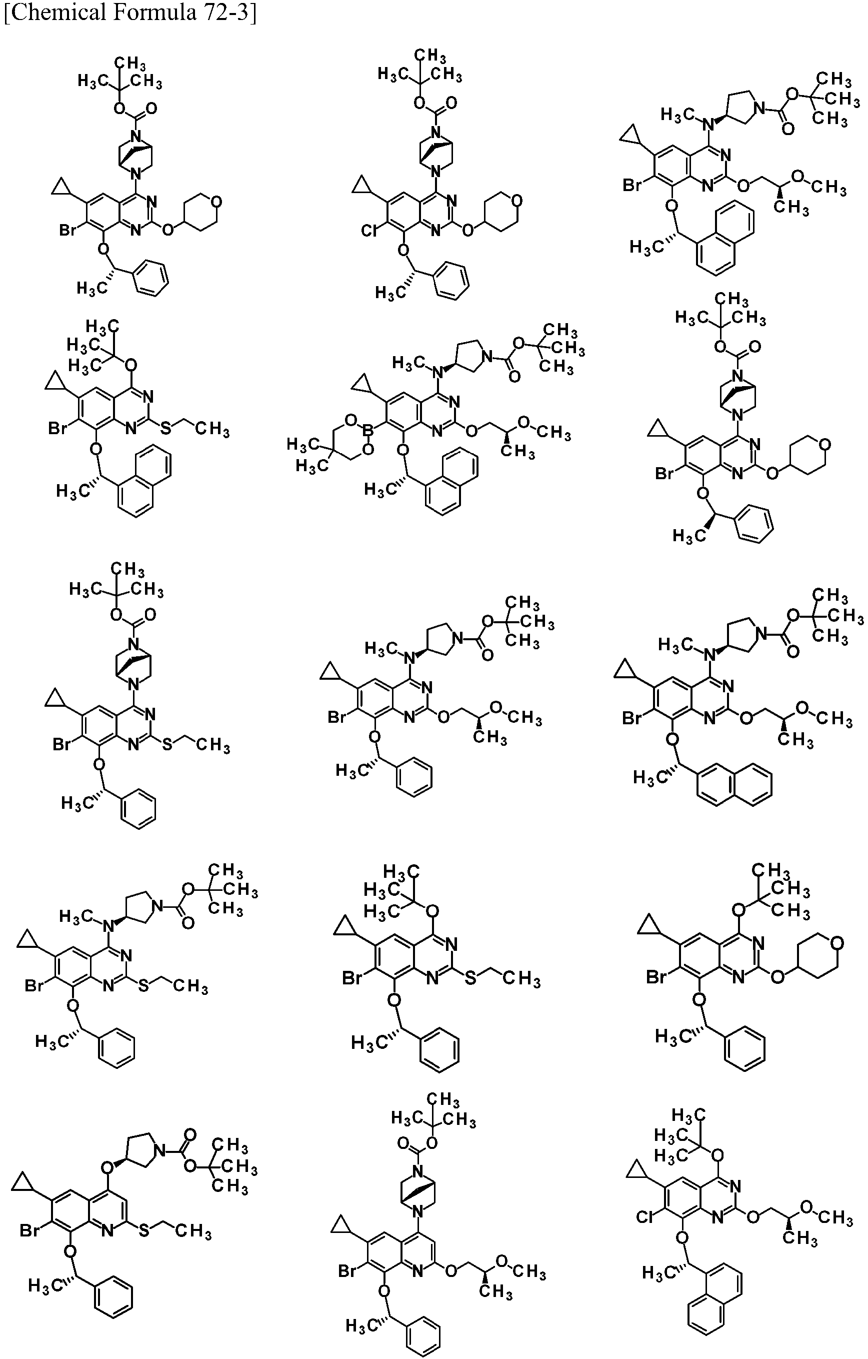

The present invention is, in one aspect, compound (3a) obtained in Step 1c or a salt thereof.

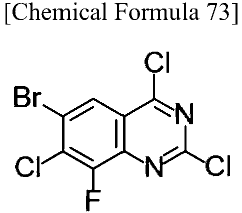

The present invention, in one aspect, is the compound (7-2), (8-2), (IIA-3), or (I-2), or a salt thereof.

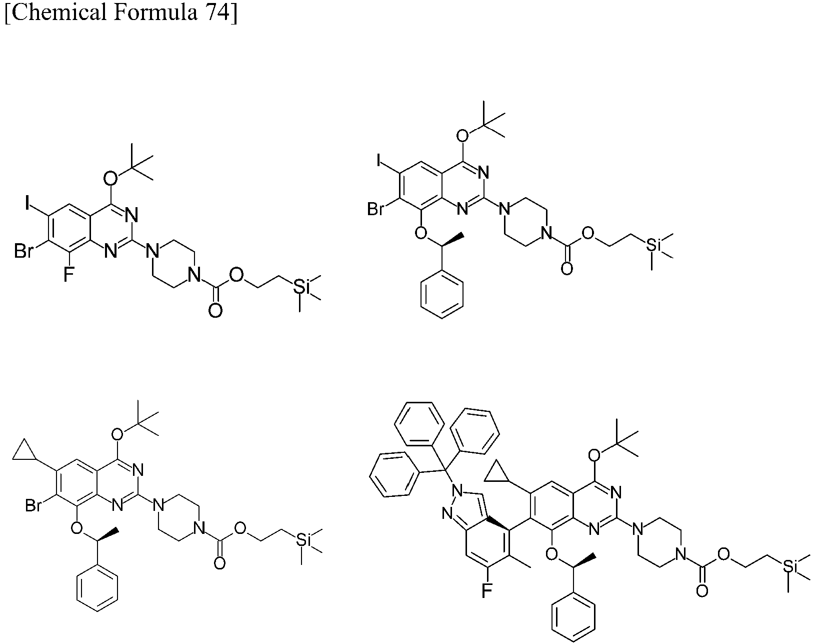

### 4. Abbreviation

In the present specification, the following abbreviations are sometimes used.

TFA: trifluoroacetic acid, DMF: N,N-dimethylformamide, THF: tetrahydrofuran, MeCN: acetonitrile, MeOH: methanol, EtOH: ethanol, iPrOH: isopropyl alcohol, tBuOH: tert-butanol, iPr₂O: diisopropyl ether, DOX: 1,4-dioxane, DMSO: dimethyl sulfoxide, TEA: triethylamine, DIPEA: N,N-diisopropylethylamine, tBuOK: potassium tert-butoxide, tBuONa: sodium tert-butoxide, PdCl₂(dppf)·CH₂Cl₂: [1,1'-bis(diphenylphosphino)ferrocene]palladium(II) dichloride·dichloromethane adduct, Pd/C: palladium carbon, HATU: 1-[bis(dimethylamino)methylene]-1H-1,2,3-triazolo[4,5-b]pyridinium 3-oxide hexafluorophosphate, DABCO: 1,4-diazabicyclo[2.2.2]octane, SPhos: dicyclohexyl(2',6'-dimethoxy-[1,1'-biphenyl]-2-yl)phosphine, RuPhos: dicyclohexyl(2',6'-diisopropoxy-[1,1'-biphenyl]-2-yl)phosphine, SPhos Pd G2: chloro(2-dicyclohexylphosphino-2',6'-dimethoxy-1,1'-biphenyl)[2-(2'-amino-1,1'-biphenyl)]palladium(II), RuPhos Pd G2: chloro(2-dicyclohexylphosphino-2',6'-diisopropoxy-1,1'-biphenyl)[2-(2'-amino-1,1'-biphenyl)]palladium(II), SPhos Pd G3: (2-dicyclohexylphosphino-2',6'-dimethoxy-1,1'-biphenyl)[2-(2'-amino-1,1'-biphenyl)]palladium(II) methanesulfonate, RuPhos Pd G3: (2-dicyclohexylphosphino-2',6'-diisopropoxy-1,1'-biphenyl)[2-(2'-amino-1,1'-biphenyl)]palladium(II) methanesulfonate, DPPF: 1,1'-bis(diphenylphosphino)ferrocene, WSC: 1-(3-dimethylaminopropyl)-3-ethylcarbodiimide, EDCI: 1-(3-dimethylaminopropyl)-3-ethylcarbodiimide, Pd(PPh₃)₄: tetrakis(triphenylphosphine)palladium(0), DMAc: dimethylacetamide, NMP: N-methyl-2-pyridone, ADMP: 2-azido-1,3-dimethylimidazolinium hexafluorophosphate, DMAP: 4-dimethylaminopyridine, NMM: N-methylmorpholine, NBS: N-bromosuccinimide, and MTBE: methyl t-butyl ether.

In the tables presented below, the following abbreviations are sometimes used:
Ex: Example No., Syn: Example No. where a compound prepared in the same manner (for example, "Syn: 9" indicates that the compound is prepared in the same manner as Example 9), DATA: physicochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H NMR spectrum (500 MHz) measured in DMSO-d₆ at 27°C, NMR (100°C): δ value (ppm) of peak in ¹H NMR spectrum (500 MHz) measured in DMSO-d₆ at 100°C, s: singlet (spectrum), d: doublet (spectrum), dd: double doublet (spectrum), ddd: double double doublet (spectrum), t: triplet (spectrum), dt: double triplet (spectrum), q: quartet (spectrum), m: multiplet (spectrum), br: broad signal (spectrum) (e.g., br s).

### EXAMPLES

The present invention is described in more detail below with reference to specific examples, but the present invention is not limited to the specific examples described below. Unless otherwise specified in this specification, concentrations and the like are based on mass, and numerical ranges include the endpoints.

### Example 1

To a mixture of tert-butyl (1S,4S)-5-{7-bromo-8-fluoro-6-iodo-2-[(oxan-4-yl)oxy]quinazolin-4-yl}-2,5-diazabicyclo[2.2.1]heptane-2-carboxylate (54.8 g), (1S)-1-phenylethan-1-ol (12.4 g), and THF (400 mL) was added tBuOK (11.3 g) in several portions under a nitrogen atmosphere under ice-bath cooling, while maintaining the internal temperature 10°C or lower, followed by stirring for 30 minutes under ice-bath cooling. The reaction was quenched with saturated aqueous ammonium chloride solution under ice-bath cooling. Water and ethyl acetate were added to separate the organic layer and the aqueous layer, and the aqueous layer was extracted twice with ethyl acetate. The combined organic layer was washed with saturated aqueous sodium chloride solution and dried over magnesium sulfate. The mixture was filtered and concentrated under reduced pressure, and hexane/iPr₂O (1/1, 400 mL) was added to the residue to form a suspension. The resulting solid was collected by filtration and dried under reduced pressure to give tert-butyl (1S,4S)-5-{7-bromo-6-iodo-2-[(oxan-4-yl)oxy]-8-[(1S)-1-phenylethoxy]quinazolin-4-yl}-2,5-diazabicyclo[2.2.1]heptane-2-carboxylate (52.3 g) as a solid.

### Example 2

Under a nitrogen atmosphere, tert-butyl (1S,4S)-5-{7-bromo-6-iodo-2-[(oxan-4-yl)oxy]-8-[(1S)-1-phenylethoxy]quinazolin-4-yl}-2,5-diazabicyclo[2.2.1]heptane-2-carboxylate (52.3 g) was suspended in MeCN (1000 mL) at room temperature, followed by addition of cyclopropylboronic acid (10 g), PdCl₂(dppf)·CH₂Cl₂ (5.6 g), tripotassium phosphate (55 g), and water (200 mL) at room temperature. The mixture was stirred at 90°C overnight. The reaction mixture was allowed to cool to room temperature and concentrated under reduced pressure until the volume was reduced to about half, followed by addition of ethyl acetate, and the mixture was filtered through Celite(R). Water was added to the filtrate, and the mixture was extracted twice with ethyl acetate. The combined organic layer was washed with saturated aqueous sodium chloride solution. Amino-modified silica gel (25 g) and activated carbon (25 g) were added to the organic layer, and the mixture was stirred at room temperature for 1 hour and then filtered through Celite(R). The filtrate was concentrated under reduced pressure. iPrOH (250 mL) was added to the residue for trituration, and the mixture was stirred at 90°C for 3 hours and then gradually cooled to room temperature with stirring. The resulting powder was collected by filtration and dried under reduced pressure to give tert-butyl (1S,4S)-5-{7-bromo-6-cyclopropyl-2-[(oxan-4-yl)oxy]-8-[(1S)-1-phenylethoxy]quinazolin-4-yl}-2,5-diazabicyclo[2.2.1]heptane-2-carboxylate (32.3 g) as a solid.

### Example 3

At room temperature, tert-butyl (1S,4S)-5-{7-bromo-6-cyclopropyl-2-[(oxan-4-yl)oxy]-8-[(1S)-1-phenylethoxy]quinazolin-4-yl}-2,5-diazabicyclo[2.2.1]heptane-2-carboxylate (1 g) and 6-fluoro-5-methyl-4-(4,4,5,5-tetramethyl-1,3,2-dioxaborolan-2-yl)-2-(triphenylmethyl)-2H-indazole (1 g) were dissolved in DOX (20 mL) and water (4 mL), followed by the addition of palladium(II) acetate (35 mg), anhydrous barium hydroxide (775 mg), and SPhos (125 mg). The mixture was stirred at 50°C overnight under an argon atmosphere, followed by the addition of 6-fluoro-5-methyl-4-(4,4,5,5-tetramethyl-1,3,2-dioxaborolan-2-yl)-2-(triphenylmethyl)-2H-indazole (200 mg) at the same temperature, and the mixture was further stirred for 4 hours. After the reaction mixture was allowed to cool, ethyl acetate and Celite(R) were added thereto, and the mixture was stirred at room temperature for 30 minutes, followed by filtration. The filtrate was washed with saturated aqueous sodium chloride solution. Amino-modified silica gel was added to the organic layer, and the mixture was stirred at room temperature for 30 minutes, followed by filtration and concentration. The resulting residue was dissolved in MeOH (40 mL) and stirred at 50°C for 1 hour and then at room temperature for 2 hours. The resulting solid was collected by filtration, washed with MeOH, and dried under reduced pressure to give tert-butyl (1S,4S)-5-{(7M)-6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (913 mg) as a solid.

### Example 3-1

At room temperature, tert-butyl (1S,4S)-5-{7-bromo-6-cyclopropyl-2-[(oxan-4-yl)oxy]-8-[(1S)-1- phenylethoxy]quinazolin-4-yl}-2,5-diazabicyclo[2.2.1]heptane-2-carboxylate (300 mg), 6-fluoro-5-methyl-4-(4,4,5,5-tetramethyl-1,3,2-dioxaborolan-2-yl)-2-(triphenylmethyl)-2H-indazole (300 mg), RuPhos Pd G3 (38 mg), RuPhos (21 mg), tripotassium phosphate (475 mg), DOX (5 mL), and water (1 mL) were mixed together and stirred at 90°C for 2 hours under an argon atmosphere. The reaction mixture was allowed to cool, followed by the addition of water and ethyl acetate, and the mixture was filtered through Celite(R) and washed with ethyl acetate. The filtrate was separated, and the aqueous layer was extracted with ethyl acetate. The combined organic layer was dried over magnesium sulfate, filtered, and concentrated under reduced pressure. The residue was purified by silica gel column chromatography (amino-modified silica gel, hexane/ethyl acetate) to give tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (367 mg) as a foam-like solid.

### Example 3-2

At room temperature, tert-butyl (1S,4S)-5-{7-bromo-6-cyclopropyl-2-[(oxan-4-yl)oxy]-8-[(1S)-1-phenylethoxy]quinazolin-4-yl}-2,5-diazabicyclo[2.2.1]heptane-2-carboxylate (200 mg), 6-fluoro-5-methyl-4-(4,4,5,5-tetramethyl-1,3,2-dioxaborolan-2-yl)-2-(triphenylmethyl)-2H-indazole (200 mg), RuPhos Pd G3 (25 mg), RuPhos (14 mg), barium hydroxide octahydrate (285 mg), DOX (3 mL), and water (0.6 mL) were mixed together and stirred at 90°C for 2 hours under an argon atmosphere. The reaction mixture was allowed to cool, and the mixture was filtered through Celite(R) while being washed with ethyl acetate. Water was added to the filtrate, and the mixture was extracted twice with ethyl acetate. The combined organic layer was washed with saturated aqueous sodium chloride solution, dried over magnesium sulfate, filtered, and concentrated under reduced pressure. The residue was purified by silica gel column chromatography (amino-modified silica gel, hexane/ethyl acetate) to give tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (291 mg) as a foam-like solid.

### Example 3-3

At room temperature, tert-butyl (1S,4S)-5-{7-bromo-6-cyclopropyl-2-[(oxan-4-yl)oxy]-8-[(1S)-1-phenylethoxy]quinazolin-4-yl}-2,5-diazabicyclo[2.2.1]heptane-2-carboxylate (200 mg), 6-fluoro-5-methyl-4-(4,4,5,5-tetramethyl-1,3,2-dioxaborolan-2-yl)-2-(triphenylmethyl)-2H-indazole (200 mg), palladium(II) acetate (7 mg), RuPhos (28 mg), barium hydroxide octahydrate (285 mg), DOX (3 mL), and water (0.6 mL) were mixed together and stirred at 50°C for 13 hours under an argon atmosphere. After the reaction mixture was allowed to cool, ethyl acetate and Celite(R) were added thereto, and the mixture was stirred at room temperature for 30 minutes, followed by filtration through Celite(R) to concentrate the filtrate. The residue was purified by silica gel column chromatography (amino-modified silica gel, hexane/ethyl acetate) to give tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (268 mg) as a foam-like solid.

### Example 3-4

At room temperature, tert-butyl (1S,4S)-5-{7-bromo-6-cyclopropyl-2-[(oxan-4-yl)oxy]-8-[(1S)-1-phenylethoxy]quinazolin-4-yl}-2,5-diazabicyclo[2.2.1]heptane-2-carboxylate (200 mg), 6-fluoro-5-methyl-4-(4,4,5,5-tetramethyl-1,3,2-dioxaborolan-2-yl)-2-(triphenylmethyl)-2H-indazole (200 mg), palladium(II) acetate (7 mg), SPhos (25 mg), anhydrous barium hydroxide (155 mg), DOX (3 mL), and water (0.6 mL) were mixed together and stirred at 50°C for 14 hours under an argon atmosphere. After the reaction mixture was allowed to cool, ethyl acetate and Celite(R) were added thereto, and the mixture was stirred at room temperature for 30 minutes, followed by filtration through Celite(R) to concentrate the filtrate. The residue was purified by silica gel column chromatography (amino-modified silica gel, hexane/ethyl acetate) to give tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (281 mg) as a foam-like solid.

### Example 4

A mixture of tert-butyl (1 S,4S)-5-1(7M)-6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (4.96 g), 10% Pd/C (about 50% water content, 1 g), sodium hydrogen carbonate (2 g), ethyl acetate (80 mL), and MeOH (20 mL) was stirred overnight at ambient temperature and atmospheric pressure under a hydrogen atmosphere. After purging with argon, the reaction liquid was filtered through Celite(R), and the filtrate was concentrated under reduced pressure to give tert-butyl (1S,4S)-5-{(7M)-6-cyclopropyl-7-[6-fluoro-5-methyl-2-(triphenylmethyl)-2H-indazol-4-yl]-8-hydroxy-2-[(oxan-4-yl)oxy]quinazolin-4-yl}-2,5-diazabicyclo[2.2.1]heptane-2-carboxylate (4.46 g) as a foam-like solid.

### Example 5

Under a nitrogen atmosphere, tert-butyl (1S,4S)-5-{(7M)-6-cyclopropyl-7-[6-fluoro-5-methyl-2-(triphenylmethyl)-2H-indazol-4-yl]-8-hydroxy-2-[(oxan-4-yl)oxy]quinazolin-4-yl}-2,5-diazabicyclo[2.2.1]heptane-2-carboxylate (34 g) and cesium carbonate (38 g) were suspended in DMF (200 mL), followed by the addition of (4-ethynylphenyl)methyl methanesulfonate (8.2 g) at room temperature, and the mixture was stirred at room temperature for 2 hours. The reaction mixture was added to ice water (about 1000 mL) and stirred at room temperature for 30 minutes. The resulting solid was collected by filtration and dried under reduced pressure to give tert-butyl (1S,4S)-5-{(7M)-6-cyclopropyl-8-[(4-ethynylphenyl)methoxy]-7-[6-fluoro-5-methyl-2-(triphenylmethyl)-2H-indazol-4-yl]-2-[(oxan-4-yl)oxy]quinazolin-4-yl}-2,5-diazabicyclo[2.2.1]heptane-2-carboxylate (35.5 g) as a solid.

### Example 6

To a solution of tert-butyl (1S,4S)-5-{(7M)-6-cyclopropyl-8-[(4-ethynylphenyl)methoxy]-7-[6-fluoro-5-methyl-2-(triphenylmethyl)-2H-indazol-4-yl]-2-[(oxan-4-yl)oxy]quinazolin-4-yl}-2,5-diazabicyclo[2.2.1]heptane-2-carboxylate (745 mg) in MeOH (8 mL) was added 4-methylbenzene-1-sulfonic acid monohydrate (145 mg) at room temperature and stirred at room temperature for 1 hour. Saturated aqueous sodium hydrogen carbonate solution was added to the reaction mixture, and the mixture was extracted twice with ethyl acetate. The combined organic layer was washed with saturated aqueous sodium chloride solution, dried over sodium sulfate, filtered, and then concentrated. The resulting solid was dissolved in ethyl acetate (3 mL)/hexane (3 mL), followed by the addition of hexane (3 mL), and the mixture was stirred at 90°C for 30 minutes and then at room temperature for 1 hour. The resulting solid was collected by filtration and dried under reduced pressure to give tert-butyl (1S,4S)-5-{(7M)-6-cyclopropyl-8-[(4-ethynylphenyl)methoxy]-7-(6-fluoro-5-methyl-1H-indazol-4-yl)-2-[(oxan-4-yl)oxy]quinazolin-4-yl}-2,5-diazabicyclo[2.2.1]heptane-2-carboxylate (408 mg) as a solid.

### Example 7

Under ice-bath cooling, (4-ethynylphenyl)methanol (10 g) was dissolved in CH₂Cl₂ (100 mL), and DIPEA (33 mL) and methanesulfonic anhydride (15.3 g) were added portionwise, and the mixture was stirred for 1 hour under ice-bath cooling. Methanesulfonic anhydride (3 g) was then added under ice-bath cooling, and the mixture was stirred for 30 minutes. Water and ethyl acetate/hexane (1/1) were added under ice-bath cooling, and the mixture was extracted twice with ethyl acetate/hexane (1/1). The combined organic layer was washed with saturated aqueous ammonium chloride solution, water, and saturated aqueous sodium chloride solution, dried over magnesium sulfate, filtered, and concentrated to give (4-ethynylphenyl)methyl methanesulfonate (16.0 g) as a solid.

### Example 8

To a mixture of N-(tert-butoxycarbonyl)-L-valine (30.8 g), (4R)-4-hydroxy-L-proline benzyl ester hydrochloride (35 g), THF (200 mL), DMF (200 mL), and DIPEA (70 mL) was added HATU (53.9 g) portionwise under ice-bath cooling (while maintaining the internal temperature 10°C or lower). The mixture was stirred for 15 minutes under ice-bath cooling and then for 45 minutes at room temperature. A semi-saturated aqueous sodium chloride solution (900 mL) was added at room temperature, and the mixture was extracted twice with ethyl acetate. The combined organic layer was washed successively with saturated aqueous sodium hydrogen carbonate solution and saturated aqueous sodium chloride solution, then dried over anhydrous magnesium sulfate. The insoluble materials were removed by filtration, and the filtrate was concentrated under reduced pressure to give N-(tert-butoxycarbonyl)-L-valyl-(4R)-4-hydroxy-L-proline benzyl ester (107 g, containing impurities) as an oil.

### Example 9

To a solution in MeOH (400 mL) of N-(tert-butoxycarbonyl)-L-valyl-(4R)-4-hydroxy-L-proline benzyl ester (107 g, containing impurities) obtained in Example 8 was added, under a nitrogen atmosphere at room temperature, 10% Pd/C (about 50% water content, 2.9 g). The atmosphere was replaced with hydrogen, and the mixture was stirred overnight at room temperature. Thereafter, the reaction liquid was stirred with Celite(R), and then filtered while being washed with MeOH. The filtrate was concentrated. To the resulting residue was added ethyl acetate (200 mL), and the mixture was stirred overnight at room temperature. The solid was collected by filtration to give N-(tert-butoxycarbonyl)-L-valyl-(4R)-4-hydroxy-L-proline (32.7 g) as a solid.

### Example 10

A mixture of N-(tert-butoxycarbonyl)-L-valyl-(4R)-4-hydroxy-L-proline (13.7 g), (2R)-2-amino-2-[4-(4-methyl-1,3-thiazol-5-yl)phenyl]ethan-1-ol dihydrochloride (13.3 g), DMF (110 mL), and THF (110 mL) was stirred with DIPEA (28 mL). Under ice-bath cooling (ice/saturated aqueous sodium chloride solution), HATU (16.5 g) was added portionwise (while maintaining the internal temperature below 0°C). After the mixture was stirred for 30 minutes under ice-bath cooling, semi-saturated aqueous sodium chloride solution (400 mL) and ethyl acetate (200 mL) were added thereto, and the mixture was stirred and then separated into an aqueous layer and an organic layer. The aqueous layer was extracted twice with ethyl acetate, and the combined organic layer was washed successively with saturated aqueous sodium hydrogen carbonate solution and saturated aqueous sodium chloride solution, then dried over anhydrous sodium sulfate. Insoluble materials were removed by filtration, and the filtrate was concentrated under reduced pressure to give N-(tert-butoxycarbonyl)-L-valyl-(4R)-4-hydroxy-N-{(1R)-2-hydroxy-1-[4-(4-methyl-1,3-thiazol-5-yl)phenyl]ethyl}-L-proline amide (32.6 g, containing impurities) as a foam-like solid. To a solution of the resulting solid in ethyl acetate (100 mL)/MeOH (100 mL), 4M hydrogen chloride (ethyl acetate solution, 100 mL) was added portionwise under ice-bath cooling, and the mixture was stirred overnight at room temperature. The resulting solid was collected by filtration, ethyl acetate (50 mL)/MeOH (100 mL) was added, and the mixture was stirred for 2 hours at room temperature. The solid was collected by filtration to give L-valyl-(4R)-4-hydroxy-N-{(1R)-2-hydroxy-1-[4-(4-methyl-1,3-thiazol-5-yl)phenyl]ethyl}-L-proline amide dihydrochloride (19.0 g) as a solid.

L-valyl-(4R)-4-hydroxy-N-{(1R)-2-hydroxy-1-[4-(4-methyl-1,3-thiazol-5-yl)phenyl]ethyl}-L-proline amide dihydrochloride, obtained in Example 10, was reacted with a diazo-transfer reagent under the reaction conditions described in International Publication No. WO 2022/173032 to give (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-proline amide.

### Example 10-2

A mixture of 1,1,2,2,3,3,4,4,4-nonafluoro-1-butanesulfonyl chloride (2.42 g), methyl tert-butyl ether (19 mL), water (19 mL), MeCN (1 mL), sodium azide (0.61 g), sodium hydrogen carbonate (0.24 g), and tetrabutylammonium chloride (0.31 g) was stirred at 5°C for 20 hours. After addition of 25% aqueous sodium chloride solution, the mixture was separated to give 1,1,2,2,3,3,4,4,4-nonafluoro-1-butanesulfonyl azide. The obtained organic layer was added to a mixture of L-valyl-(4R)-4-hydroxy-N-{(1R)-2-hydroxy-1-[4-(4-methyl-1,3-thiazol-5-yl)phenyl]ethyl}-L-proline amide dihydrochloride (2.00 g), potassium hydrogen carbonate (1.93 g), DMSO (20 mL), and water (4 mL), and the mixture was stirred at 15°C for 16 hours. To the reaction liquid were added 25% aqueous sodium chloride solution and ethyl acetate, and the mixture was subjected to separation. To the obtained organic layer were added silica gel and sodium sulfate, and the mixture was stirred at room temperature for 30 minutes. Insoluble materials were removed by filtration, and the filtrate was concentrated under reduced pressure. Then, crystallization was performed from methanol and methyl tert-butyl ether to give (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-proline amide (1.15 g) as a solid.

### Example 11

Under a nitrogen atmosphere, to a solution of tert-butyl (1 S,4S)-5-{(7M)-6-cyclopropyl-8-[(4-ethynylphenyl)methoxy]-7-(6-fluoro-5-methyl-1H-indazol-4-yl)-2-[(oxan-4-yl)oxy]quinazolin-4-yl}-2,5-diazabicyclo[2.2.1]heptane-2-carboxylate (310 mg) and (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-proline amide (200 mg) in tBuOH (0.4 mL)/THF (0.5 mL)/water (0.4 mL) were added sodium ascorbate (80 mg) and copper(I) iodide (20 mg) at room temperature, and the mixture was stirred for 4 hours. At room temperature, copper(I) iodide (20 mg) was added, and the mixture was further stirred for 15 hours. To this mixture was added a solution of disodium ethylenediaminetetraacetate (750 mg) in water (20 mL), and the mixture was diluted with ethyl acetate (20 mL) and stirred at room temperature for 1 hour. The mixture was extracted twice with ethyl acetate, and the combined organic layer was washed with water and saturated aqueous sodium chloride solution, dried over sodium sulfate, filtered, and concentrated under reduced pressure. The residue was purified by silica gel column chromatography (amino-modified silica gel, CHCl₃/MeOH) to give tert-butyl (1S,4S)-5-{(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl} carbamoyl)pyrrolidin-1-yl]-3-methyl-1-oxobutan-2-yl}-1H-1,2,3-triazol-4-yl)phenyl]methoxy}-2-[(oxan-4-yl)oxy]quinazolin-4-yl}-2,5-diazabicyclo[2.2.1]heptane-2-carboxylate (452 mg) as a solid.

### Example 12

Under a nitrogen atmosphere, to a solution of tert-butyl (1 S,4S)-5-{(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(oxan-4-yl)oxy]quinazolin-4-yl}-2,5-diazabicyclo[2.2.1]heptane-2-carboxylate (200 mg) in MeOH (1 mL) was added methanesulfonic acid (100 µL) at room temperature, and the mixture was stirred at 50°C for 4 hours. The mixture was concentrated under reduced pressure, and the residue was purified by reverse-phase chromatography (ODS column, 0.1% formic acid in water/0.1% formic acid in MeCN). Fractions containing the desired product was combined, to which 5% aqueous sodium hydrogen carbonate solution was added. The mixture was extracted twice with CHCl₃/MeOH (9/1). The combined organic layer was dried over sodium sulfate and concentrated under reduced pressure to give (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-proline amide (139 mg) as a solid.

### Example 14

Under an argon atmosphere, 6-chloro-7-fluoro-1H-indole-2,3-dione (250.8 g) and MeCN (1.25 L) were mixed together and stirred at room temperature. Methanesulfonic acid (24.2 g) and 1,3-dibromo-5,5-dimethylhydantoin (359.3 g) were added, and the mixture was stirred at room temperature for 10 minutes. The mixture was stirred at 55°C for 20 hours and then cooled to 10°C or lower, and a solution of sodium ascorbate (497.9 g) in water (2.5 L) was added thereto. After the mixture was stirred at 10°C or lower for 1 hour, water (2.5 L) was added at room temperature. The mixture was stirred for 1 hour, and the precipitate was collected by filtration, washed with water, and dried at 60°C under reduced pressure to give 5-bromo-6-chloro-7-fluoro-1H-indole-2,3-dione (320.4 g) as a solid.

### Example 15

Under an argon atmosphere, 5-bromo-6-chloro-7-fluoro-1H-indole-2,3-dione (320 g), EtOH (2.24 L), and sodium acetate (141 g) were mixed together and stirred at room temperature for 10 minutes, followed by addition of hydroxylamine hydrochloride (87.8 g). The mixture was stirred at 50°C for 2 hours. After addition of water (4.16 L) at 50°C, the mixture was cooled to room temperature and stirred for 30 minutes to collect the resulting solid by filtration. The filter cake was washed successively with EtOH/water (1/2) and water, and dried at 60°C under reduced pressure to give 5-bromo-6-chloro-7-fluoro-3-(hydroxyimino)-1,3-dihydro-2H-indol-2-one (314 g) as a solid.

### Example 16

Under a nitrogen atmosphere, 5-bromo-6-chloro-7-fluoro-3-(hydroxyimino)-1,3-dihydro-2H-indol-2-one (1 g), toluene (10 mL), triphenylphosphine oxide (192 mg), and DIPEA (0.12 mL) were mixed together and stirred at room temperature. After thionyl chloride (1.24 mL) was added portionwise while maintaining the internal temperature below 50°C, the mixture was heated to maintain an internal temperature of 65 to 75°C and stirred for 17 hours. Thionyl chloride (1.24 mL) was added at the same temperature, and the mixture was stirred at an internal temperature of 95 to 105°C for 6 days. The reaction liquid was cooled to room temperature and was added dropwise to an ice-cooled solution of dipotassium hydrogen phosphate (11.9 g) in water (10 mL) (while maintaining the internal temperature below 30°C). The obtained mixture was filtered while being washed with toluene, and the filtrate was subjected to separation. The organic layer was washed with aqueous sodium hydrogen carbonate solution to give a toluene solution of 6-bromo-2,4,7-trichloro-8-fluoroquinazoline. To this toluene solution were added DIPEA (875 µL) and tert-butyl (1S,4S)-2,5-diazabicyclo[2.2.1]heptane-2-carboxylate (0.71 g) at room temperature, and the mixture was stirred for 2.5 hours. Water was added, and the mixture was separated. The organic layer was washed with water and concentrated under reduced pressure. EtOH was added to the residue, and the mixture was again concentrated under reduced pressure to azeotropically remove toluene, after which the residue was dissolved in EtOH (4 mL). The mixture was stirred at 50°C, then cooled to room temperature, and the precipitated solid was collected by filtration and dried at 60°C under reduced pressure to give tert-butyl (1S,4S)-5-(6-bromo-2,7-dichloro-8-fluoroquinazolin-4-yl)-2,5-diazabicyclo[2.2.1]heptane-2-carboxylate (1.27 g) as a solid.

### Example 17

At room temperature, tert-butyl (1S,4S)-5-(6-bromo-2,7-dichloro-8-fluoroquinazolin-4-yl)-2,5-diazabicyclo[2.2.1]heptane-2-carboxylate (5 g) and tetrahydro-2H-pyran-4-ol (1.24 g) were dissolved in DMF (15 mL), and cesium carbonate (9.93 g) was added, followed by stirring. DABCO (228 mg) was then added thereto at room temperature, and the mixture was stirred for 20 minutes, and then at 55°C for 1.5 hours. The mixture was cooled to room temperature, toluene and water were added, and the mixture was separated. The organic layer was washed twice with water. The organic layer was concentrated, and EtOH (50 mL) and water (10 mL) were added to the residue. The precipitate was collected by filtration, washed with EtOH/water (1/1), and dried at 60°C under reduced pressure to give tert-butyl (1S,4S)-5-{6-bromo-7-chloro-8-fluoro-2-[(oxan-4-yl)oxy]quinazolin-4-yl}-2,5-diazabicyclo[2.2.1]heptane-2-carboxylate (4.13 g) as a solid.

### Example 18

Under a nitrogen atmosphere, tBuONa (620 mg) was dissolved in DMF (5 mL) at room temperature and stirred. A solution of (1 S)-1-phenylethan-1-ol (821 mg) and tert-butyl (1S,4S)-5-{6-bromo-7-chloro-8-fluoro-2-[(oxan-4-yl)oxy]quinazolin-4-yl}-2,5-diazabicyclo[2.2.1]heptane-2-carboxylate (3 g) in DMF (10 mL) was added thereto at room temperature, and the mixture was stirred for 2 hours, followed by addition of toluene (15 mL). After 10 minutes, (1S)-1-phenylethan-1-ol (82.1 mg) and tBuONa (62 mg) were added, followed by stirring for 45 minutes. Then, tBuONa (103 mg) was added, and the mixture was stirred at room temperature for 45 minutes. Thereafter, toluene and water were added, and the mixture was separated. The organic layer was washed twice with water, dried over sodium sulfate, filtered to remove insoluble materials, and concentrated under reduced pressure. The residue was stirred with EtOH, and the precipitated solid was collected by filtration, washed with EtOH, and dried at 50°C under reduced pressure to give tert-butyl (1S,4S)-5-{6-bromo-7-chloro-2-[(oxan-4-yl)oxy]-8-[(1S)-1-phenylethoxy]quinazolin-4-yl}-2,5-diazabicyclo[2.2.1]heptane-2-carboxylate (3.07 g) as a solid.

### Example 19

A mixture of tert-butyl (1S,4S)-5-{6-bromo-7-chloro-2-[(oxan-4-yl)oxy]-8-[(1S)-1-phenylethoxy]quinazolin-4-yl}-2,5-diazabicyclo[2.2.1]heptane-2-carboxylate (1 g), toluene (7 mL), water (2 mL), tripotassium phosphate (964 mg), and cyclopropylboronic acid (221 mg) was degassed under reduced pressure at room temperature. PdCl₂(dppf)·CH₂Cl₂ (55.4 mg) was added, and the mixture was degassed under reduced pressure and stirred at 80°C for 16 hours. After cooling to room temperature, the reaction liquid was stirred with activated carbon for 30 minutes at room temperature. Insoluble materials were removed by filtration while being washed with toluene and water, and the filtrate was separated. The resulting organic layer was dried over sodium sulfate, filtered, and concentrated under reduced pressure to give tert-butyl (1S,4S)-5-{7-chloro-6-cyclopropyl-2-[(oxan-4-yl)oxy]-8-[(1S)-1-phenylethoxy]quinazolin-4-yl}-2,5-diazabicyclo[2.2.1]heptane-2-carboxylate (0.95 g) as a solid.

### Example 20

Tert-butyl 6-fluoro-5-methyl-4-(4,4,5,5-tetramethyl-1,3,2-dioxaborolan-2-yl)-2-(triphenylmethyl)-2H-indazole (100 mg), (1S,4S)-5-{7-chloro-6-cyclopropyl-2-[(oxan-4-yl)oxy]-8-[(1S)-1-phenylethoxy]quinazolin-4-yl}-2,5-diazabicyclo[2.2.1]heptane-2-carboxylate (100 mg), barium hydroxide octahydrate (152 mg), RuPhos (4 mg), 2-methyl-2-butanol (0.8 mL), and water (0.2 mL) were mixed together and degassed under reduced pressure. RuPhos Pd G3 (7 mg) was then added, and the mixture was degassed under reduced pressure and stirred at 65°C for 1.5 hours. After cooling to room temperature, water (0.6 mL) and activated carbon (10 mg) were added, and the mixture was stirred for 30 minutes.

The insoluble material was filtered while being washed with toluene, and the filtrate was subjected to separation. The resulting organic layer was dried over sodium sulfate, filtered, and concentrated under reduced pressure to give a solid. MeOH/water (4/1) was added to the resulting solid, and the precipitated solid was collected by filtration and washed with MeOH/water (4/1) (ratio of isomers derived from axial chirality: M:P = 4.5:1). The resulting solid was suspended in MeOH, collected by filtration while being washed with MeOH, suspended again in MeOH, collected by filtration while being washed with MeOH, and dried under reduced pressure to give tert-butyl (1S,4S)-5-{6-cyclopropyl-7-[6-fluoro-5-methyl-2-(triphenylmethyl)-2H-indazol-4-yl]-2-[(oxan-4-yl)oxy]-8-[(1S)-1-phenylethoxy]quinazolin-4-yl}-2,5-diazabicyclo[2.2.1]heptane-2-carboxylate (53.9 mg, ratio of isomers derived from axial chirality: M:P = 58:1) as a solid.

### Example 21

Tert-butyl (3S)-3-[{7-bromo-6-cyclopropyl-2-[(2S)-2-methoxypropoxy]-8-[(1S)-1-(naphthalen-1-yl)ethoxy]quinazolin-4-yl}(methyl)amino]pyrrolidin-1-carboxylate (1.40 g), 6-fluoro-5-methyl-4-(4,4,5,5-tetramethyl-1,3,2-dioxaborolan-2-yl)-2-(triphenylmethyl)-2H-indazole (1.50 g), SPhos (85 mg), SPhos Pd G3 (155 mg), tripotassium phosphate (1.70 g), DOX (30 mL), and water (6 mL) were mixed together and subjected to repeated cycles of degassing and argon gas purging, and the mixture was then stirred at 80°C for 3 hours under an argon atmosphere. Ethyl acetate was added to the cooled reaction suspension, and the mixture was filtered through Celite(R) while being washed with ethyl acetate. The filtrate was washed with water and saturated aqueous sodium chloride solution. The organic layer was dried over anhydrous magnesium sulfate and concentrated under reduced pressure, and the residue was purified by silica gel column chromatography (hexane/ethyl acetate) to give tert-butyl (3S)-3-[{6-cyclopropyl-7-[6-fluoro-5-methyl-2-(triphenylmethyl)-2H-indazol-4-yl]-2-[(2S)-2-methoxypropoxy]-8-[(1S)-1-(naphthalen-1-yl)ethoxy]quinazolin-4-yl}(methyl)amino]pyrrolidin-1-carboxylate (1.47 g) as a foam-like solid.

### Example 22

Under an argon atmosphere, a solution of 7-bromo-4-tert-butoxy-6-cyclopropyl-2-(ethylsulfanyl)-8-[(1S)-1-(naphthalen-1-yl)ethoxy]quinazoline (1.5 g) in DOX (30 mL) and water (6 mL) was mixed at room temperature with 6-fluoro-5-methyl-4-(4,4,5,5-tetramethyl-1,3,2-dioxaborolan-2-yl)-2-(triphenylmethyl)-2H-indazole (1.17 g), SPhos (81 mg), SPhos Pd G3 (153 mg), and tripotassium phosphate (1.66 g), and the mixture was stirred at 80°C for 3 hours under an argon atmosphere. After cooling to room temperature, water and ethyl acetate were added to the reaction solution, and the mixture was separated. The organic layer was washed with saturated aqueous sodium chloride solution and dried over anhydrous magnesium sulfate. The mixture was concentrated under reduced pressure, and the residue was purified by silica gel column chromatography (hexane/ethyl acetate) to give 4-tert-butoxy-6-cyclopropyl-2-(ethylsulfanyl)-7-[6-fluoro-5-methyl-2-(triphenylmethyl)-2H-indazol-4-yl]-8-[(1S)-1-(naphthalen-1-yl)ethoxy]quinazoline (1.00 g) as a foam-like solid.

### Example 23

Under an argon atmosphere, tert-butyl (3S)-3-[{6-cyclopropyl-7-(5,5-dimethyl-1,3,2-dioxaborinan-2-yl)-2-[(2S)-2-methoxypropoxy]-8-[(1S)-1-(naphthalen-1-yl)ethoxy]quinazolin-4-yl}(methyl)amino]pyrrolidin-1-carboxylate (1.00 g, about 83% purity), 4-bromo-6-fluoro-5-methyl-2-(triphenylmethyl)-2H-indazole (630 mg), SPhos (90.0 mg), barium hydroxide octahydrate (1.06 g), 2-methyl-2-butanol (4.15 mL), and water (4.15 mL) were mixed together and degassed under reduced pressure. SPhos Pd G3 (170 mg) was added to the reaction liquid, and after degassing again under reduced pressure, the mixture was stirred at 60°C for 3 hours. The mixture was cooled to room temperature and stirred for 68 hours with activated carbon (80 mg). The insoluble material was filtered through Celite(R), and the filter cake was washed with toluene (33 mL) and water (17 mL). The filtrate was subjected to separation, and the organic layer was washed twice with water (4.1 mL) and concentrated under reduced pressure. To the resulting residue (ratio of isomers derived from axial chirality: M:P = 8.2:1) were added toluene (4 mL), and heptane (6 mL), followed by addition of amino-modified silica gel (3.3 g), neutral silica gel (1.6 g), and heptane (40 mL), and the mixture was stirred for 2 hours 40 minutes. The mixture was filtered and washed with heptane and ethyl acetate, and the filtrate was concentrated. MeOH was added to the residue, the mixture was stirred and then concentrated. One half of the resulting residue was purified twice by silica gel column chromatography (hexane/ethyl acetate), followed by purification by silica gel column chromatography (amino-modified silica gel, hexane/ethyl acetate), to give tert-butyl (3S)-3-[{(7M)-6-cyclopropyl-7-[6-fluoro-5-methyl-2-(triphenylmethyl)-2H-indazol-4-yl]-2-[(2S)-2-methoxypropoxy]-8-[(1S)-1-(naphthalen-1-yl)ethoxy]quinazolin-4-yl}(methyl)amino]pyrrolidin-1-carboxylate (105 mg) as a foam-like solid.

The starting material used in Example 23 was prepared by the following method.

Under an argon atmosphere, tert-butyl (3S)-3-[{7-chloro-6-cyclopropyl-2-[(2S)-2-methoxypropoxy]-8-[(1S)-1-(naphthalen-1-yl)ethoxy]quinazolin-4-yl}(methyl)amino]pyrrolidin-1-carboxylate (2.21 g), 5,5,5',5'-tetramethyl-2,2'-bi-1,3,2-dioxaborinane (1.51 g), potassium propionate (1.12 g), 2-(2,6-dimethoxyphenyl)-3-(diphenylphosphanyl)-1-methyl-1H-indole (0.15 g), and toluene (17.7 mL) were mixed together at room temperature and degassed under reduced pressure. Di-µ-chlorobis(2'-amino-1,1'-biphenyl-2-yl-C,N)dipalladium(II) (0.05 g) was added to the reaction liquid, followed by degassing under reduced pressure and stirring. The mixture was then stirred at 70°C for 24 hours. After the reaction liquid was cooled to room temperature, the insoluble materials were filtered through Celite(R) and washed with toluene. The filtrate was concentrated under reduced pressure. MeOH was added to the residue and azeotropically evaporated three times. Thereafter, the residue was dissolved in MeOH (6 mL) and then added dropwise into water (80 mL) while rinsing with MeOH (2 mL). The precipitated solid was collected by filtration and dried under reduced pressure to give tert-butyl (3S)-3-[{6-cyclopropyl-7-(5,5-dimethyl-1,3,2-dioxaborinan-2-yl)-2-[(2S)-2-methoxypropoxy]-8-[(1S)-1-(naphthalen-1-yl)ethoxy]quinazolin-4-yl}(methyl)amino]pyrrolidine-1-carboxylate (2.99 g, about 83% purity) as a solid.

### Example 34

At room temperature, 4-tert-butoxy-7-chloro-6-cyclopropyl-2-[(2S)-2-methoxypropoxy]-8-[(1S)-1-(naphthalen-1-yl)ethoxy]quinazoline (0.7 g), DOX (5.6 mL), water (1.4 mL), 6-fluoro-5-methyl-4-(4,4,5,5-tetramethyl-1,3,2-dioxaborolan-2-yl)-2-(triphenylmethyl)-2H-indazole (814 mg), barium hydroxide octahydrate (1.24 g), and SPhos (53.7 mg) were mixed together and degassed under reduced pressure. SPhos Pd G3 (102 mg) was then added, and the mixture was degassed under reduced pressure and stirred at 65°C for 6 hours. After cooling to room temperature, the mixture was stirred for 10 minutes with activated carbon (70 mg), filtered, and washed with toluene. The filtrate was separated, and the organic layer was washed with water, dried over sodium sulfate, filtered, and concentrated. The residue was dissolved in iPrOH (14 mL), and water (4.9 mL) was added portionwise. The resulting solid was collected by filtration, washed with iPrOH/water (3/1), and dried under reduced pressure to give 4-tert-butoxy-6-cyclopropyl-7-[6-fluoro-5-methyl-2-(triphenylmethyl)-2H-indazol-4-yl]-2-[(2S)-2-methoxypropoxy]-8-[(1S)-1-(naphthalen-1-yl)ethoxy]quinazoline (0.74 g) as a solid.

### Example 35

At room temperature, 2-(trimethylsilyl)ethyl 4-{7-bromo-4-tert-butoxy-6-cyclopropyl-8-[(1S)-1-phenylethoxy]quinazolin-2-yl}piperazin-1-carboxylate (200 mg), 6-fluoro-5-methyl-4-(4,4,5,5-tetramethyl-1,3,2-dioxaborolan-2-yl)-2-(triphenylmethyl)-2H-indazole (220 mg), SPhos Pd G3 (47 mg), SPhos (25 mg), anhydrous barium hydroxide (73 mg), toluene (6 mL), and water (6 mL) were mixed together and subjected to repeated cycles of degassing and argon gas purging. The mixture was then stirred at 60°C for 3 hours under an argon atmosphere. The reaction mixture was allowed to cool, ethyl acetate was added thereto, and the mixture was filtered through Celite(R). The aqueous layer was extracted twice with ethyl acetate. The combined organic layer was washed with saturated aqueous sodium chloride solution, dried over anhydrous magnesium sulfate, filtered, and concentrated under reduced pressure. The residue was purified by silica gel column chromatography (hexane/ethyl acetate) to give 2-(trimethylsilyl)ethyl 4-{4-tert-butoxy-6-cyclopropyl-7-[6-fluoro-5-methyl-2-(triphenylmethyl)-2H-indazol-4-yl]-8-[(1S)-1-phenylethoxy]quinazolin-2-yl} piperazine-1-carboxylate (237 mg) as a foam-like solid.

### Example 36

To a mixture of 7-bromo-4-tert-butoxy-2-chloro-8-fluoro-6-iodoquinazoline (21.0 g), molecular sieves 4A (21.0 g), DIPEA (10.35 mL), and THF (210 mL) was added 2-(trimethylsilyl)ethyl piperazine-1-carboxylate (8.9 g), and the mixture was stirred at 40°C for 12 hours. Water (400 mL) was added to the mixture, and the mixture was extracted with ethyl acetate three times. The combined organic layer was washed twice with saturated aqueous sodium chloride solution, dried over anhydrous sodium sulfate, filtered, and concentrated under reduced pressure. MeOH (100 mL) was added to the resulting residue for trituration, and the resultant was collected by filtration and dried under reduced pressure to give 2-(trimethylsilyl)ethyl 4-(7-bromo-4-tert-butoxy-8-fluoro-6-iodoquinazolin-2-yl)piperazine-1-carboxylate (12.2 g) as a solid.

### Example 37

Under an argon gas atmosphere, to a mixture of 2-(trimethylsilyl)ethyl 4-(7-bromo-4-tert-butoxy-8-fluoro-6-iodoquinazolin-2-yl)piperazine-1-carboxylate (2.00 g), (1S)-1-phenylethan-1-ol (0.42 g), and THF (20 mL) was added tBuOK (0.72 g) with stirring at room temperature, and the mixture was stirred at room temperature for 40 minutes. Ice and saturated aqueous ammonium chloride solution were added to the reaction mixture, and the mixture was extracted twice with ethyl acetate. The combined organic layer was washed with saturated aqueous sodium chloride solution and dried over anhydrous magnesium sulfate. The mixture was filtered and concentrated under reduced pressure, and the residue was purified by silica gel column chromatography (amino-modified silica gel, hexane/ethyl acetate) to give 2-(trimethylsilyl)ethyl 4-{7-bromo-4-tert-butoxy-6-iodo-8-[(1S)-1-phenylethoxy]quinazolin-2-yl}piperazine-1-carboxylate (2.13 g) as a foam-like solid.

### Example 38

At room temperature, 2-(trimethylsilyl)ethyl 4-{7-bromo-4-tert-butoxy-6-iodo-8-[(1S)-1-phenylethoxy]quinazolin-2-yl} piperazine-1-carboxylate (2.02 g), cyclopropylboronic acid (400 mg), tripotassium phosphate (2.00 g), PdCl₂(dppf)·CH₂Cl₂ (230 mg), MeCN (50 mL), and water (10 mL) were mixed together and stirred at 90°C for 6 hours under an argon atmosphere. After the reaction mixture was allowed to cool, ethyl acetate was added thereto, and the mixture was filtered through Celite(R). Water was added to the filtrate to separate two layers, and the organic layer was dried over anhydrous magnesium sulfate. The insoluble materials were removed by filtration, and the filtrate was concentrated under reduced pressure. The residue was purified by silica gel column chromatography (hexane/ethyl acetate) to give 2-(trimethylsilyl)ethyl 4-{7-bromo-4-tert-butoxy-6-cyclopropyl-8-[(1S)-1-phenylethoxy]quinazolin-2-yl}piperazine-1-carboxylate (1.41 g) as a foam-like solid.

**[Table 1-1]**

| Ex | Syn | Synthetic Compound | DATA |
|---|---|---|---|
| 1 | 1 | 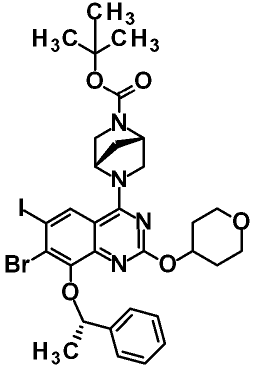 | ESI+: 753.0 |
| 2 | 2 | 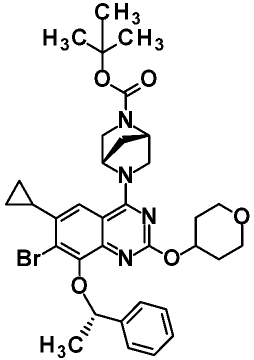 | ESI+: 667.2 |
| 4 | 4 | 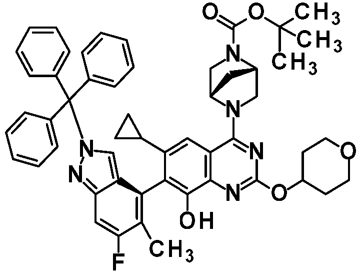 | ESI+: 873.4 |

**[Table 1-2]**

| | | | |
|---|---|---|---|
| 5 | 5 | 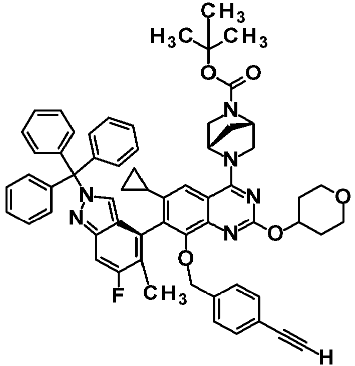 | ESI+: 987.3 |
| 6 | 6 | 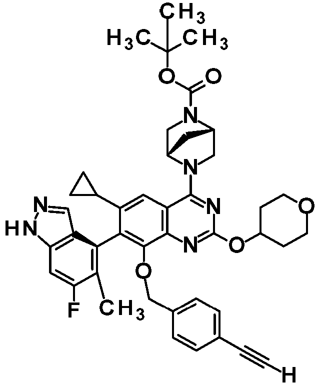 | ESI+: 745.5 |
| 7 | 7 | 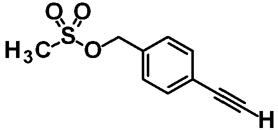 | ESI+: 233.0 |
| | | | [M+Na]+ |
| 8 | 8 | 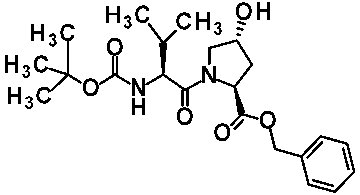 | ESI+: 421.3 |
| 9 | 9 | 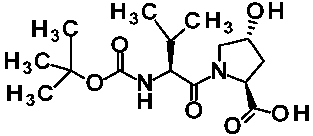 | ESI+: 331.2 |

**[Table 1-3]**

| | | | |
|---|---|---|---|
| 10 | 10 | 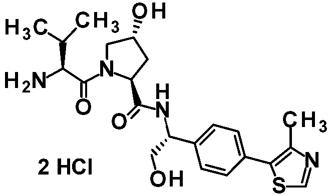 | ESI+: 447.3 |
| 10-2 | 10-2 | 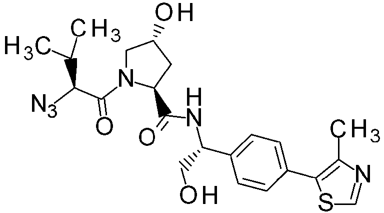 | ESI+: 473.5 |
| 11 | 11 | 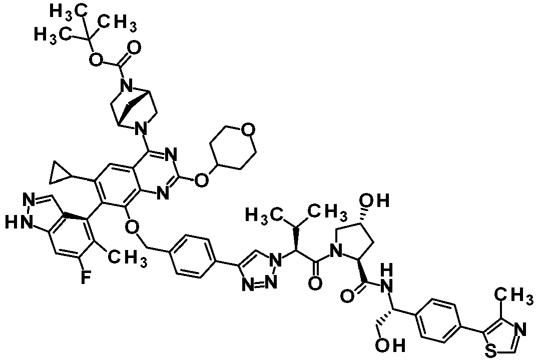 | ESI-: 1215.6 |

**[Table 1-4]**

| | | | |
|---|---|---|---|
| 12 | 12 | 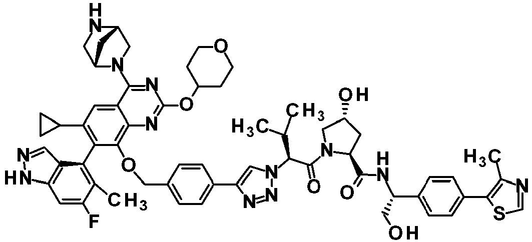 | ESI+: 1117.7 |
| | | | NMR (100°C): 0.48-0.68 (4H, m), 0.77 (3H, br d), 1.07 (3H, br d), 1.35-1.43 (1H, m), 1.66-1.77 (3H, m), 1.87 (1H, br d), 1.89-1.97 (1H, m), 1.98-2.10 (3H, m), 2.01 (3H, d), 2.10-2.21 (1H, m), 2.45 (3H, s), 2.50-2.59 (1H, m), 3.06 (1H, dd), 3.13 (1H, d), 3.35-3.45 (2H, m), 3.57-3.64 (1H, m), 3.65-3.75 (3H, m), 3.75-3.79 (1H, m), 3.80-3.90 (3H, m), 4.16 (1H, dd), 4.35 (1H, br s), 4.41-4.48 (1H, m), 4.48-4.56 (1H, m), 4.78-4.84 (2H, m), 4.84-4.95 (1H, m), 5.13 (1H, br s), 5.17-5.24 (1H, m), 5.24-5.31 (2H, m), 6.82 (2H, d), 7.30 (1H, d), 7.38-7.44 (4H, m), 7.44-7.48 (2H, m), 7.61 (2H, br d), 8.00 (1H, br d), 8.43 (1H, br s), 8.88 (1H, s), 12.75 (1H, br s) |
| 14 | 14 | 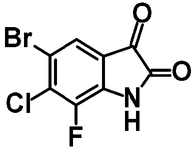 | ESI-: 275.8, 277.8, 279.8 |
| 15 | 15 | 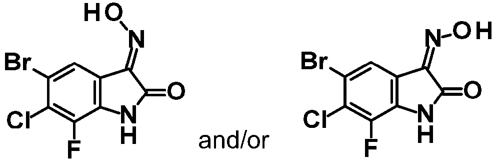 | ESI+: 292.9, 294.9, 296.9 |

**[Table 1-5]**

| | | | |
|---|---|---|---|
| 16 | 16 | 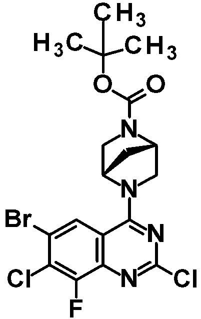 | ESI+: 491.1, 493.1, 495.1 |
| 17 | 17 | 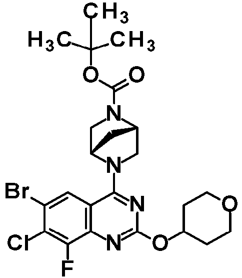 | ESI+: 557.2, 559.2, 561.2 |
| 18 | 18 | 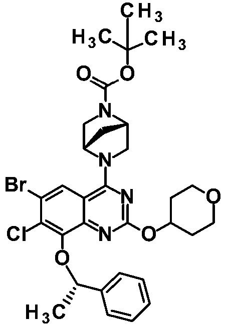 | ESI+: 659.3, 661.3 |

**[Table 1-6]**

| | | | |
|---|---|---|---|
| 19 | 19 | 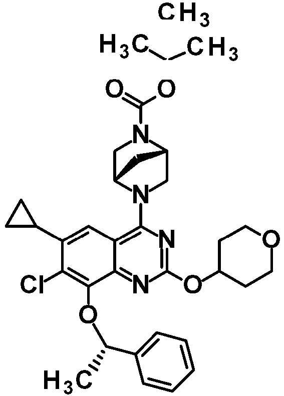 | ESI+: 621.4, 623.4 |

**[Table 2-1]**

| Ex | Syn | Starting Material | Synthetic Compound (Structure of major isomer) | DATA | Axial Enantioselectivity (M:P) |
|---|---|---|---|---|---|
| | | | | Yield | HPLC Analysis Condition |
| 3 | 3 | 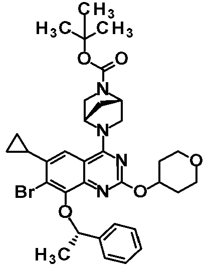 | 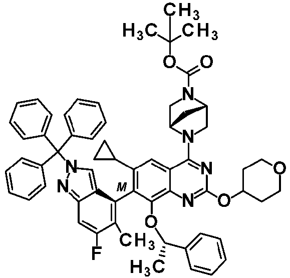 | ESI+: 977.3 | 96:1 (HPLC) |
| | | | | 62% | Analysis Condition 1 |
| | | | | | Retention time |
| | | | | | 10.39(M isomer) |
| | | | | | 12.36(P isomer) |
| 3-1 | 3-1 | 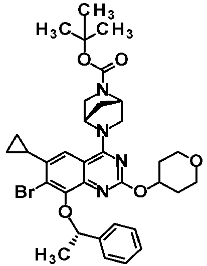 | 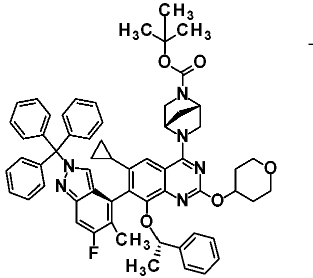 | ESI+: 977.4 | 4.4:1 (NMR) |
| | | | | 83% | |
| 3-2 | 3-2 | 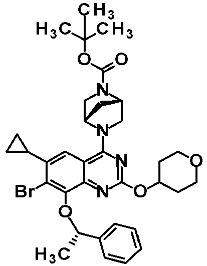 | 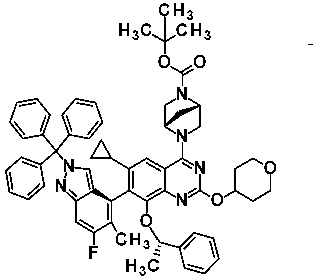 | ESI+: 977.3 | 4.5:1 (HPLC) |
| | | | | | 4.4:1 (NMR) |
| | | | | 99% | Analysis Condition 1 |
| | | | | | Retention time |
| | | | | | 10.52(M isomer) |
| | | | | | 12.48(P isomer) |
| 3-3 | 3-3 | 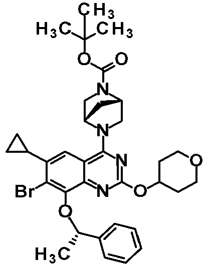 | 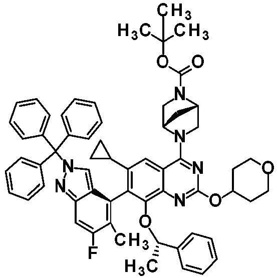 | ESI+: 977.3 | 5.0:1 (HPLC) |
| | | | | | 4.9:1 (NMR) |
| | | | | 91% | Analysis Condition 1 |
| | | | | | Retention time |
| | | | | | 10.63(M isomer) |
| | | | | | 12.55(P isomer) |

**[Table 2-2]**

| | | | | | |
|---|---|---|---|---|---|
| 3-4 | 3-4 | 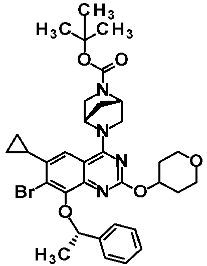 | 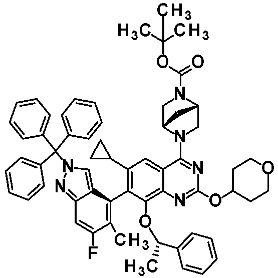 | ESI+: 977.3 | 6.3:1 (HPLC) |
| | | | | | 6.5:1 (NMR) |
| | | | | 96% | Analysis Condition 1 |
| | | | | | Retention time |
| | | | | | 10.62(M isomer) |
| | | | | | 12.54(P isomer) |
| 20 | 20 | 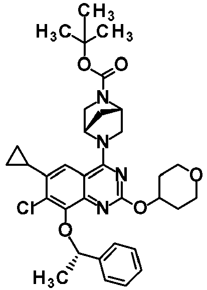 | 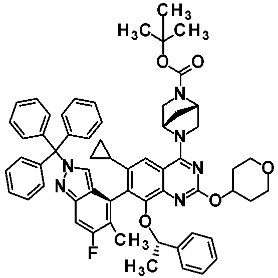 | ESI+: 977.7 | 58:1 (HPLC) |
| | | | | 34% | Analysis Condition 2 |
| | | | | | Retention time |
| | | | | | 12.01(M isomer) |
| | | | | | 12.35(P isomer) |
| 21 | 21 | 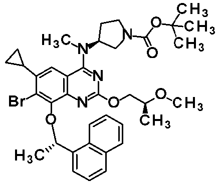 | 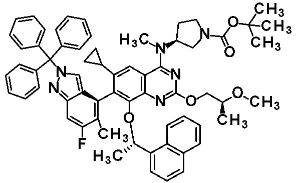 | ESI+: 1017.8 | 8.5:1 (HPLC) |
| | | | | 73% | Analysis Condition 3 |
| | | | | | Retention time |
| | | | | | 21.65(M isomer) |
| | | | | | 22.03(P isomer) |

**[Table 2-3]**

| | | | | | |
|---|---|---|---|---|---|
| 22 | 22 | 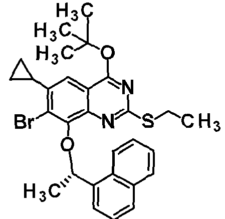 | 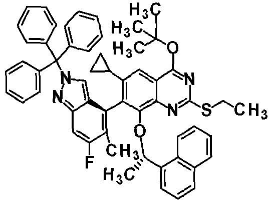 | ESI+: 863.6 | 13 : 1 (HPLC) |
| | | | | 59% | Analysis Condition 4 |
| | | | | | Retention time |
| | | | | | 13.30(M isomer) |
| | | | | | 13.64(P isomer) |
| 23 | 23 | 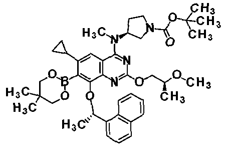 | 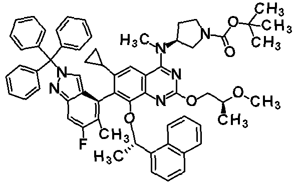 | ESI+: 1017.8 | 8.2:1 (before column purification, HPLC) |
| | | | | | > 99:1 (after column purification, HPLC) |
| | | | | 9% | Analysis Condition 5 |
| | | | | | Retention time |
| | | | | | 17.34(M isomer) |
| | | | | | 17.92(P isomer) |
| 24 | 3-1 | 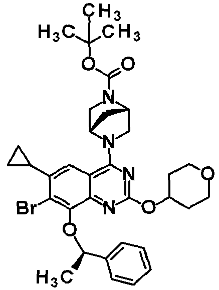 | 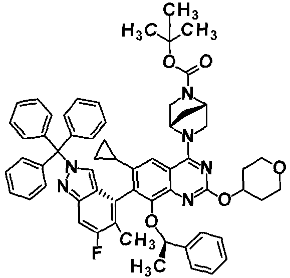 | ESI+: 977.6 | 1:1.7 (HPLC) |
| | | | | | 1:1.5 (NMR) |
| | | | | 84% | Analysis Condition 1 |
| | | | | | Retention time |
| | | | | | 11.54(P isomer) |
| | | | | | 12.08(M isomer) |
| 25 | 3-3 | 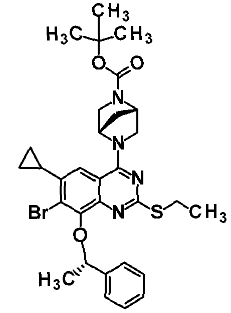 | 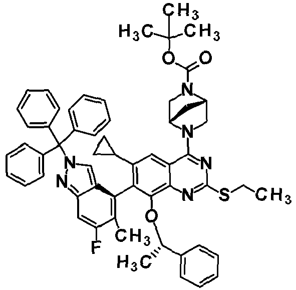 | ESI+: 937.6 | 4.5:1 (HPLC) |
| | | | | | 4.7:1 (NMR) |
| | | | | 78% | Analysis Condition 3 |
| | | | | | Retention time |
| | | | | | 21.22(M isomer) |
| | | | | | 22.49(P isomer) |

**[Table 2-4]**

| | | | | | |
|---|---|---|---|---|---|
| 26 | 3-3 | 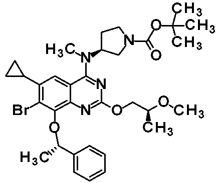 | 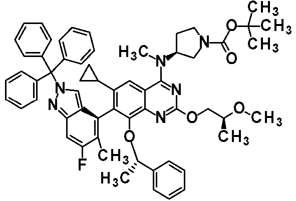 | ESI+: 967.8 | 2.1:1 (HPLC) |
| | | | | | 2.0:1 (NMR) |
| | | | | 55% | Analysis Condition 6 |
| | | | | | Retention time |
| | | | | | 5.67(P isomer) |
| | | | | | 5.80(M isomer) |
| 27 | 3-3 | 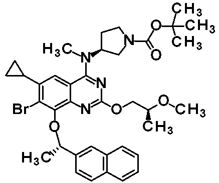 | 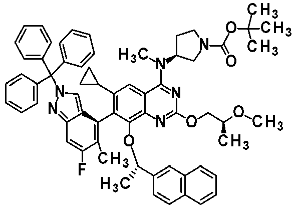 | ESI+: 1017.9 | 1.6:1 (HPLC) |
| | | | | | 1.3:1 (NMR) |
| | | | | 23% | Analysis Condition 7 |
| | | | | | Retention time |
| | | | | | 12.14(M isomer) |
| | | | | | 13.68(P isomer) |
| 28 | 3-3 | 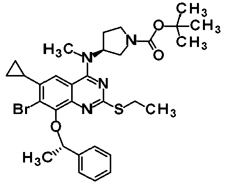 | 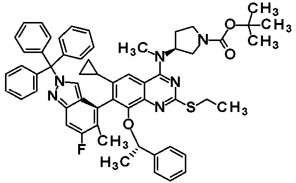 | ESI+: 939.8 | 2.5:1 (NMR) |
| | | | | 70% | |
| 29 | 3-3 | 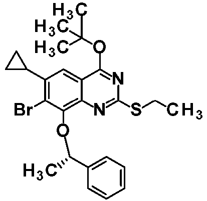 | 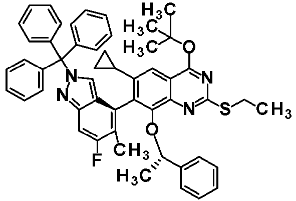 | ESI+: 813.4 | 3.2:1 (NMR) |
| | | | | 71% | |
| 30 | 3-3 | 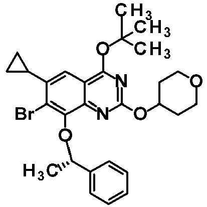 | 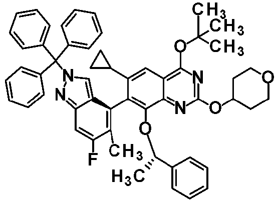 | ESI+: 853.4 | 3.6:1 (NMR) |
| | | | | 91% | |
| 31 | 3-3 | 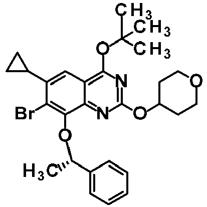 | 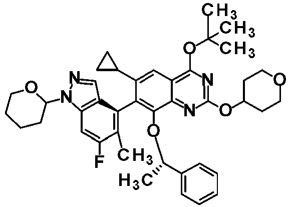 | ESI+: 695.4 | 1.6:1 (HPLC) |
| | | | | 88% | Analysis Condition 6 |
| | | | | | Retention time |
| | | | | | 5.36(P isomer) |
| | | | | | 5.40(M isomer) |

**[Table 2-5]**

| | | | | | |
|---|---|---|---|---|---|
| 32 | 3-3 | 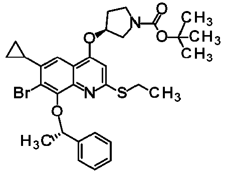 | 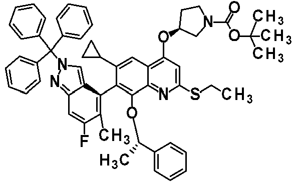 | ESI+: 925.7 | 7.9:1 (NMR) |
| | | | | 51% | |
| 33 | 3-3 | 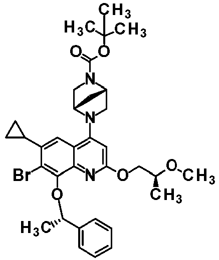 | 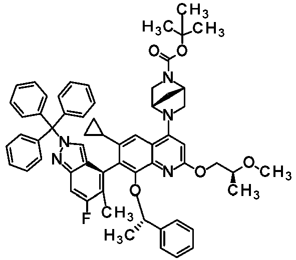 | ESI+: 964.6 | 4.0:1 (NMR) |
| | | | | 78% | |
| 34 | 34 | 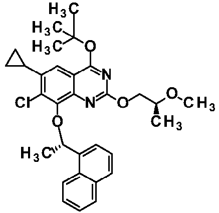 | 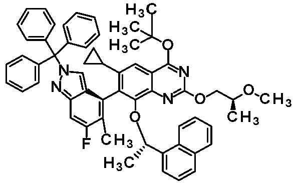 | ESI+: 891.6 | 15 : 1 (HPLC) |
| | | | | 63% | Analysis Condition 8 |
| | | | | | Retention time |
| | | | | | 27.05(M isomer) |
| | | | | | 28.53(P isomer) |

**[Table 3]**

| Ex | Syn | Starting Material | Synthetic Compound (Structure of major isomer) | DATA | Axial Enantioselectivity (M:P) |
|---|---|---|---|---|---|
| | | | | Yield | HPLC Analysis Condition |
| 35 | 35 | 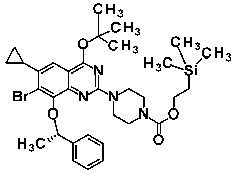 | 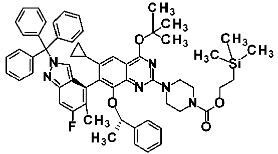 | ESI+: 981.7 | 3.5 : 1 (HPLC) |
| | | | | | 3.4:1 (NMR) |
| | | | | 80.9% | Analysis Condition 9 |
| | | | | | Retention time |
| | | | | | 45.6(M isomer) |
| | | | | | 51.1(P isomer) |

**[Table 4]**

| Ex | Syn | Synthetic Compound | DATA |
|---|---|---|---|
| 36 | 36 | 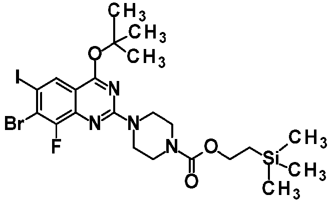 | ESI+: 652.9, 654.9 |
| 37 | 37 | 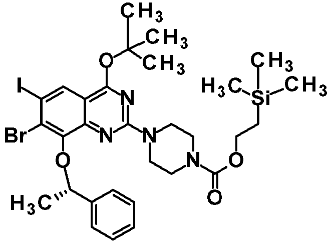 | ESI+: 755.3, 757.3 |
| 38 | 38 | 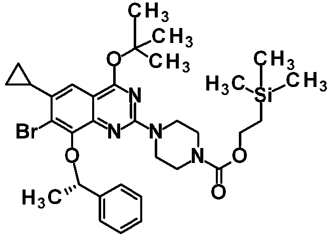 | ESI+: 669.5, 671.5 |

### INDUSTRIAL APPLICABILITY

According to the method of the present invention, in the production of a compound of formula (I) having axial chirality or a salt thereof, the axial chirality can be controlled using a chiral auxiliary, thereby enabling the selective preparation of one axially chiral compound or a salt thereof. Use of the method of the present invention allows for increased yield and a reduction in fractionation steps compared with conventional methods, and therefore the method is highly useful.

## Claims

1. A method for producing a compound of formula (I) or a salt thereof: 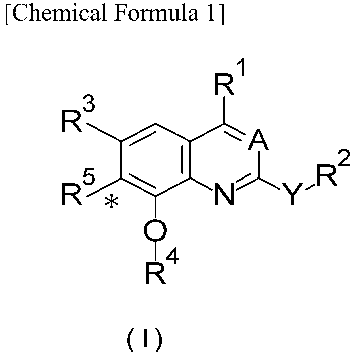 wherein
A is N or CH,
Y is a bond, -CH₂-, -O-, -S-, or -NR^{Y}-,
R^{Y} is H or optionally substituted C₁₋₃ alkyl,
R¹ is formula (IV) or (V), 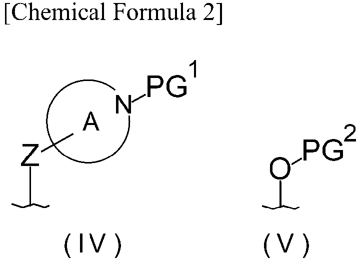
ring A is a 7- to 9-membered optionally substituted bridged heterocycloalkane containing 1 or 2 nitrogen atoms or a 4- to 6-membered optionally substituted heterocycloalkane containing 1 or 2 nitrogen atoms,
Z is a bond, -CH₂-, -O-, -S-, or -N(R^{Z1})-,
R^{Z1} is H or optionally substituted C₁₋₃ alkyl,
PG¹ is a protective group of NH contained in ring A,
PG² is a protective group of OH,
R² is optionally substituted C₁₋₁₅ alkyl or optionally substituted heterocycloalkyl,
R³ is halogen, C₃₋₆ cycloalkyl, vinyl, or optionally substituted C₁₋₃ alkyl,
R⁴ is formula (VI) or (VII), 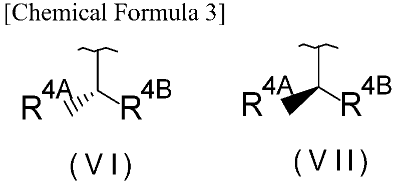
R^{4A} is C₁₋₃ alkyl,
R^{4B} is naphthyl, phenanthrenyl, or optionally substituted phenyl,
R⁵ is formula (VIII) or (IX), 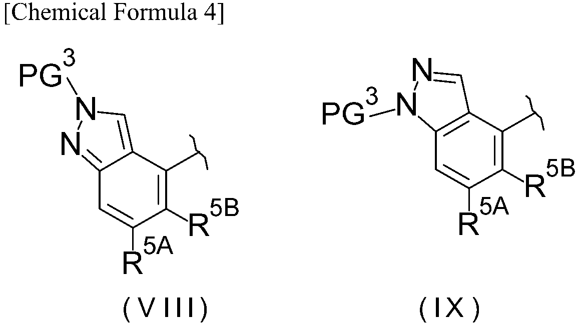
PG³ is a protective group of NH,
R^{5A} is H, methyl, F or Cl,
R^{5B} is Cl, methyl, ethyl, or vinyl, and
* denotes an axis of chirality,
the method comprising reacting a compound of formula (IIA) or a salt thereof with a compound of formula (IIIA) or a salt thereof, or alternatively reacting a compound of formula (IIB) or a salt thereof with a compound of formula (IIIB) or a salt thereof, to obtain the compound of formula (I) or a salt thereof: 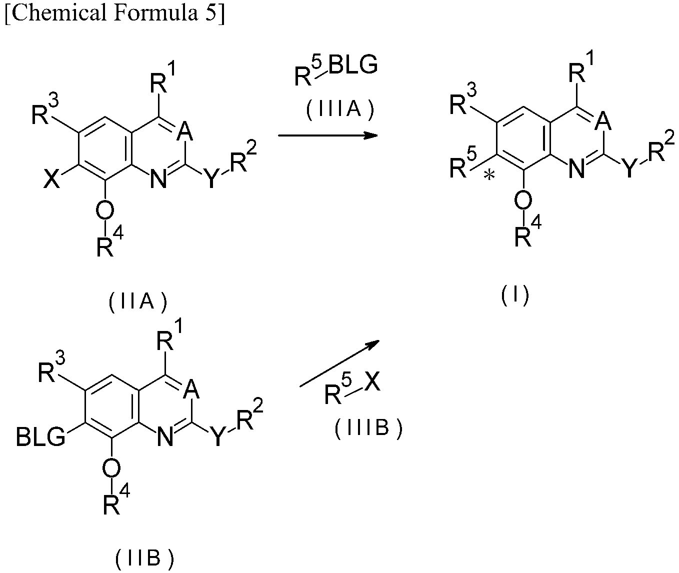 wherein
A, Y, R^{Y}, R¹, ring A, Z, R^{Z1}, PG¹, PG², R², R³, R⁴, R^{4A}, R^{4B}, R⁵, PG³, R^{5A}, R^{5B}, and * are as defined above,
X is Cl, Br, I, a methanesulfonyloxy group, a p-toluenesulfonyloxy group, and
BLG is a boronic acid group, a boronic ester group, a trifluoroborate group, or a triolborate group, and
wherein one axially chiral compound or a salt thereof among compounds of formula (I) having axial chirality or salts thereof is selectively produced.

2. The method according to claim 1, comprising
reacting the compound of formula (IIA) or a salt thereof with the compound of formula (IIIA) or a salt thereof,
wherein
X is Cl or Br,
Y is -O- or -S-,
R¹ is a group selected from the group consisting of formulas (IV-1), (IV-2), (IV-3), and (V): 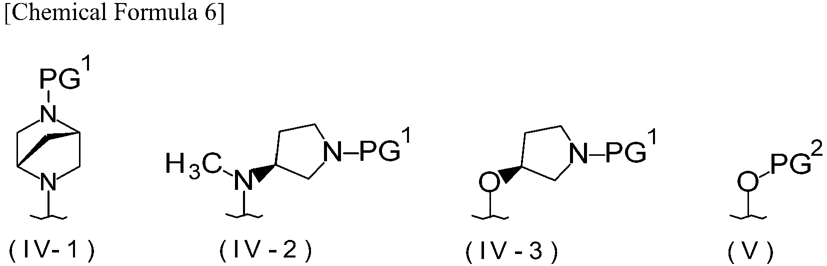
R² is tetrahydropyranyl or C₁₋₃ alkyl optionally substituted with -OCH₃,
R³ is cyclopropyl,
R⁴ is a group selected from the group consisting of formulas (VI-1), (VI-2), and (VI-3): 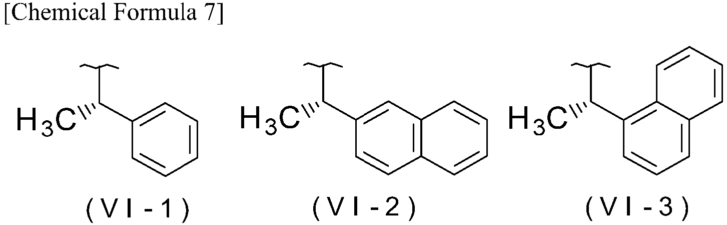 , and
R⁵ is a group selected from the group consisting of formulas (VIII-1) and (IX-1).

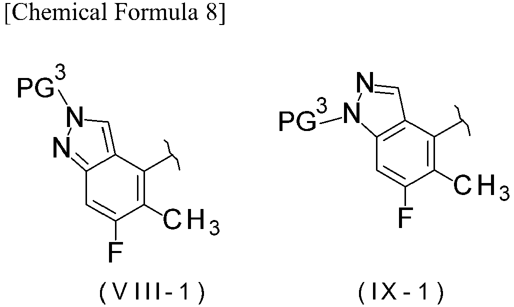

3. The method according to claim 2, comprising
reacting the compound of formula (IIA) or a salt thereof with the compound of formula (IIIA) or a salt thereof,
wherein
A is N,
R¹ is a group selected from the group consisting of formulas (IV-1), (IV-2), and (V): 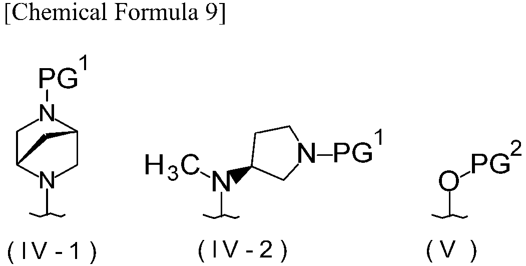 , and
R⁵ is a group represented by formula (VIII-1).

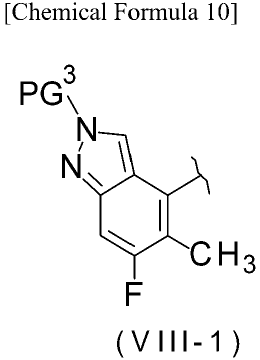

4. The method according to claim 3, wherein
the compound of formula (IIA) or a salt thereof is a compound represented by formula (IIA-1): 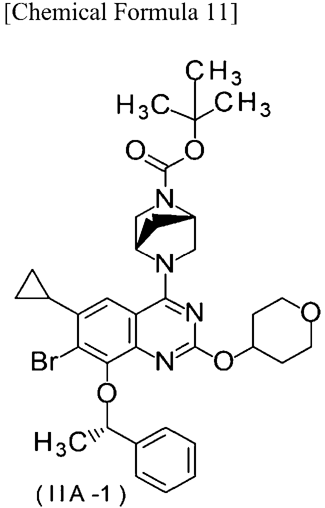 , and
the compound of formula (IIIA) or a salt thereof is a compound represented by formula (IIIA-1): 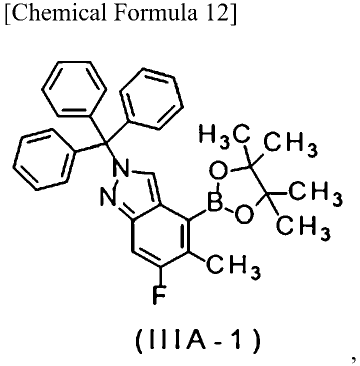
the method comprising obtaining a compound represented by formula (I-1) by the following step.

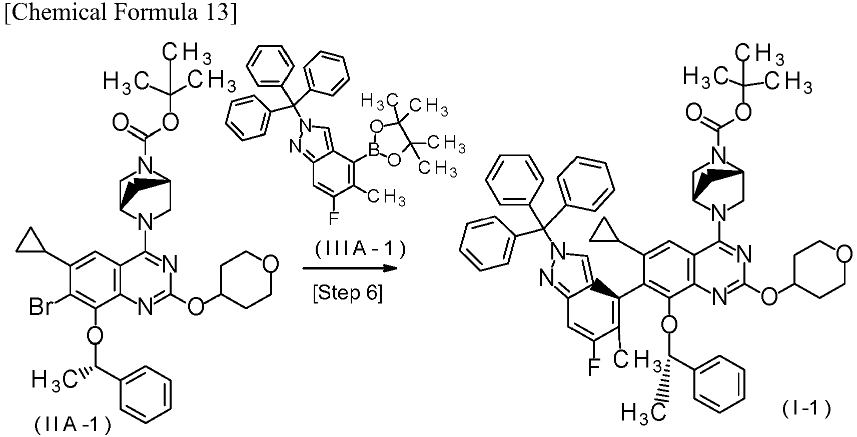

5. The method according to claim 4, comprising obtaining compound (1) or a salt thereof by the following steps.

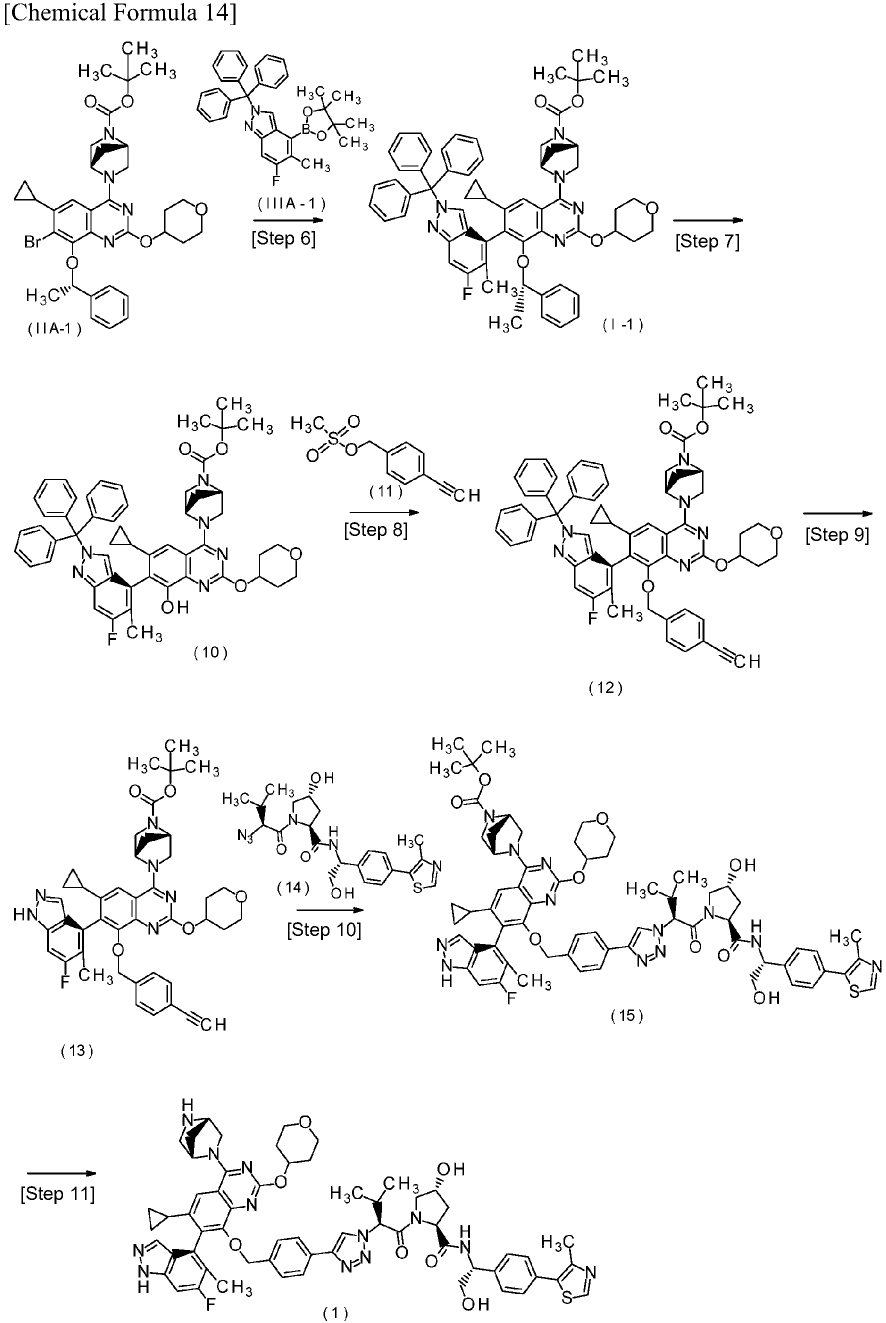

6. The method according to claim 5, comprising obtaining compound (14) from compound (16) and compound (17) by the following steps.

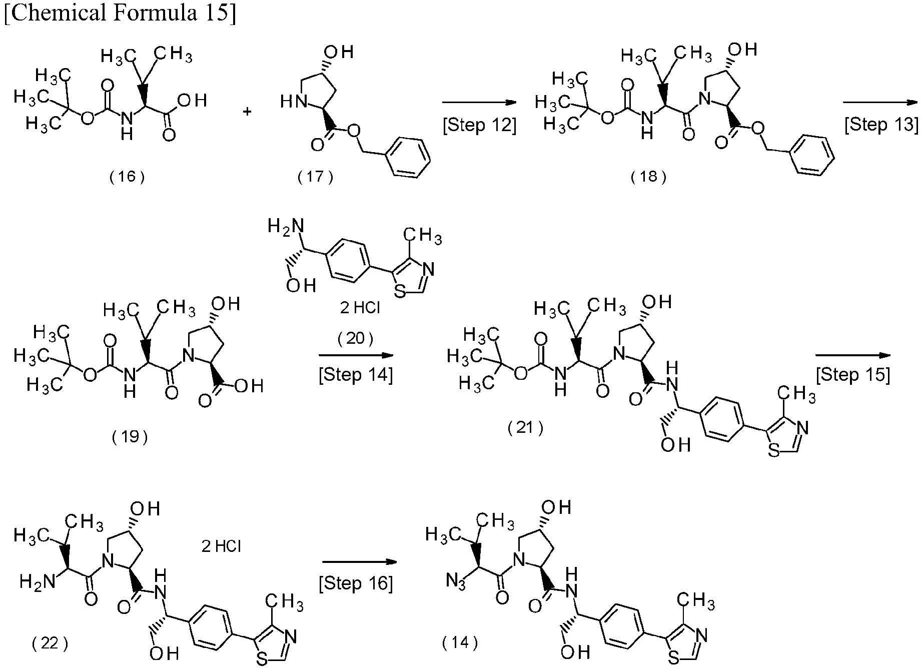

7. The method according to claim 5, comprising obtaining the compound of formula (IIA-1) from compound (2) by the following steps.

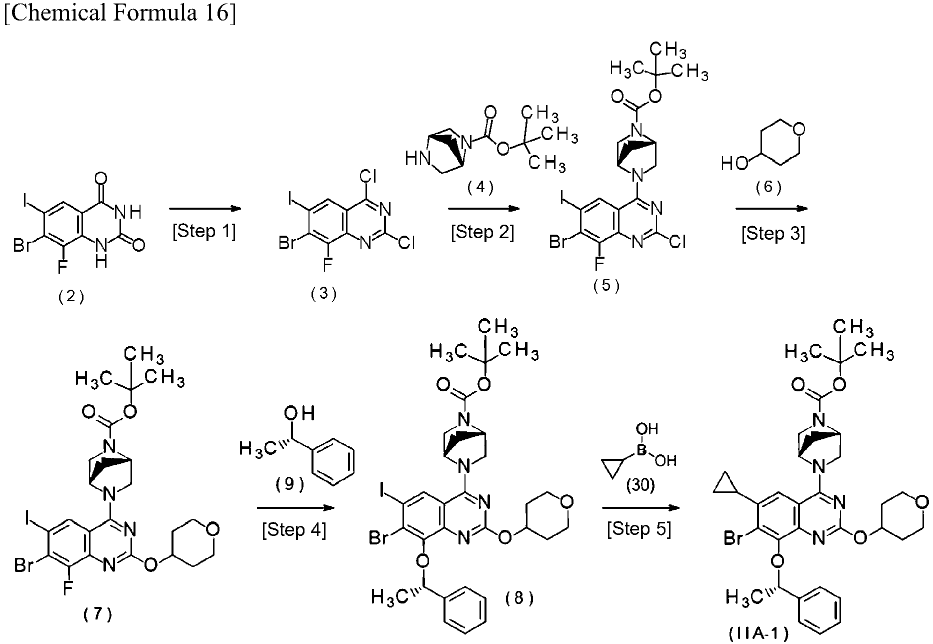

8. The method according to claim 6, comprising obtaining compound (20) from compound (24) and compound (25) by the following steps.

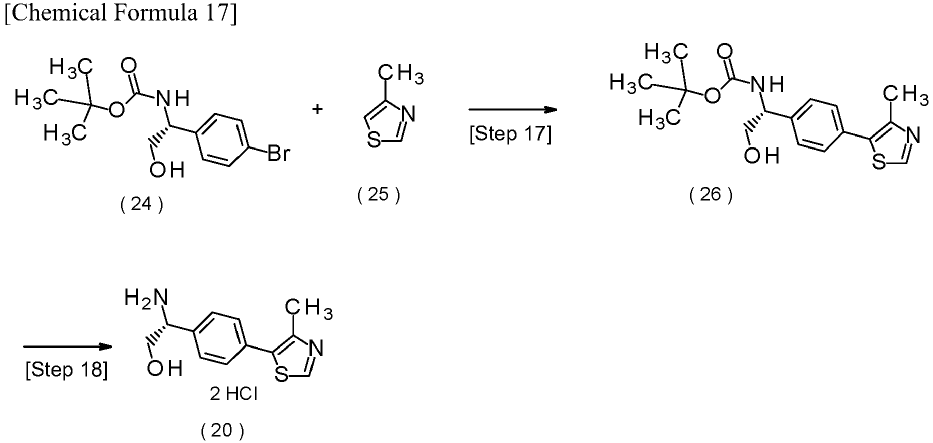

9. The method according to any one of claims 4 to 8, comprising obtaining the compound of formula (IIIA-1) from compound (27) by the following steps.

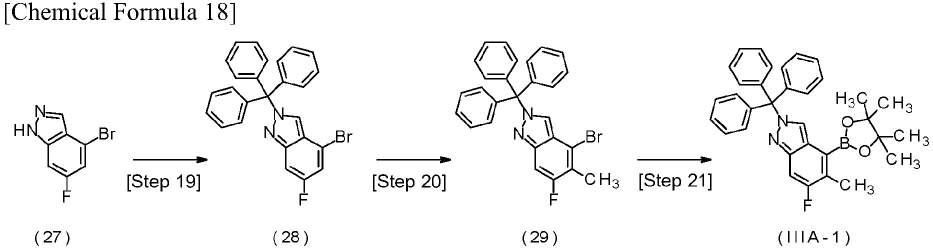

10. The method according to claim 3, wherein
the compound of formula (IIA) or a salt thereof is a compound represented by formula (IIA-2): 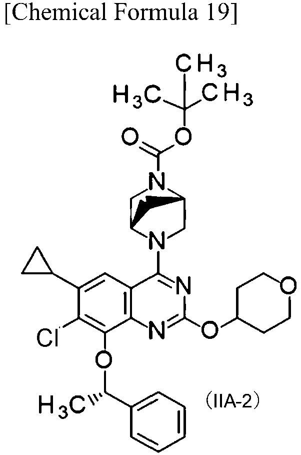 , and
the compound of formula (IIIA) or a salt thereof is a compound represented by formula (IIIA-1): 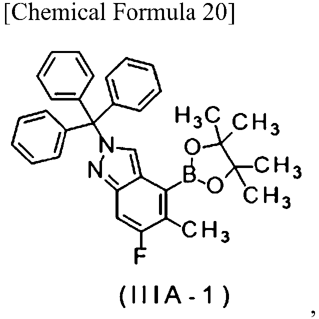
the method comprising obtaining a compound represented by formula (I-1) by the following step.

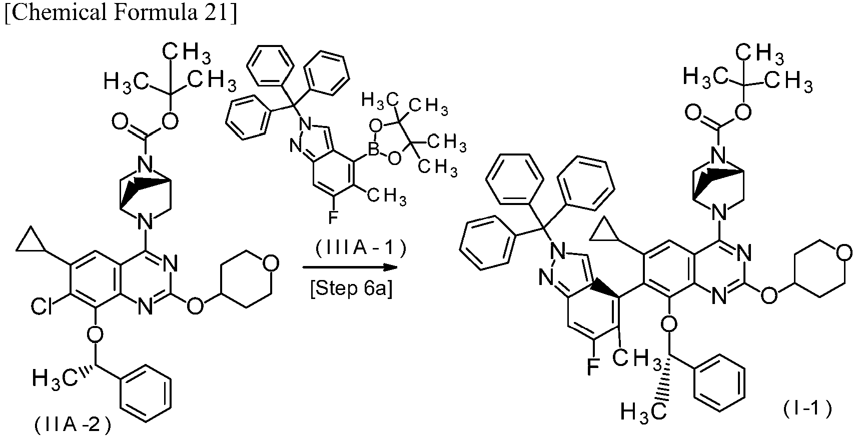

11. The method according to claim 10, comprising obtaining compound (1) or a salt thereof by the following steps.

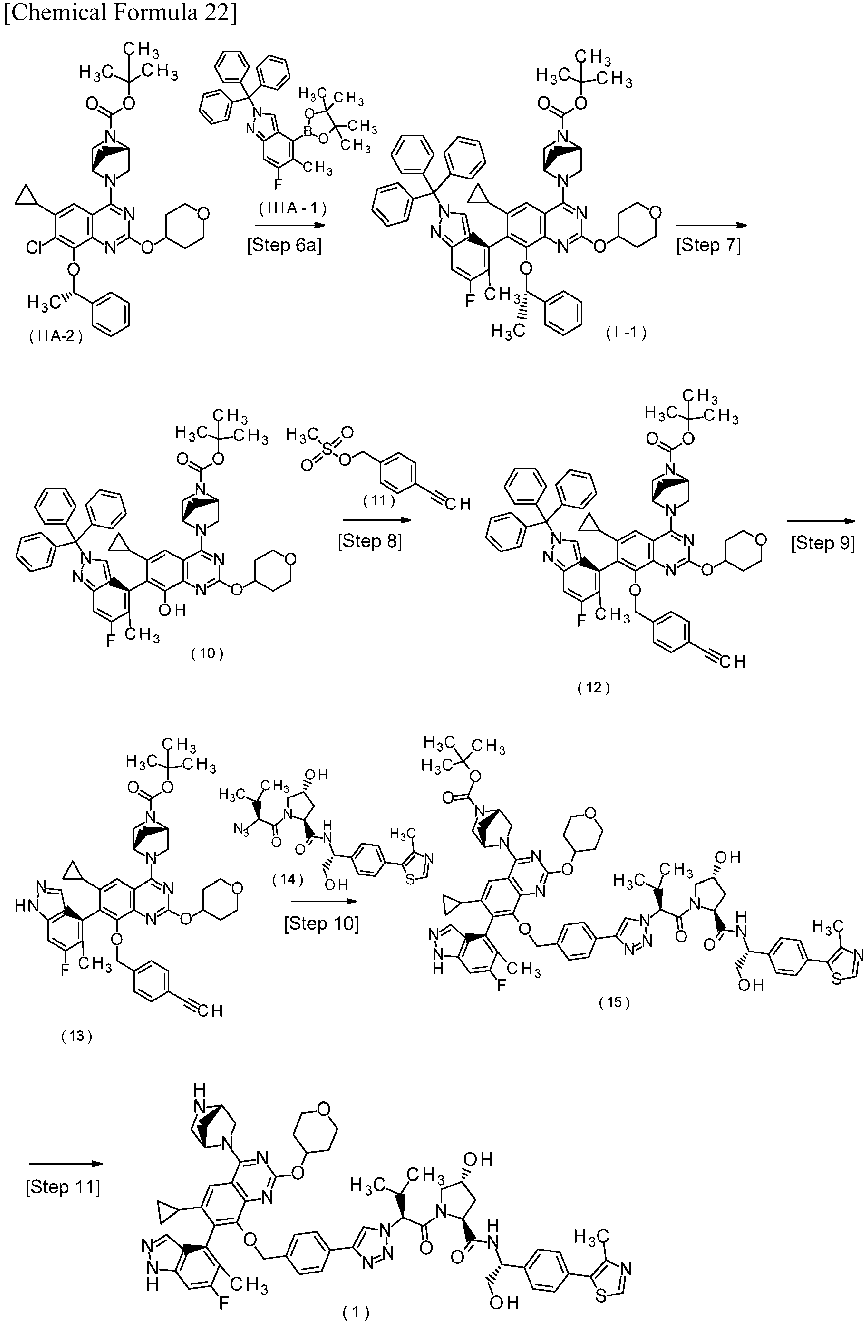

12. The method according to claim 10 or 11, comprising obtaining the compound of formula (IIA-2) by the following steps.

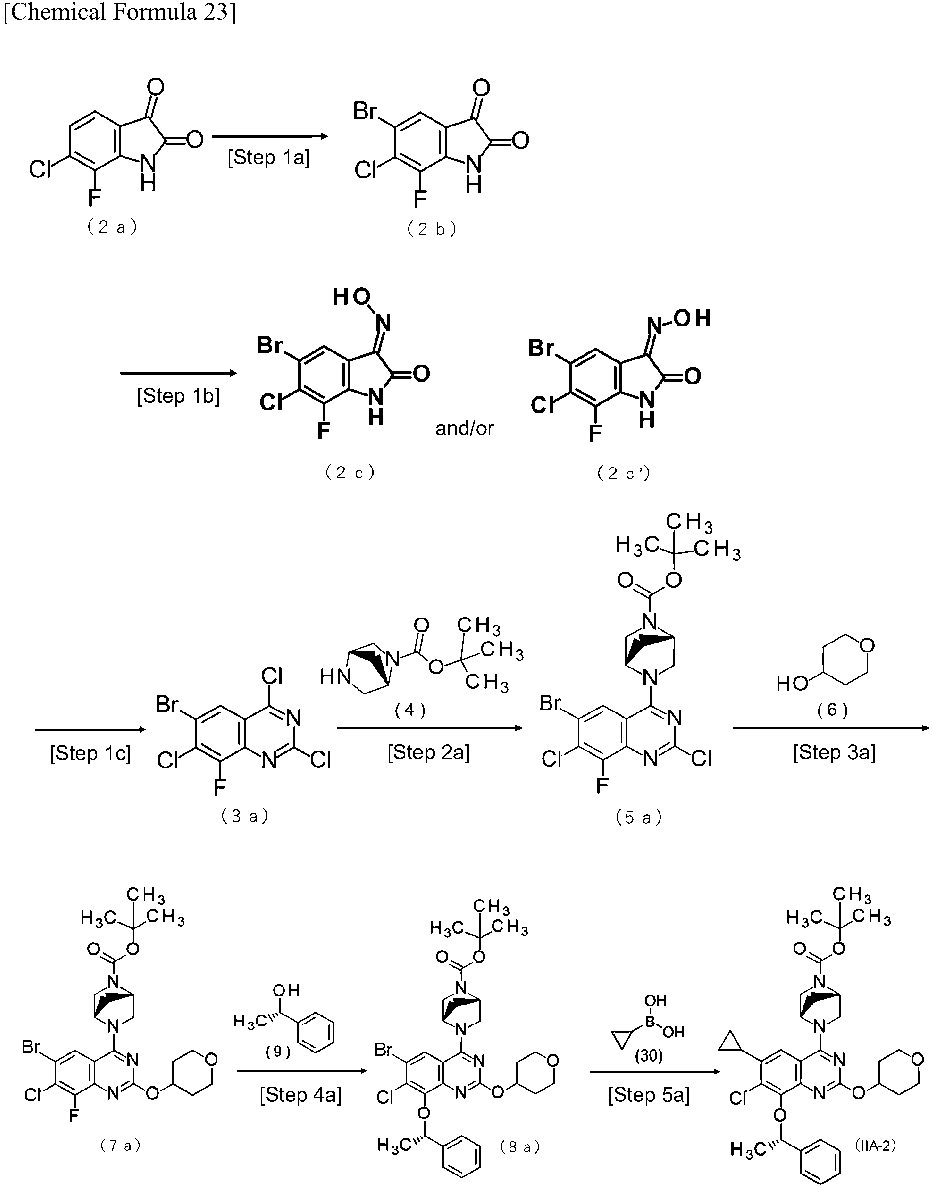

13. A compound represented by any one of the following formulas, or a salt thereof.
